# EUROPÄISCHE PATENTSCHRIFT

(11) **EP 0 873 307 B1**
(45) Veröffentlichungstag und Bekanntmachung des Hinweises auf die Patenterteilung: **31.05.2000**
(21) Anmeldenummer: 96943130.3
(22) Anmeldetag: 16.12.1996
(51) Int. Cl.: C07C 255/61, A01N 37/34

(54) **CYANIMINOOXIMETHER, VERFAHREN UND ZWISCHENPRODUKTE ZU IHRER HERSTELLUNG UND IHRE VERWENDUNG ZUR BEKÄMPFUNG VON SCHADPILZEN UND TIERISCHEN SCHÄDLINGEN**
CYANIMINO OXIME ETHERS, PROCESS AND INTERMEDIATES FOR THE PREPARATION AND USE THEREOF FOR THE CONTROL OF NOXIOUS FUNGI AND ANIMAL PESTS
ETHERS D'OXIME CYANIMINO ET PRODUITS INTERMEDIAIRES UTILISES POUR LES PREPARER ET LEUR UTILISATION POUR LUTTER CONTRE DES CHAMPIGNONS NOCIFS ET DES PARASITES ANIMAUX

(30) Priorität: 27.12.1995 DE 19548783
(43) Veröffentlichungstag der Anmeldung: 28.10.1998
(73) Patentinhaber: BASF AKTIENGESELLSCHAFT, 67056 Ludwigshafen (DE)
(72) Erfinder: BAYER, Herbert, D-68159 Mannheim (DE); MÜLLER, Ruth, D-67159 Friedelsheim (DE); SAUTER, Hubert, D-68167 Mannheim (DE); GRAMMENOS, Wassilios, D-67063 Ludwigshafen (DE); GROTE, Thomas, D-67105 Schifferstadt (DE); KIRSTGEN, Reinhard, D-67434 Neustadt (DE); MÜLLER, Bernd, D-67227 Frankenthal (DE); OBERDORF, Klaus, D-69117 Heidelberg (DE); RÖHL, Franz, D-67105 Schifferstadt (DE); AMMERMANN, Eberhard, D-64646 Heppenheim (DE); HARRIES, Volker, D-67227 Frankenthal (DE); LORENZ, Gisela, D-67434 Hambach (DE); STRATHMANN, Siegfried, D-67117 Limburgerhof (DE)
(86) Internationale Anmeldenummer: EP9605641
(87) Internationale Veröffentlichungsnummer: WO9724319

(56) Entgegenhaltungen:
- EP-A- 0 564 984
- WO-A-93/16986
- WO-A-95/18789

## Beschreibung

Die vorliegende Erfindung betrifft Cyaniminooximether der Formel I 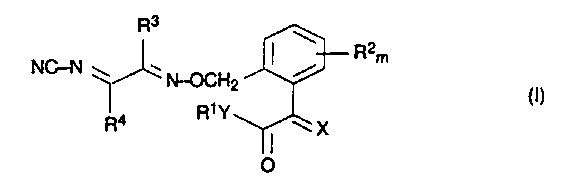 in der die Variablen die folgenden Bedeutungen haben:
- X: NOCH₃, CHOCH₃ oder CHCH₃;
- Y: O oder NZ, wobei Z für Wasserstoff oder C₁-C₄-Alkyl steht;
- R¹: Wasserstoff oder C₁-C₄-Alkyl;
- R²: Cyano, Nitro, Halogen, C₁-C₄-Alkyl, Trifluormethyl oder C₁-C₄-Alkoxy;
- m: 0, 1 oder 2, wobei die Reste R² verschieden sein können, wenn m für 2 steht;
- R³: Wasserstoff, Cyano, Alkyl, Halogenalkyl, Alkoxy oder Cycloalkyl;
- R⁴: Wasserstoff oder gegebenenfalls substituiertes: Alkyl, Alkenyl, Alkinyl, Cycloalkyl, Cycloalkenyl, Heterocyclyl, Aryl oder Heteroaryl,
sowie deren Salze.

Außerdem betrifft die Erfindung Verfahren und Zwischenprodukte zur Herstellung dieser Verbindungen sowie sie enthaltende Mittel und die Verwendung der Verbindungen I zur Bekämpfung tierischer Schädlinge und Schadpilze.

Phenylessigsäure-Derivate des Typs I mit fungizider und insektizider Wirkung sind aus folgenden Druckschriften bekannt: WO-A 95/18789 und WO-A 93/16986. Hinsichtlich ihrer Wirkung vermögen die dort offenbarten Wirkstoffe jedoch noch nicht zu befriedigen.

Der vorliegenden Erfindung lagen daher neue Verbindungen mit verbesserter Wirkung gegen Schadpilze und Schädlinge als Aufgabe zugrunde.

Demgemäß wurden die eingangs definierten Cyaniminooximether I gefunden. Außerdem wurden Verfahren und Zwischenprodukte zu ihrer Herstellung sowie sie enthaltende Mittel zur Bekämpfung von tierischen Schädlingen und Schadpilzen und die Verwendung der Verbindungen I hierzu gefunden.

Die Verbindungen I sind auf verschiedenen Wegen nach an sich bekannten Verfahren erhältlich.

Grundsätzlich ist es bei der Synthese der Verbindungen I unerheblich, ob zunächst die Gruppierung -C(X)-CO-YR¹ oder die Gruppierung -CH₂ON=C(R³)-C(R⁴) =N-CN aufgebaut wird.

Der Aufbau der Gruppierung -C(X)-CO-YR¹ ist beispielsweise aus folgenden Druckschriften bekannt: EP-A 422 597, EP-A 463 488, EP-A 370 629, EP-A 460 575, EP-A 472 300, WO-A 90/07493, WO-A 92/13830, WO-A 92/18487, DE-Anm. P 44 20 416.7.

Bei den folgenden Synthesebeschreibungen steht "Houben-Weyl" für: Houben-Weyl, Methoden der Organischen Chemie, Georg Thieme Verlag, Stuttgart.
- 1.1: Man geht beim Aufbau der Gruppierung -CH₂ON=C(R³)-C(R⁴)=N-CN im allgemeinen so vor, daß man ein Benzylderivat der Formel II mit einem Hydroxyimin der Formel III umsetzt. 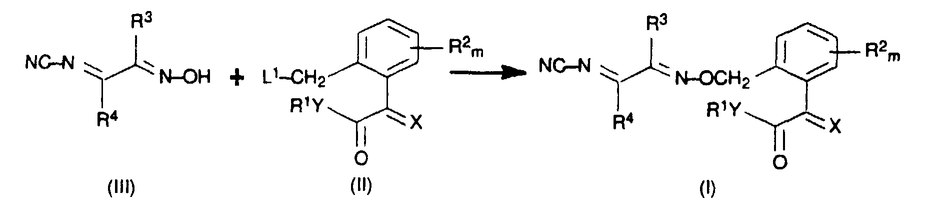 L¹ in der Formel II steht für eine nukleophil austauschbare Abgangsgruppe, z.B. Halogen oder eine Sulfonatgruppe, vorzugsweise Chlor, Brom, Iod, Mesylat, Tosylat oder Triflat.
Die Umsetzung erfolgt in an sich bekannter Weise in einem inerten organischen Lösungsmittel in Gegenwart einer Base, z.B. Natriumhydrid, Kaliumhydroxid, Kaliumcarbonat oder Triethylamin, gemäß literaturbekannten Methoden (vgl. Houben-Weyl, 4. Auflage, Bd. E 14b, S. 370 ff. und Bd. 10/1, S. 1189 ff).
- 1.2: Die Hydroxyimine III erhält man beispielsweise durch Umsetzung eines Carbonylhydroxyiminoderivates IV mit Bis(trimethylsilyl)carbodiimid. 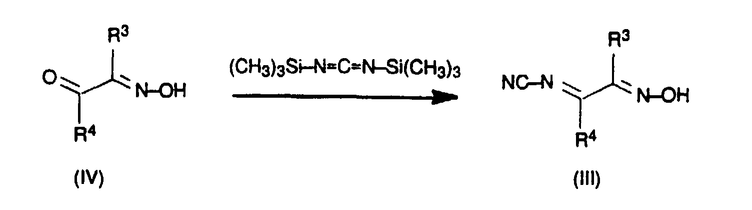
Die Umsetzung erfolgt in an sich bekannter Weise in einem inerten organischen Lösungsmittel, vorzugsweise unter Fluorid-oder Cyanidkatalyse oder in Gegenwart von Lewis-Säuren wie Titantetrachlorid (Liebigs Ann. Chem. 1986, Seite 142 ff.; Angew. Chemie 96, 1984, Seite 437 ff.).
Es kann bisweilen vorteilhaft sein, die Oximfunktion von IV vor der Reaktion nach allgemein bekannten Methoden mit einer Schutzgruppe zu versehen, z.B. einer Acylgruppe (Alkylcarbonylgruppe) oder einer Sulfonylgruppe, welche später wieder abgespalten werden kann. Diese Schutzgruppentechnik läßt sich auch für nucleophile Zentren im Rest R⁴ anwenden.
Die Carbonylhydroxyiminoderivate IV sind bekannt oder können nach an sich bekannten Methoden hergestellt werden (vgl. Org. Synth. 16, 1936, Seite 46; Bull. Chem. Soc. Jap. 44, 1971, Seite 219 ff.; DE-A-27 22 416; Gazz. Chim. Ital. 61, 1931, Seite 943 ff; Chem. Ber 106, 1973 Seite 1688 ff.).
- 2.: Alternativ können die Verbindungen I auch dadurch erhalten werden, daß man das Benzylderivat II zunächst mit dem Carbonylhydroxyiminoderivat IV in ein entsprechendes Benzyloxim der Formel V, und V anschließend mit Bis(trimethylsilyl)carbodiimid zu I umsetzt. 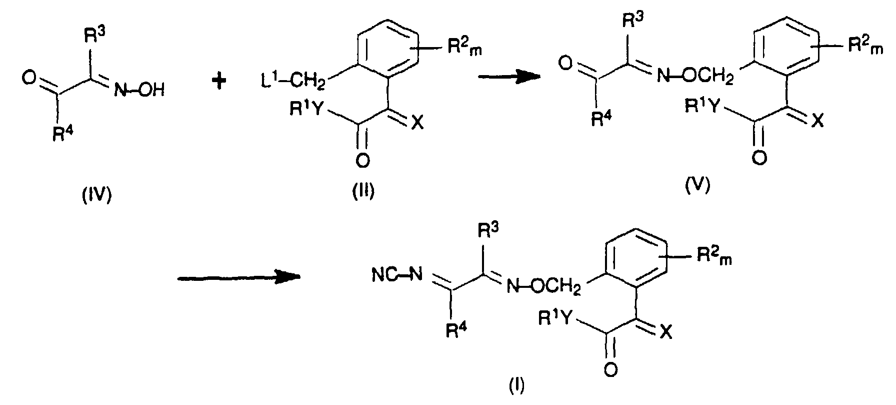
Die Umsetzung von IV mit II erfolgt analog zur Synthese, wie sie unter 1.1 beschrieben ist. Die Umsetzung von V mit Bis(trimethylsilyl)carbodiimid erfolgt analog zur Synthese, wie sie unter 1.2 beschrieben ist.
- 3.: Des weiteren erhält man die Verbindungen I dadurch, daß man eine Verbindung III gemäß der EP-A 493 711 mit einem Lacton VI zunächst in die entsprechende Benzoesäure VII überführt und VII über die entsprechenden Halogenide VIII in die Cyanocarbonsäuren IX überführt, welche im Wege der Pinner-Reaktion (Angew. Chem. 94, Seite 1, 1982) in die α-Ketoester X überführt und ggf. weiter zu den α-Ketoamiden XI umgesetzt werden (vgl. EP-A 348 766, DE-A 37 05 389, EP-A 178 826, DE-A 36 23 921, Houben-Weyl, 4. Auflage, Bd. E5, S. 941 ff.). 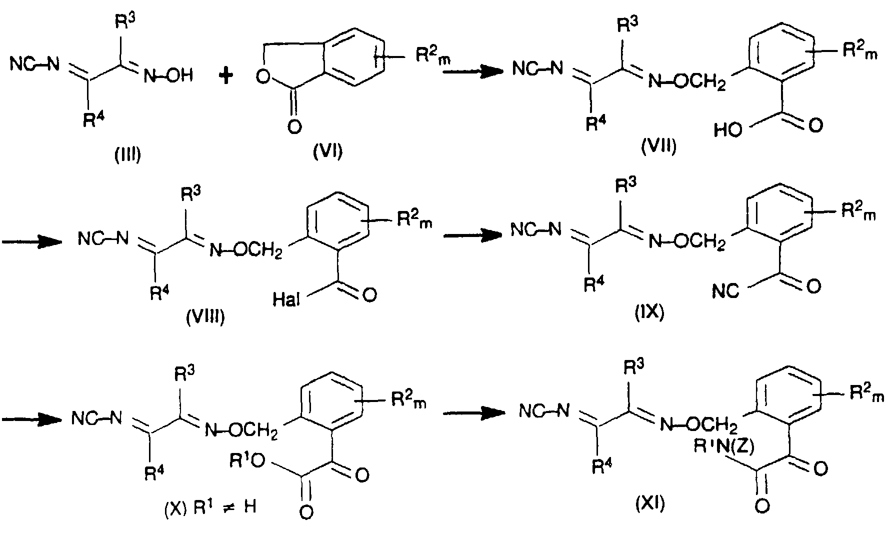
(Z =Wasserstoff oder C₁-C₄-Alkyl)

Die α-Ketoester X und die α-Ketoamide XI können gemäß üblichen Verfahren in die Verbindungen I überführt werden (vgl. EP-A 178 826, EP-A 348 766, DE-A 36 23 921, DE-A 37 05 389 sowie die eingangs zitierte Literatur).

Verbindungen I, in denen R¹ Wasserstoff bedeutet, erhält man durch Verseifung der Ester X und anschließende Umsetzung zu I.

Die Verbindungen I, in denen Y für NZ steht, können auch aus den Estern (Y=O) durch Umsetzung mit den entsprechenden Aminen HN(Z)R¹ erhalten werden.

Die Verbindungen II sind bekannt aus EP-A 513 580, EP-A 477 631, EP-A 463 488, EP-A 251 082, EP-A 400 417 und/oder EP-A 585 751 oder können nach den dort beschriebenen Methoden hergestellt werden.

Die Verbindungen I können bei der Herstellung aufgrund ihrer C=C- und C=N-Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung der Isomeren nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen, in der behandelten Pflanze oder im zu bekämpfenden Schadpilz oder tierischen Schädling erfolgen.

In Bezug auf die C=X- Doppelbindung werden hinsichtlich ihrer Wirksamkeit die E-Isomere der Verbindungen I bevorzugt (Konfiguration bezogen auf die -OCH₃ bzw. die -CH₃ Gruppe im Verhältnis zur -COYR¹ Gruppe).

In Bezug auf die -C(R³)=NOCH₂- Doppelbindung werden hinsichtlich ihrer Wirksamkeit die cis-Isomere der Verbindungen I bevorzugt (der Rest R³ und die -OCH₂- Gruppe stehen auf der gleichen Seite der Doppelbindung).

Teil der Erfindung sind auch die Salze der säurebeständigen Verbindungen I, welche basische Zentren, vor allem basische Stickstoffatome enthalten, insbesondere mit Mineralsäuren wie Schwefelsäure und Phosphorsäure oder Lewis-Säuren wie Zinkchlorid. Üblicherweise kommt es hierbei auf die Art des Salzes nicht an. Im Sinne der Erfindung sind solche Salze bevorzugt, die die von Schadpilzen oder tierischen Schädlingen freizuhaltenden Pflanzen, Flächen, Materialien oder Räume nicht schädigen und die Wirkung der Verbindungen I nicht beeinträchtigen. Besonders bedeutsam sind derartige, für landwirtschaftliche Zwecke geeignete Salze.

Die Salze der Verbindungen I sind in an sich bekannter Weise zugänglich, vor allem durch Umsetzen der entsprechenden Verbindung I mit den genannten Säuren in Wasser oder einem inerten organischen Lösungsmittel bei Temperaturen von -80 bis 120°C, vorzugsweise 0 bis 60°C.

Bei den eingangs angegebenen Definitionen der Verbindungen I wurden Sammelbegriffe verwendet, die allgemein repräsentativ für die folgenden Gruppen stehen:
**Halogen:** Fluor, Chlor, Brom und Jod;
**Alkyl:** geradkettige oder verzweigte Alkylgruppen mit 1 bis 4, 6 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-l-methylpropyl und 1- Ethyl-2-methylpropyl;
**Alkylamino:** eine Aminogruppe, welche eine geradkettige oder verzweigte Alkylgruppe mit 1 bis 6 Kohlenstoffatomen wie vorstehend genannt trägt;
**Dialkylamino:** eine Aminogruppe, welche zwei voneinander unabhängige, geradkettige oder verzweigte Alkylgruppen mit jeweils 1 bis 6 Kohlenstoffatomen wie vorstehend genannt, trägt;
**Alkylcarbonyl:** geradkettige oder verzweigte Alkylgruppen mit 1 bis 10 Kohlenstoffatomen, welche über eine Carbonylgruppe (-CO-) an das Gerüst gebunden sind;
**Alkylsulfonyl:** geradkettige oder verzweigte Alkylgruppen mit 1 bis 6 oder 10 Kohlenstoffatomen, welche über eine Sulfonylgruppe (-SO₂-) an das Gerüst gebunden sind;
**Alkylsulfoxyl:** geradkettige oder verzweigte Alkylgruppen mit 1 bis 6 Kohlenstoffatomen, welche über eine Sulfoxylgruppe (-S(=O)-) an das Gerüst gebunden sind;
**Alkylaminocarbonyl:** Alkylaminogruppen mit 1 bis 6 Kohlenstoffatomen wie vorstehend genannt, welche über eine Carbonylgruppe (-CO-) an das Gerüst gebunden sind;
**Dialkylaminocarbonyl:** Dialkylaminogruppen mit jeweils 1 bis 6 Kohlenstoffatomen pro Alkylrest wie vorstehend genannt, welche über eine Carbonylgruppe (-CO-) an das Gerüst gebunden sind;
**Alkylaminothiocarbonyl:** Alkylaminogruppen mit 1 bis 6 Kohlenstoffatomen wie vorstehend genannt, welche über eine Thiocarbonylgruppe (-CS-) an das Gerüst gebunden sind;
**Dialkylaminothiocarbonyl:** Dialkylaminogruppen mit jeweils 1 bis 6 Kohlenstoffatomen pro Alkylrest wie vorstehend genannt, welche über eine Thiocarbonylgruppe (-CS-) an das Gerüst gebunden sind;
**Halogenalkyl:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 4 oder 6 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
**Alkoxycarbonyl:** geradkettige oder verzweigte Alkylgruppen mit 1 bis 6 Kohlenstoffatomen, welche über eine Oxycarbonylgruppe (-OC(=O)-) an das Gerüst gebunden sind;
**Alkylendioxy:** z.B. C₁-C₄-Alkylendioxy: geradkettige oder verzweigte Alkylengruppen mit 1 bis 4 Kohlenstoffatomen, welche an zwei Stellen über je ein Sauerstoffatom (-O-) in das Gerüst eingebunden oder an das Gerüst gebunden sind wie Methylendioxy (-O-CH₂-O-) oder 2,2-Propylendioxy (-O-C(CH₃)₂-O-);
**Halogenalkoxy:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, und wobei diese Gruppen über ein Sauerstoffatom an das Gerüst gebunden sind;
**Alkylthio:** geradkettige oder verzweigte Alkylgruppen mit 1 bis 4 oder 6 Kohlenstoffatomen wie vorstehend genannt, welche über ein Schwefelatom (-S-) an das Gerüst gebunden sind, z.B. C₁-C₆-Alkylthio wie Methylthio, Ethylthio, Propylthio, 1-Methylethylthio, Butylthio, 1-Methylpropylthio, 2-Methylpropylthio, 1,1-Dimethylethylthio, Pentylthio, 1-Methylbutylthio, 2-Methylbutylthio, 3-Methylbutylthio, 2,2-Di-methylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-l-methylpropylthio und 1-Ethyl-2-methylpropylthio;
**Cycloalkyl:** monocyclische Alkylgruppen mit 3 bis 6 Kohlenstoffringgliedern, z.B. Cyclopropyl, Cyclobutyl, Cyclopentyl und Cyclohexyl;
**Alkenyl:** geradkettige oder verzweigte Alkenylgruppen mit 2 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1- Ethyl-1-methyl-2-propenyl, 1-Ethyl-2-methyl-1-propenyl und 1-Ethyl-2-methyl-2-propenyl;
**Alkenyloxy:** geradkettige oder verzweigte Alkenylgruppen mit 2 bis 6 Kohlenstoffatomen und einer Doppelbindung in einer beliebigen Position, welche über ein Sauerstoffatom (-O-) an das Gerüst gebunden sind;
**Alkenylthio bzw. Alkenylamino:** geradkettige oder verzweigte Alkenylgruppen mit 2 bis 6 Kohlenstoffatomen und einer Doppelbindung in einer beliebigen Position, welche (Alkenylthio) über ein Schwefelatom bzw. (Alkenylamino) ein Stickstoffatom an das Gerüst gebunden sind.
**Alkenylcarbonyl:** geradkettige oder verzweigte Alkenylgruppen mit 2 bis 10 Kohlenstoffatomen und einer Doppelbindung in einer beliebigen Position, welche über eine Carbonylgruppe (-CO-) an das Gerüst gebunden sind;
**Alkinyl:** geradkettige oder verzweigte Alkinylgruppen mit 2 bis 10 Kohlenstoffatomen und einer Dreifachbindung in einer beliebigen Position, z.B. C₂-C₆-Alkinyl wie Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
**Alkinyloxy bzw. Alkinylthio und Alkinylamino:** geradkettige oder verzweigte Alkinylgruppen mit 2 bis 6 Kohlenstoffatomen und einer Dreifachbindung in einer beliebigen Position, welche (Alkinyloxy) über ein Sauerstoffatom bzw. (Alkinylthio) über ein Schwefelatom oder (Alkinylamino) über ein Stickstoffatom an das Gerüst gebunden sind.
**Alkinylcarbonyl:** geradkettige oder verzweigte Alkinylgruppen mit 3 bis 10 Kohlenstoffatomen und einer Dreifachbindung in einer beliebigen Position, welche über eine Carbonylgruppe (-CO-) an das Gerüst gebunden sind;
**Cycloalkenyl bzw. Cycloalkenyloxy, Cycloalkenylthio und Cycloalkenylamino:** monocyclische Alkenylgruppen mit 3 bis 6 Kohlenstoffringgliedern, welche direkt bzw. (Cycloalkenyloxy) über ein Sauerstoffatom oder (Cycloalkenylthio) ein Schwefelatom oder Cycloalkenylamino) über ein Stickstoffatom an das Gerüst gebunden sind, z.B. Cyclopropenyl, Cyclobutenyl, Cyclopentenyl oder Cyclohexenyl.
**Cycloalkyloxy bzw. Cycloalkylthio und Cycloalkylamino:** monocyclische Alkylgruppen mit 3 bis 6 Kohlenstoffringgliedern, welche (Cycloalkyloxy) über ein Sauerstoffatom oder (Cycloalkylthio) ein Schwefelatom oder (Cycloalkylamino) über ein Stickstoffatom an das Gerüst gebunden sind, z.B. Cyclopropyl, Cyclobutyl, Cyclopentyl oder Cyclohexyl;
**Heterocyclyl bzw. Heterocyclyloxy, Heterocyclylthio und Heterocyclylamino:** drei- bis sechsgliedrige, gesättigte oder partiell ungesättigte mono- oder polycyclische Heterocyclen, die ein bis drei Hereroatome ausgewählt aus einer Gruppe bestehend aus Sauerstoff, Stickstoff und Schwefel enthalten, und welche direkt bzw. (Heterocyclyloxy) über ein Sauerstoffatom oder (Heterocyclylthio) über ein Schwefelatom oder (Heterocyclylamino) über ein Stickstoffatom an das Gerüst gebunden sind, wie z.B. 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydro-fur-4-yl, 2,3-Dihydro-fur-5-yl, 2,5-Dihydro-fur-2-yl, 2,5-Dihydro-fur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisothiazol-3-yl, 2,5-Dihydroisothiazol-4-yl, 2,5-Dihydroisothiazol-5-yl, 2,3-Dihydroisopyrazol-3-yl, 2,3-Dihydroisopyrazol-4-yl, 2,3-Dihydroisopyrazol-5-yl, 4,5-Dihydroisopyrazol-3-yl, 4,5-Dihydroisopyrazol-4-yl, 4,5-Dihydroisopyrazol-5-yl, 2,5-Dihydroisopyrazol-3-yl, 2,5-Dihydroisopyrazol-4-yl, 2,5-Dihydroisopyrazol-5-yl, 2,3-Dihydrooxazol-3-yl, 2,3-Dihydrooxazol-4-yl, 2,3-Dihydrooxazol-5-yl, 4,5-Dihydrooxazol-3-yl, 4,5-Dihydrooxazol-4-yl, 4,5-Dihydrooxazol-5-yl, 2,5-Dihydrooxazol-3-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl,

**Aryl bzw. Aryloxy, Arylthio, Arylcarbonyl und Arylsulfonyl:** aromatische mono- oder polycyclische Kohlenwasserstoffreste welche direkt bzw. (Aryloxy) über ein Sauerstoffatom (-O-) oder (Arylthio) ein Schwefelatom (-S-), (Arylcarbonyl) über eine Carbonylgruppe (-CO-) oder (Arylsulfonyl) über eine Sulfonylgruppe (-SO₂-) an das Gerüst gebunden sind, z.B. Phenyl, Naphthyl und Phenanthrenyl bzw. Phenyloxy, Naphthyloxy und Phenanthrenyloxy und die entsprechenden Carbonyl- und Sulfonylreste;

**Arylamino:** aromatische mono- oder polycyclische Kohlenwasserstoffreste, welche über ein Stickstoffatom an das Gerüst gebunden sind.

**Heteroaryl bzw. Heteroaryloxy, Heteroarylthio, Heteroarylcarbonyl und Hetarylsulfonyl:** aromatische mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können und welche direkt bzw. (Hetaryloxy) über ein Sauerstoffatom (-O-) oder (Hetarylthio) ein Schwefelatom (-S-), (Hetarylcarbonyl) über eine Carbonylgruppe (-CO-) oder (Hetarylsulfonyl) über eine Sulfonylgruppe (-SO₂-) an das Gerüst gebunden sind, z.B.
- 5-gliedriges Heteroaryl, enthaltend ein bis drei Stickstoffatome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
- 5-gliedriges Heteroaryl, enthaltend ein bis vier Stickstoffatome oder ein bis drei Stickstoffatome und ein Schwefel- oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
- benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
- über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien- 1,4-diylgruppe verbrückt sein können, wobei diese Ringe über eines der Stickstoffringglieder an das Gerüst gebunden sind;
- 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
- benzokondensiertes 6-gliedriges Heteroaryl, enthaltend ein bis vier Stickstoffatome: 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin,
bzw. die entsprechenden Oxy-, Thio-, Carbonyl- oder Sulfonylgruppen.

**Heteroarylamino:** aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom enthalten können und welche über ein Stickstoffatom an das Gerüst gebunden sind.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome, wie vorstehend genannt, ersetzt sein können.

Die Angabe "gegebenenfalls substituiert" soll zum Ausdruck bringen, daß in den derart charakterisierten Gruppen die Wasserstoffatome zum Teil oder vollständig durch gleiche oder verschiedene beispielsweise derjenigen Gruppen ersetzt sein können, die unter den vorstehend ausgeführten Sammelbegriffen genannt sind.

Im Hinblick auf ihre biologische Wirkung sind Verbindungen der Formel I bevorzugt, in der die Variablen die folgenden Bedeutungen haben:
- X: NOCH₃, CHOCH₃ oder CHCH₃;
- Y: O oder NZ, wobei Z für Wasserstoff oder C₁-C₄-Alkyl steht;
- R¹: Wasserstoff oder C₁-C₄-Alkyl;
- R²: Cyano, Nitro, Halogen, C₁-C₄-Alkyl, Trifluormethyl oder C₁-C₄-Alkoxy;
- m: 0, 1 oder 2, wobei die Reste R² verschieden sein können, wenn m für 2 steht;
- R³: Wasserstoff, Cyano, C₁-C₄-Alkyl, C₁-C₄-Halogenalkyl, C₁-C₄-Alkoxy, Cyclopropyl;
- R⁴: Wasserstoff;
C₁-C₆-Alkyl, C₂-C₆-Alkenyl, C₂-C₆-Alkinyl, wobei diese Reste partiell oder vollständig halogeniert sein und/oder eine bis drei der folgenden Gruppen tragen können: Cyano, Nitro, Hydroxy, Mercapto, Amino, Carboxyl, Aminocarbonyl, Aminothiocarbonyl, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Heteroaryl, Heteroaryloxy, Heteroaryl-C₁-C₄-alkoxy, Heteroarylthio und Heteroaryl-C₁-C₄-alkylthio, wobei die cyclischen Gruppen ihrerseits partiell oder vollständig halogeniert sein und/oder einen bis drei der folgenden Substituenten tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy,
C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio, C₃-C₆-Alkinyloxy, C₁-C₄-Alkylendioxy, welches halogeniert sein kann, und C(=NOR⁵)-Aₙ-R⁶, wobei A für Sauerstoff, Schwefel oder Stickstoff steht und wobei der Stickstoff Wasserstoff oder C₁-C₆-Alkyl trägt, n gleich 0 oder 1 ist, R⁵ Wasserstoff oder C₁-C₆-Alkyl und R⁶ Wasserstoff oder C₁-C₆-Alkyl bedeutet;
C₃-C₆-Cycloalkyl, C₃-C₆-Cycloalkenyl, Heterocyclyl, wobei diese Reste partiell oder vollständig halogeniert sein und/oder eine bis drei der folgenden Gruppen tragen können: Cyano, C₁-C₆-Alkyl, C₁-C₆-Halogenalkyl, C₂-C₆-Alkenyl, C₁-C₆-Alkoxy und C₁-C₆-Alkylthio;
Aryl oder Heteroaryl, wobei diese Reste partiell oder vollständig halogeniert sein und/oder eine bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylcarbonyl. C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Heteroaryl, Heteroaryloxy, Heteroarylthio, C₃-C₆-Alkinyloxy, C₁-C₄-Alkylendioxy, welches halogeniert sein kann, und C(=NOR⁵)-Aₙ-R⁶,
wobei A für Sauerstoff, Schwefel oder Stickstoff steht und der Stickstoff Wasserstoff oder C₁-C₆-Alkyl trägt, n gleich 0 oder 1 ist, R⁵ Wasserstoff oder C₁-C₆-Alkyl und R⁶ Wasserstoff oder C₁-C₆-Alkyl bedeutet
und wobei die cyclischen Gruppen ihrerseits partiell oder vollständig halogeniert sein und/oder einen bis drei der folgenden Substituenten tragen können: Cyano, Nitro, Hydroxy, C₁-C₆-Alkyl, C₁-C₆-Halogenalkyl, C₁-C₆-Alkylcarbonyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyl, C₂-C₆-Alkenyloxy, C₃-C₆-Alkinyloxy und C₁-C₄-Alkylendioxy, welches halogeniert sein kann,
sowie deren Salze.

Weiterhin sind Verbindungen der Formel I bevorzugt, in denen m für 0 steht.

Gleichermaßen bevorzugt sind Verbindungen der Formel I, in denen R¹ für Methyl steht.

Daneben werden Verbindungen I bevorzugt, in denen R³ für Wasserstoff, Cyclopropyl, Methyl, Ethyl, 1-Methylethyl, Trifluormethyl, Cyano oder Methoxy steht.

Besonders bevorzugt sind Verbindungen I, in denen R³ für Methyl steht.

Des weiteren werden Verbindungen I bevorzugt, in denen R³ für Trifluormethyl steht.

Besonders bevorzugt sind auch Verbindungen I, in denen R³ für Methoxy steht.

Des weiteren werden Verbindungen I bevorzugt, in denen R⁴ für gegebenenfalls substituiertes C₁-C₆-Alkyl, C₂-C₆-Alkenyl oder C₂-C₆-Alkinyl steht.

Außerdem werden Verbindungen I bevorzugt, in denen R⁴ für gegebenenfalls substituiertes Heterocyclyl oder gegebenenfalls substituiertes C₃-C₆-Cycloalkenyl steht.

Besonders bevorzugt werden Verbindungen I, in denen R⁴ für C₁-C₄-Alkyl steht.

Außerdem werden Verbindungen I bevorzugt, in denen R⁴ für gegebenenfalls substituiertes C₃-C₆-Cycloalkyl steht.

Des weiteren werden Verbindungen I bevorzugt, in denen R⁴ für gegebenenfalls substituiertes Aryl oder Hetaryl steht.

Des weiteren werden Verbindungen I bevorzugt, in denen R⁴ für ggf. subst. Pyridyl, Pyrimidyl, Pyrazinyl, Pyridazinyl oder Triazinyl steht.

Des weiteren werden Verbindungen I bevorzugt, in denen R⁴ für ggf. subst. Furyl, Thienyl oder Pyrrolyl steht.

Des weiteren werden Verbindungen I bevorzugt, in denen R⁴ für ggf. subst. Oxazolyl, Thiazolyl, Isoxazolyl, Isothiazolyl, Pyrazolyl oder Imidazolyl steht.

Des weiteren werden Verbindungen I bevorzugt, in denen R⁴ für ggf. subst. Oxdiazolyl, Thiadiazolyl oder Triazolyl steht.

Außerdem werden Verbindungen I bevorzugt, in denen R⁴ für Phenyl steht, welches unsubstituiert ist oder eine oder zwei der folgenden Gruppen trägt: Nitro, Cyano, Hydroxy, Amino, Aminocarbonyl, Aminothiocarbonyl, Halogen, C₁-C₄-Alkyl, C₁-C₄-Halogenalkyl, C₃-C₆-Cycloalkyl, C₁-C₄-Alkoxy, C₁-C₄-Halogenalkoxy, C₁-C₄-Alkylamino, C₁-C₄-Alkylcarbonyl, Di-C₁-C₄-Alkylamino, C₁-C₄-Alkylsulfonyl, C₁-C₄-Alkoxycarbonyl, C₁-C₄-Alkylaminocarbonyl, Di-C₁-C₄-Alkylaminocarbonyl, C₁-C₄-Alkylthio, C₂-C₆-Alkenyl, C₂-C₆-Alkenyloxy, C₃-C₆-Alkinyloxy und C₁-C₄-Alkylendioxy, welches halogeniert sein kann.

Ganz besonders werden Verbindungen I bevorzugt, in denen R⁴ für Phenyl steht, welches unsubstituiert ist oder eine oder zwei der folgenden Gruppen trägt: Nitro, Cyano, Halogen, C₁-C₄-Alkyl, C₁-C₄-Halogenalkyl, C₃-C₆-Cycloalkyl, C₁-C₄-Alkoxy, C₁-C₄-Halogenalkoxy und C₁-C₄-Alkylendioxy, welches halogeniert sein kann.

Daneben werden Verbindungen der Formel I bevorzugt, in denen X für NOCH₃ steht.

Daneben werden Verbindungen der Formel I bevorzugt, in denen X für CHOCH₃ steht.

Daneben werden Verbindungen der Formel I bevorzugt, in denen X für CHCH₃ steht.

Des weiteren werden Verbindungen der Formel I bevorzugt, in denen Y für O steht.

Außerdem werden Verbindungen der Formel I bevorzugt, in denen Y für NH steht.

Insbesondere sind im Hinblick auf ihre Verwendung die in den folgenden Tabellen zusammengestellten Verbindungen I bevorzugt.

Den folgenden Tabellen (1 bis 36) liegen die Formeln I.1, I.2, I.3 und I.4 zugrunde.

Den folgenden Tabellen (1 bis 36) liegen die Formeln I.1, I.2, I.3 und I.4 zugrunde.

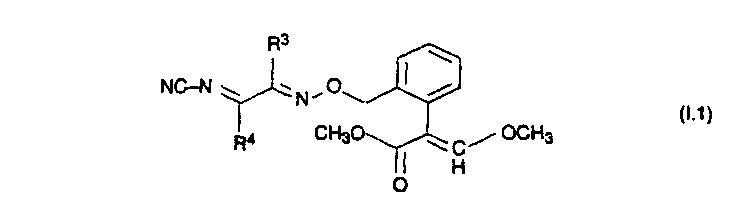

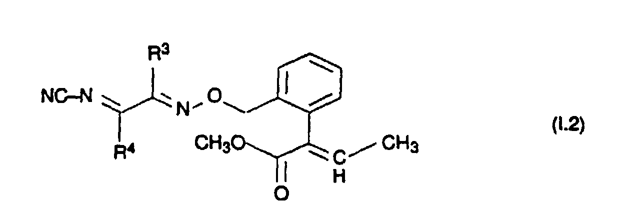

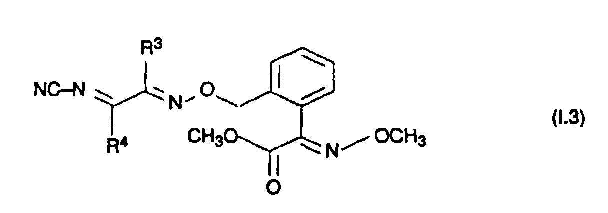

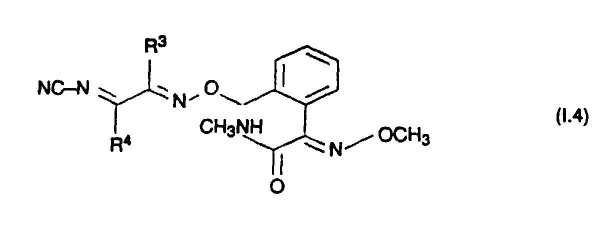

### Tabelle 1

Verbindungen der Formel I.1, mit
R³ = Wasserstoff;
R⁴ = jeweils eine Zeile der Tabelle A.

### Tabelle 2

Verbindungen der Formel I.2, mit
R³ = Wasserstoff;
R⁴ = jeweils eine Zeile der Tabelle A.

### Tabelle 3

Verbindungen der Formel I.3, mit
R³ = Wasserstoff;
R⁴ = jeweils eine Zeile der Tabelle A.

### Tabelle 4

Verbindungen der Formel I.4, mit
R³ = Wasserstoff;
R⁴ = jeweils eine Zeile der Tabelle A.

### Tabelle 5

Verbindungen der Formel I.1, mit
R³ = Methyl;
R⁴ = jeweils eine Zeile der Tabelle A.

### Tabelle 6

Verbindungen der Formel I.2, mit
R³ = Methyl;
R⁴ = jeweils eine Zeile der Tabelle A.

### Tabelle 7

Verbindungen der Formel I.3, mit
R³ = Methyl;
R⁴ = jeweils eine Zeile der Tabelle A.

### Tabelle 8

Verbindungen der Formel I.4, mit
R³ = Methyl;
R⁴ = jeweils eine Zeile der Tabelle A.

### Tabelle 9

Verbindungen der Formel I.1, mit
R³ = Ethyl;
R⁴ = jeweils eine Zeile der Tabelle A.

### Tabelle 10

Verbindungen der Formel I.2, mit
R³ = Ethyl;
R⁴ = jeweils eine Zeile der Tabelle A.

### Tabelle 11

Verbindungen der Formel I.3, mit
R³ = Ethyl;
R⁴ = jeweils eine Zeile der Tabelle A.

### Tabelle 12

Verbindungen der Formel I.4, mit
R³ = Ethyl;
R⁴ = jeweils eine Zeile der Tabelle A.

### Tabelle 13

Verbindungen der Formel I.1, mit
R³ = n-Propyl;
R⁴ = jeweils eine Zeile der Tabelle A.

### Tabelle 14

Verbindungen der Formel I.2, mit
R³ = n-Propyl;
R⁴ = jeweils eine Zeile der Tabelle A.

### Tabelle 15

Verbindungen der Formel I.3, mit
R³ = n-Propyl;
R⁴ = jeweils eine Zeile der Tabelle A.

### Tabelle 16

Verbindungen der Formel I.4, mit
R³ = n-Propyl;
R⁴ = jeweils eine Zeile der Tabelle A.

### Tabelle 17

Verbindungen der Formel I.1, mit
R³ = iso-Propyl;
R⁴ = jeweils eine Zeile der Tabelle A.

### Tabelle 18

Verbindungen der Formel I.2, mit
R³ = iso-Propyl;
R⁴ = jeweils eine Zeile der Tabelle A.

### Tabelle 19

Verbindungen der Formel I.3, mit
R³ = iso-Propyl;
R⁴ = jeweils eine Zeile der Tabelle A.

### Tabelle 20

Verbindungen der Formel I.4, mit
R³ = iso-Propyl;
R⁴ = jeweils eine Zeile der Tabelle A.

### Tabelle 21

Verbindungen der Formel I.1, mit
R³ = Cyclopropyl;
R⁴ = jeweils eine Zeile der Tabelle A.

### Tabelle 22

Verbindungen der Formel I.2, mit
R³ = Cyclopropyl;
R⁴ = jeweils eine Zeile der Tabelle A.

### Tabelle 23

Verbindungen der Formel I.3, mit
R³ = Cyclopropyl;
R⁴ = jeweils eine Zeile der Tabelle A.

### Tabelle 24

Verbindungen der Formel I.4, mit
R³ = Cyclopropyl;
R⁴ = jeweils eine Zeile der Tabelle A.

### Tabelle 25

Verbindungen der Formel I.1, mit
R³ = Trifluormethyl;
R⁴ = jeweils eine Zeile der Tabelle A.

### Tabelle 26

Verbindungen der Formel I.2, mit
R³ = Trifluormethyl;
R⁴ = jeweils eine Zeile der Tabelle A.

### Tabelle 27

Verbindungen der Formel I.3, mit
R³ = Trifluormethyl;
R⁴ = jeweils eine Zeile der Tabelle A.

### Tabelle 28

Verbindungen der Formel I.4, mit
R³ = Trifluormethyl;
R⁴ = jeweils eine Zeile der Tabelle A.

### Tabelle 29

Verbindungen der Formel I.1, mit
R³ = Cyano;
R⁴ = jeweils eine Zeile der Tabelle A.

### Tabelle 30

Verbindungen der Formel I.2, mit
R³ = Cyano;
R⁴ = jeweils eine Zeile der Tabelle A.

### Tabelle 31

Verbindungen der Formel I.3, mit
R³ = Cyano;
R⁴ = jeweils eine Zeile der Tabelle A.

### Tabelle 32

Verbindungen der Formel I.4, mit
R³ = Cyano;
R⁴ = jeweils eine Zeile der Tabelle A.

### Tabelle 33

Verbindungen der Formel I.1, mit
R³ = Methoxy;
R⁴ = jeweils eine Zeile der Tabelle A.

### Tabelle 34

Verbindungen der Formel I.2, mit
R³ = Methoxy;
R⁴ = jeweils eine Zeile der Tabelle A.

### Tabelle 35

Verbindungen der Formel I.3, mit
R³ = Methoxy;
R⁴ = jeweils eine Zeile der Tabelle A.

### Tabelle 36

Verbindungen der Formel I.4, mit
R³ = Methoxy;
R⁴ = jeweils eine Zeile der Tabelle A.

**Tabelle A**

| Nr. | R⁵ |
|---|---|
| 1 | H |
| 2 | CH₃ |
| 3 | C₂H₅ |
| 4 | n-C₃H₇ |
| 5 | i-C₃H₇ |
| 6 | Cyclopropyl |
| 7 | n-C₄H₉ |
| 8 | s-C₄H₉ |
| 9 | i-C₄H₉ |
| 10 | t-C₄H₉ |
| 11 | n-C₅H₁₁ |
| 12 | i-C₅H₁₁ |
| 13 | neo-C₅H₁₁ |
| 14 | Cyclopentyl |
| 15 | n-C₆H₁₃ |
| 16 | Cyclohexyl |
| 17 | Cyclobutyl |
| 18 | CH₂CH₂Cl |
| 19 | (CH₂)₄Cl |
| 20 | CH₂CN |
| 21 | CH₂CH₂CN |
| 22 | (CH₂)₃CN |
| 23 | (CH₂)₄CN |
| 24 | (CH₂)₆CN |
| 25 | Cyclohexylmethyl |
| 26 | 2-Cyclohexyleth-1-yl |
| 27 | Cyclopropylmethyl |
| 28 | 2-Cyclopropyleth-1-yl |
| 29 | 2-Methoxyeth-1-yl |
| 30 | 2-Ethoxyeth-1-yl |
| 31 | 2-Isopropoxyeth-1-yl |
| 32 | 3-Methoxyprop-1-yl |
| 33 | 3-Ethoxyprop-1-yl |
| 34 | 3-Isopropoxyprop-1-yl |
| 35 | 4-Methoxybut-1-yl |
| 36 | 4-Isopropoxybut-1-yl |
| 37 | Prop-2-en-1-yl |
| 38 | But-2-en-1-yl |
| 39 | 3-Methylbut-2-en-1-yl |
| 40 | 2-Vinyloxyeth-1-yl |
| 41 | Allyloxyeth-1-yl |
| 42 | 2-Trifluormethoxyeth-1-yl |
| 43 | 3-Trifluormethoxyprop-1-yl |
| 44 | 4-Difluormethoxybut-1-yl |
| 45 | Hydroxycarbonylmethyl |
| 46 | Methoxycarbonylmethyl |
| 47 | Aminocarbonylmethyl |
| 48 | N-Methylaminocarbonylmethyl |
| 49 | N,N-Dimethylaminocarbonyl-methyl |
| 50 | 2-Hydroxycarbonyleth-1-yl |
| 51 | 2-Methoxycarbonyleth-1-yl |
| 52 | 2-Aminocarbonyleth-1-yl |
| 53 | 2-N-Methylaminocarbonyleth-1-yl |
| 54 | 2-Dimethylaminocarbonyleth-1-yl |
| 55 | 2-Aminoeth-1-yl |
| 56 | 2-Aminoprop-1-yl |
| 57 | 4-Aminobut-1-yl |
| 58 | 3-Dimethylaminoprop-1-yl |
| 59 | 4-Aminothiocarbonylbut-1-yl |
| 60 | E-3-Chlorprop-2-en-1-yl |
| 61 | Z-3-Chlorprop-2-en-1-yl |
| 62 | Prop-2-in-1-yl |
| 63 | But-2-in-1-yl |
| 64 | But-3-in-1-yl |
| 65 | 3-Chlorprop-2-in-1-yl |
| 66 | 6-Aminocarbonylhex-1-yl |
| 67 | 3-Aminothiocarbonylprop-1-yl |
| 68 | 2-Aminothiocarbonyleth-1-yl |
| 69 | Aminothiocarbonylmethyl |
| 70 | 4-(N,N-Dimethylamino)but-1-yl |
| 71 | 2-(Methylthio)eth-1-yl |
| 72 | 2-(Methylsulfonyl)eth-1-yl |
| 73 | 4-(Methylthio)prop-1-yl |
| 74 | 4-(Methylsulfonyl)prop-1-yl |
| 75 | Benyzl |
| 76 | 2-F-C₆H₄-CH₂ |
| 77 | 3-F-C₆H₄-CH₂ |
| 78 | 4-F-C₆H₄-CH₂ |
| 79 | 2,3-F₂-C₆H₃-CH₂ |
| 80 | 2,4-F₂-C₆H₃-CH₂ |
| 81 | 2,5-F₂-C₆H₃-CH₂ |
| 82 | 2,6-F₂-C₆H₃-CH₂ |
| 83 | 3,4-F₂-C₆H₃-CH₂ |
| 84 | 3,5-F₂-C₆H₃-CH₂ |
| 85 | 2-Cl-C₆H₄-CH₂ |
| 86 | 3-Cl-C₆H₄-CH₂ |
| 87 | 4-Cl-C₆H₄-CH₂ |
| 88 | 2,3-Cl₂-C₆H₃-CH₂ |
| 89 | 2,4-Cl₂-C₆H₃-CH₂ |
| 90 | 2,5-Cl₂-C₆H₃-CH₂ |
| 91 | 2,6-Cl₂-C₆H₃-CH₂ |
| 92 | 3,4-Cl₂-C₆H₃-CH₂ |
| 93 | 3,5-Cl₂-C₆H₃-CH₂ |
| 94 | 2,3,4-Cl₃-C₆H₂-CH₂ |
| 95 | 2,3,5-Cl₃-C₆H₂-CH₂ |
| 96 | 2,3,6-Cl₃-C₆H₂-CH₂ |
| 97 | 2,4,5-Cl₃-C₆H₂-CH₂ |
| 98 | 2,4,6-Cl₃-C₆H₂-CH₂ |
| 99 | 3,4,5-Cl₃-C₆H₂-CH₂ |
| 100 | 2-Br-C₆H₄-CH₂ |
| 101 | 3-Br-C₆H₄-CH₂ |
| 102 | 4-Br-C₆H₄-CH₂ |
| 103 | 2,3-Br₂-C₆H₃-CH₂ |
| 104 | 2,4-Br₂-C₆H₃-CH₂ |
| 105 | 2,5-Br₂-C₆H₃-CH₂ |
| 106 | 2,6-Br₂-C₆H₃-CH₂ |
| 107 | 3,4-Br₂-C₆H₃-CH₂ |
| 108 | 3,5-Br₂-C₆H₃-CH₂ |
| 109 | 2-F, 3-Cl-C₆H₃-CH₂ |
| 110 | 2-F, 4-Cl-C₆H₃-CH₂ |
| 111 | 2-F, 5-Cl-C₆H₃-CH₂ |
| 112 | 2-F, 3-Br-C₆H₃-CH₂ |
| 113 | 2-F, 4-Br-C₆H₃-CH₂ |
| 114 | 2-F, 5-Br-C₆H₃-CH₂ |
| 115 | 2-Cl, 3-Br-C₆H₃-CH₂ |
| 116 | 2-Cl, 4-Br-C₆H₃-CH₂ |
| 117 | 2-Cl, 5-Br-C₆H₃-CH₂ |
| 118 | 3-F, 4-Cl-C₆H₃-CH₂ |
| 119 | 3-F, 5-Cl-C₆H₃-CH₂ |
| 120 | 3-F, 6-Cl-C₆H₃-CH₂ |
| 121 | 3-F, 4-Br-C₆H₃-CH₂ |
| 122 | 3-F, 5-Br-C₆H₃-CH₂ |
| 123 | 3-F, 6-Br-C₆H₃-CH₂ |
| 124 | 3-Cl, 4-Br-C₆H₃-CH₂ |
| 125 | 3-Cl, 5-Br-C₆H₃-CH₂ |
| 126 | 3-Cl, 6-Br-C₆H₃-CH₂ |
| 127 | 4-F, 5-Cl-C₆H₃-CH₂ |
| 128 | 4-F, 6-Cl-C₆H₃-CH₂ |
| 129 | 4-F, 5-Br-C₆H₃-CH₂ |
| 130 | 4-F, 6-Br-C₆H₃-CH₂ |
| 131 | 4-Cl, 5-Br-C₆H₃-CH₂ |
| 132 | 5-F, 6-Cl-C₆H₃-CH₂ |
| 133 | 5-F, 6-Br-C₆H₃-CH₂ |
| 134 | 5-Cl, 6-Br-C₆H₃-CH₂ |
| 135 | 3-Br, 4-Cl, 5-Br-C₆H₂-CH₂ |
| 136 | 2-CN-C₆H₄-CH₂ |
| 137 | 3-CN-C₆H₄-CH₂ |
| 138 | 4-CN-C₆H₄-CH₂ |
| 139 | 2-NO₂-C₆H₄-CH₂ |
| 140 | 3-NO₂-C₆H₄-CH₂ |
| 141 | 4-NO₂-C₆H₄-CH₂ |
| 142 | 2-CH₃-C₆H₄-CH₂ |
| 143 | 3-CH₃-C₆H₄-CH₂ |
| 144 | 4-CH₃-C₆H₄-CH₂ |
| 145 | 2,3-(CH₃)₂-C₆H₃-CH₂ |
| 146 | 2,4-(CH₃)₂-C₆H₃-CH₂ |
| 147 | 2,5-(CH₃)₂-C₆H₃-CH₂ |
| 148 | 2,6-(CH₃)₂-C₆H₃-CH₂ |
| 149 | 3,4-(CH₃)₂-C₆H₃-CH₂ |
| 150 | 3,5-(CH₃)₂-C₆H₃-CH₂ |
| 151 | 2-C₂H₅-C₆H₄-CH₂ |
| 152 | 3-C₂H₅-C₆H₄-CH₂ |
| 153 | 4-C₂H₅-C₆H₄-CH₂ |
| 154 | 2-i-C₃H₇-C₆H₄-CH₂ |
| 155 | 3-i-C₃H₇-C₆H₄-CH₂ |
| 156 | 4-i-C₃H₇-C₆H₄-CH₂ |
| 157 | 2-Cyclohexyl-C₆H₄-CH₂ |
| 158 | 3-Cyclohexyl-C₆H₄-CH₂ |
| 159 | 4-Cyclohexyl-C₆H₄-CH₂ |
| 160 | 2-Vinyl-C₆H₄-CH₂ |
| 161 | 3-Vinyl-C₆H₄-CH₂ |
| 162 | 4-Vinyl-C₆H₄-CH₂ |
| 163 | 2-Allyl-C₆H₄-CH₂ |
| 164 | 3-Allyl-C₆H₄-CH₂ |
| 165 | 4-Allyl-C₆H₄-CH₂ |
| 166 | 2-C₆H₅-C₆H₄-CH₂ |
| 167 | 3-C₆H₅-C₆H₄-CH₂ |
| 168 | 4-C₆H₅-C₆H₄-CH₂ |
| 169 | 3-CH₃, 5-t-C₄H₉-C₆H₃-CH₂ |
| 170 | 2-OH-C₆H₄-CH₂ |
| 171 | 3-OH-C₆H₄-CH₂ |
| 172 | 4-OH-C₆H₄-CH₂ |
| 173 | 2-OCH₃-C₆H₄-CH₂ |
| 174 | 3-OCH₃-C₆H₄-CH₂ |
| 175 | 4-OCH₃-C₆H₄-CH₂ |
| 176 | 2,3-(OCH₃)₂-C₆H₃-CH₂ |
| 177 | 2,4-(OCH₃)₂-C₆H₃-CH₂ |
| 178 | 2,5-(OCH₃)₂-C₆H₃-CH₂ |
| 179 | 3,4-(OCH₃)₂-C₆H₃-CH₂ |
| 180 | 3,5-(OCH₃)₂-C₆H₃-CH₂ |
| 181 | 3,4,5-(OCH₃)₃-C₆H₂-CH₂ |
| 182 | 2-OC₂H₅-C₆H₄-CH₂ |
| 183 | 3-OC₂H₅-C₆H₄-CH₂ |
| 184 | 4-OC₂H₅-C₆H₄-CH₂ |
| 185 | 2-O-(n-C₃H₇)-C₆H₄-CH₂ |
| 186 | 3-O-(n-C₃H₇)-C₆H₄-CH₂ |
| 187 | 4-O-(n-C₃H₇)-C₆H₄-CH₂ |
| 188 | 2-O-(i-C₃H₇)-C₆H₄-CH₂ |
| 189 | 3-O-(i-C₃H₇)-C₆H₄-CH₂ |
| 190 | 4-O-(i-C₃H₇)-C₆H₄-CH₂ |
| 191 | 4-O-(n-C₄H₉)-C₆H₄-CH₂ |
| 192 | 3-O-(t-C₄H₉)-C₆H₄-CH₂ |
| 193 | 4-O-(n-C₆H₁₃)-C₆H₄-CH₂ |
| 194 | 2-O-Allyl-C₆H₄-CH₂ |
| 195 | 3-O-Allyl-C₆H₄-CH₂ |
| 196 | 4-O-Allyl-C₆H₄-CH₂ |
| 197 | 2-CF₃-C₆H₄-CH₂ |
| 198 | 3-CF₃-C₆H₄-CH₂ |
| 199 | 4-CF₃-C₆H₄-CH₂ |
| 200 | 2-Acetyl-C₆H₄-CH₂ |
| 201 | 3-Acetyl-C₆H₄-CH₂ |
| 202 | 4-Acetyl-C₆H₄-CH₂ |
| 203 | 2-Methoxycarbonyl-C₆H₄-CH₂ |
| 204 | 3-Methoxycarbonyl-C₆H₄-CH₂ |
| 205 | 4-Methoxycarbonyl-C₆H₄-CH₂ |
| 206 | 2-Aminocarbonyl-C₆H₄-CH₂ |
| 207 | 3-Aminocarbonyl-C₆H₄-CH₂ |
| 208 | 4-Aminocarbonyl-C₆H₄-CH₂ |
| 209 | 2-Dimethylaminocarbonyl-C₆H₄-CH₂ |
| 210 | 3-Dimethylaminocarbonyl-C₆H₄-CH₂ |
| 211 | 4-Dimethylaminocarbonyl-C₆H₄-CH₂ |
| 212 | 2-(N-Methylaminocarbonyl)-C₆H₄-CH₂ |
| 213 | 3-(N-Methylaminocarbonyl)-C₆H₄-CH₂ |
| 214 | 4-(N-Methylaminocarbonyl)-C₆H₄-CH₂ |
| 215 | 2-H₂N-C₆H₄-CH₂ |
| 216 | 3-H₂N-C₆H₄-CH₂ |
| 217 | 4-H₂N-C₆H₄-CH₂ |
| 218 | 2-Aminothiocarbonyl-C₆H₄-CH₂ |
| 219 | 3-Aminothiocarbonyl-C₆H₄-CH₂ |
| 220 | 4-Aminothiocarbonyl-C₆H₄-CH₂ |
| 221 | 2-Methoxyiminomethyl-C₆H₄-CH₂ |
| 222 | 3-Methoxyiminomethyl-C₆H₄-CH₂ |
| 223 | 4-Methoxyiminomethyl-C₆H₄-CH₂ |
| 224 | 3,4-Methylendioxy-C₆H₃-CH₂ |
| 225 | 3,4-Difluormethylendioxy-C₆H₃-CH₂ |
| 226 | 2,3-Methylendioxy-C₆H₃-CH₂ |
| 227 | 2-(1'-Methoxyiminoeth-1'-yl)-C₆H₄-CH₂ |
| 228 | 3-(1'-Methoxyiminoeth-1'-yl)-C₆H₄-CH₂ |
| 229 | 4-(1'-Methoxyiminoeth-1'-yl)-C₆H₄-CH₂ |
| 230 | 2-SCH₃-C₆H₄-CH₂ |
| 231 | 3-SCH₃-C₆H₄-CH₂ |
| 232 | 4-SCH₃-C₆H₄-CH₂ |
| 233 | 2-SO₂CH₃-C₆H₄-CH₂ |
| 234 | 3-SO₂CH₃-C₆H₄-CH₂ |
| 235 | 4-SO₂CH₃-C₆H₄-CH₂ |
| 236 | 2-OCF₃-C₆H₄-CH₂ |
| 237 | 3-OCF₃-C₆H₄-CH₂ |
| 238 | 4-OCF₃-C₆H₄-CH₂ |
| 239 | 2-OCHF₂-C₆H₄-CH₂ |
| 240 | 3-OCHF₂-C₆H₄-CH₂ |
| 241 | 4-OCHF₂-C₆H₄-CH₂ |
| 242 | 3-CF₃, 4-OCF₃-C₆H₃-CH₂ |
| 243 | 1-Naphthyl-CH₂ |
| 244 | 2-Naphthyl-CH₂ |
| 245 | 2-Phenoxyeth-1-yl |
| 246 | 2-(2'-Chlorphenoxy)eth-1-yl |
| 247 | 2-(3'-Chlorphenoxy)eth-1-yl |
| 248 | 2-(4'-Chlorphenoxy)eth-1-yl |
| 249 | 2-(3',5'-Dichlorphenoxy)eth-1-yl |
| 250 | 2-(2'-Cyanophenoxy)eth-1-yl |
| 251 | 2-(3'-Cyanophenoxy)eth-1-yl |
| 252 | 2-(4'-Cyanophenoxy)eth-1-yl |
| 253 | 2-(2'-Methylphenoxy)eth-1-yl |
| 254 | 2-(3'-Methylphenoxy)eth-1-yl |
| 255 | 2-(4'-Methylphenoxy)eth-1-yl |
| 256 | 2-(3'-t-Butylphenoxy)eth-1-yl |
| 257 | 2-(4'-t-Butylphenoxy)eth-1-yl |
| 258 | 2-(2'-Nitrophenoxy)eth-1-yl |
| 259 | 2-(3'-Nitrophenoxy)eth-1-yl |
| 260 | 2-(4'-Nitrophenoxy)eth-1-yl |
| 261 | 2-(2'-Methoxyphenoxy)eth-1-yl |
| 262 | 2-(3'-Methoxyphenoxy)eth-1-yl |
| 263 | 2-(4'-Methoxyphenoxy)eth-1-yl |
| 264 | 2-(2'-Trifluormethylphenoxy)eth-1-yl |
| 265 | 2-(3'-Trifluormethylphenoxy)eth-1-yl |
| 266 | 2-(4'-Trifluormethylphenoxy)eth-1-yl |
| 267 | 2-(2'-Acetylphenoxy)eth-1-yl |
| 268 | 2-(3'-Acetylphenoxy)eth-1-yl |
| 269 | 2-(4'-Acetylphenoxy)eth-1-yl |
| 270 | 2-(2'-Methoxycarbonyl)eth-1-yl |
| 271 | 2-(3'-Methoxycarbonyl)eth-1-yl |
| 272 | 2-(4'-Methoxycarbonyl)eth-1-yl |
| 273 | 2-(2'-Dimethylaminocarbonyl)eth-1-yl |
| 274 | 2-(3'-Dimethylaminocarbonyl)eth-1-yl |
| 275 | 2-(4'-Dimethylaminocarbonyl)eth-1-yl |
| 276 | 2-(2'-Aminothiocarbonyl)eth-1-yl |
| 277 | 2-(3'-Aminothiocarbonyl)eth-1-yl |
| 278 | 2-(4'-Aminothiocarbonyl)eth-1-yl |
| 279 | 2-(2'-Methylsulfonyl)eth-1-yl |
| 280 | 2-(3'-Methylsulfonyl)eth-1-yl |
| 281 | 2-(4'-Methylsulfonyl)eth-1-yl |
| 282 | 3-Phenoxyprop-1-yl |
| 283 | 3-(2'-Chlorphenoxy)prop-1-yl |
| 284 | 3-(3'-Chlorphenoxy)prop-1-yl |
| 285 | 3-(4'-Chlorphenoxy)prop-1-yl |
| 286 | 3-(3',5',Dichlorphenoxy)prop-1-yl |
| 287 | 3-(2'-Cyanophenoxy)prop-1-yl |
| 288 | 3-(3'-Cyanophenoxy)prop-1-yl |
| 289 | 3-(4'-Cyanophenoxy)prop-1-yl |
| 290 | 3-(2'-Methylphenoxy)prop-1-yl |
| 291 | 3-(3'-Methylphenoxy)prop-1-yl |
| 292 | 3-(4'-Methylphenoxy)prop-1-yl |
| 293 | 3-(2'-Methoxyphenoxy)prop-1-yl |
| 294 | 3-(3'-Methoxyphenoxy)prop-1-yl |
| 295 | 3-(4'-Methoxyphenoxy)prop-1-yl |
| 296 | 3-(2'-Trifluormethylphenoxy)prop-1-yl |
| 297 | 3-(3'-Trifluormethylphenoxy)prop-1-yl |
| 298 | 3-(4'-Trifluormethylphenoxy)prop-1-yl |
| 299 | 4-Phenoxybut-1-yl |
| 300 | 2-Phenyleth-1-yl |
| 301 | 2-(2'-Chlorphenyl)eth-1-yl |
| 302 | 2-(3'-Chlorphenyl)eth-1-yl |
| 303 | 2-(4'-Chlorphenyl)eth-1-yl |
| 304 | 2-(3',5'-Dichlorphenyl)eth-1-yl |
| 305 | 2-(2'-Cyanophenyl)eth-1-yl |
| 306 | 2-(3'-Cyanophenyl)eth-1-yl |
| 307 | 2-(4'-Cyanophenyl)eth-1-yl |
| 308 | 2-(2'-Methylphenyl)eth-1-yl |
| 309 | 2-(3'-Methylphenyl)eth-1-yl |
| 310 | 2-(4'-Methylphenyl)eth-1-yl |
| 311 | 2-(2'-Methoxyphenyl)eth-1-yl |
| 312 | 2-(3'-Methoxyphenyl)eth-1-yl |
| 313 | 2-(4'-Methoxyphenyl)eth-1-yl |
| 314 | 2-(2'-Trifluormethylphenyl)eth-1-yl |
| 315 | 2-(3'-Trifluormethylphenyl)eth-1-yl |
| 316 | 2-(4'-Trifluormethylphenyl)eth-1-yl |
| 317 | 3-Phenylprop-1-yl |
| 318 | 3-(2'-Chlorphenyl)prop-1-yl |
| 319 | 3-(3'-Chlorphenyl)prop-1-yl |
| 320 | 3-(4'-Chlorphenyl)prop-1-yl |
| 321 | 3-(2'-Cyanophenyl)prop-1-yl |
| 322 | 3-(3'-Cyanophenyl)prop-1-yl |
| 323 | 3-(4'-Cyanophenyl)prop-1-yl |
| 324 | 3-(2'-Trifluormethylphenyl)prop-1-yl |
| 325 | 4-Phenylbut-1-yl |
| 326 | 4-(4'-Chlorphenyl)but-1-yl |
| 327 | 6-(4'-Chlorphenyl)hex-1-yl |
| 328 | 2-Pyridylmethyl |
| 329 | 3-Pyridylmethyl |
| 330 | 4-Pyridylmethyl |
| 331 | 4-Chlorpyridin-2-ylmethyl |
| 332 | 5-Chlorpyridin-2-ylmethyl |
| 333 | 6-Chlorpyridin-2-ylmethyl |
| 334 | 5-Chlorpyridin-3-ylmethyl |
| 335 | 6-Chlorpyridin-3-ylmethyl |
| 336 | 2-Chlorpyridin-4-ylmethyl |
| 337 | 2-Pyrimidinylmethyl |
| 338 | 4-Chlorpyrimidin-2-ylmethyl |
| 339 | 5-Chlorpyrimidin-2-ylmethyl |
| 340 | 2-Chlorpyrimidin-4-ylmethyl |
| 341 | 6-Chlorpyrimidin-4-ylmethyl |
| 342 | 2-Chlorpyrimidin-5-ylmethyl |
| 343 | 4-Pyridazinylmethyl |
| 344 | 2-Pyrazinylmethyl |
| 345 | 5-Chlorpyrazin-2-ylmethyl |
| 346 | 6-Chlorpyrazin-2-ylmethyl |
| 347 | 3-Pyridazinylmethyl |
| 348 | 6-Chlorpyridazin-3-ylmethyl |
| 349 | 1,3,5-Triazinylmethyl |
| 350 | 2-Furylmethyl |
| 351 | 3-Furylmethyl |
| 352 | 4-Bromfur-2-ylmethyl |
| 353 | 5-Chlorfur-2-ylmethyl |
| 354 | 2-Thienylmethyl |
| 355 | 3-Thienylmethyl |
| 356 | 5-Methylthien-3-ylmethyl |
| 357 | 5-Chlorthien-2-ylmethyl |
| 358 | 2-Chlorthien-4-ylmethyl |
| 359 | 2-Pyrrolylmethyl |
| 360 | 3-Pyrrolylmethyl |
| 361 | 2-Oxazolylmethyl |
| 362 | 4-Methyloxazol-2-ylmethyl |
| 363 | 5-Methyloxazol-2-ylmethyl |
| 364 | 4-Chloroxazol-2-ylmethyl |
| 365 | 5-Chloroxazol-2-ylmethyl |
| 366 | 4-Oxazolylmethyl |
| 367 | 2-Methyloxazol-4-ylmethyl |
| 368 | 5-Methyloxazol-4-ylmethyl |
| 369 | 2-Chloroxazol-4-ylmethyl |
| 370 | 5-Chloroxazol-4-ylmethyl |
| 371 | 5-Oxazolylmethyl |
| 372 | 2-Methyloxazol-5-ylmethyl |
| 373 | 4-Methyloxazol-5-ylmethyl |
| 374 | 2-Chloroxazol-5-ylmethyl |
| 375 | 4-Chloroxazol-5-ylmethyl |
| 376 | 2-Thiazolylmethyl |
| 377 | 4-Methylthiazol-2-ylmethyl |
| 378 | 5-Methylthiazol-2-ylmethyl |
| 379 | 4-Chlorthiazol-2-ylmethyl |
| 380 | 5-Chlorthiazol-2-ylmethyl |
| 381 | 4-Thiazolylmethyl |
| 382 | 2-Methylthiazol-4-ylmethyl |
| 383 | 5-Methylthiazol-4-ylmethyl |
| 384 | 2-Chlorthiazol-4-ylmethyl |
| 385 | 5-Chlorthiazol-4-ylmethyl |
| 386 | 5-Thiazolylmethyl |
| 387 | 2-Methylthiazol-5-ylmethyl |
| 388 | 4-Methylthiazol-5-ylmethyl |
| 389 | 2-Chlorthiazol-5-ylmethyl |
| 390 | 4-Chlorthiazol-5-ylmethyl |
| 391 | 3-Isoxazolylmethyl |
| 392 | 4-Methylisoxazol-3-ylmethyl |
| 393 | 5-Methylisoxazol-3-ylmethyl |
| 394 | 4-Chlorisoxazol-3-ylmethyl |
| 395 | 5-Chlorisoxazol-3-ylmethyl |
| 396 | 4-Isoxazolylmethyl |
| 397 | 3-Methylisoxazol-4-ylmethyl |
| 398 | 5-Methylisoxazol-4-ylmethyl |
| 399 | 3-Chlorisoxazol-4-ylmethyl |
| 400 | 5-chlorisoxazol-4-ylmethyl |
| 401 | 5-Isoxazolylmethyl |
| 402 | 3-Methylisoxazol-5-ylmethyl |
| 403 | 4-Methylisoxazol-5-ylmethyl |
| 404 | 3-Chlorisoxazol-5-ylmethyl |
| 405 | 4-Chlorisoxazol-5-ylmethyl |
| 406 | 3-Isothiazolylmethyl |
| 407 | 4-Methylisothiazol-3-ylmethyl |
| 408 | 5-Methylisothiazol-3-ylmethyl |
| 409 | 4-Chlorisothiazol-3-ylmethyl |
| 410 | 5-Chlorisothiazol-3-ylmethyl |
| 411 | 4-Isothiazolylmethyl |
| 412 | 3-Methylisothiazol-4-ylmethyl |
| 413 | 5-Methylisothiazol-4-ylmethyl |
| 414 | 3-Chlorisothiazol-4-ylmethyl |
| 415 | 5-Chlorisothiazol-4-ylmethyl |
| 416 | 5-Isothiazolylmethyl |
| 417 | 3-Methylisothiazol-5-ylmethyl |
| 418 | 4-Methylisothiazol-5-ylmethyl |
| 419 | 3-Chlorisothiazol-5-ylmethyl |
| 420 | 4-Chlorisothiazol-5-ylmethyl |
| 421 | 4-Imidazolylmethyl |
| 422 | 1-Phenylpyrazol-3-ylmethyl |
| 423 | 1-Methylimidazol-4-ylmethyl |
| 424 | 1-Phenyl-1,2,4-triazol-3-ylmethyl |
| 425 | 1,2,4-Oxadiazol-3-ylmethyl |
| 426 | 5-Chlor-1,2,4-oxadiazol-3-ylmethyl |
| 427 | 5-Methyl-1,2,4-oxadiazol-3-ylmethyl |
| 428 | 5-Trifluormethyl-1,2,4-oxadiazol-3-ylmethyl |
| 429 | 1,3,4-Oxadiazol-2-ylmethyl |
| 430 | 5-Chlor-1,3,4-oxadiazol-2-ylmethyl |
| 431 | 5-Methyl-1,3,4-oxadiazol-2-ylmethyl |
| 432 | 5-Methoxy-1,3,4-oxadiazol-2-ylmethyl |
| 433 | 1,2,4-Thiadiazol-3-ylmethyl |
| 434 | 5-Chlor-1,2,4-thiadiazol-3-ylmethyl |
| 435 | 5-Methyl-1,2,4-thiadiazol-3-ylmethyl |
| 436 | 1,3,4-Thiadiazol-2-ylmethyl |
| 437 | 5-Chlor-1,3,4-thiadiazol-2-ylmethyl |
| 438 | 5-Methyl-1,3,4-thiadiazol-2-ylmethyl |
| 439 | 5-Cyano-1,3,4-thiadiazol-2-ylmethyl |
| 440 | 2-(2'-Pyridinyloxy)eth-1-yl |
| 441 | 2-(3'-Pyridinyloxy)eth-1-yl |
| 442 | 2-(4'-Pyridinyloxy)eth-1-yl |
| 443 | 2-(2'-Pyrimidinyloxy)eth-1-yl |
| 444 | 2-(4'-Pyrimidinyloxy)eth-1-yl |
| 445 | 2-(5'-Pyrimidinyloxy)eth-1-yl |
| 446 | 2-(2'-Pyrazinyloxy)eth-1-yl |
| 447 | 2-(2'-Pyridazinyloxy)eth-1-yl |
| 448 | 2-(3' -Pyridazinyloxy)eth-1-yl |
| 449 | 2-(1',3',5'-Triazinyloxy)eth-1-yl |
| 450 | 2-(5'-Methylisoxazol-3'-yloxy)eth-1-yl |
| 451 | 2-(5'-Chlorisoxazol-3'-yloxy)eth-1-yl |
| 452 | 2-(2'-Methoxythiazol-4'-yloxy)eth-1-yl |
| 453 | 2-(4'-Chloroxazol-2'-yloxy)eth-1-yl |
| 454 | 2-(1'-Phenyl-1'H-1',2',4'-triazol-3'-yloxy)eth-1-yl |
| 455 | 2-(1'-Phenylpyrazol-3'-yloxy)eth-1-yl |
| 456 | C₆H₅ |
| 457 | 2-F-C₆H₄ |
| 458 | 3-F-C₆H₄ |
| 459 | 4-F-C₆H₄ |
| 460 | 2,3-F₂-C₆H₃ |
| 461 | 2,4-F₂-C₆H₃ |
| 462 | 2,5-F₂-C₆H₃ |
| 463 | 2,6-F₂-C₆H₃ |
| 464 | 3,4-F₂-C₆H₃ |
| 465 | 3,5-F₂-C₆H₃ |
| 466 | 2-Cl-C₆H₄ |
| 467 | 3-Cl-C₆H₄ |
| 468 | 4-Cl-C₆H₄ |
| 469 | 2,3-Cl₂-C₆H₃ |
| 470 | 2,4-Cl₂-C₆H₃ |
| 471 | 2,5-Cl₂-C₆H₃ |
| 472 | 2,6-Cl₂-C₆H₃ |
| 473 | 3,4-Cl₂-C₆H₃ |
| 474 | 3,5-Cl₂-C₆H₃ |
| 475 | 2,3,4-Cl₃-C₆H₂ |
| 476 | 2,3,5-Cl₃-C₆H₂ |
| 477 | 2,3,6-Cl₃-C₆H₂ |
| 478 | 2,4,5-Cl₃-C₆H₂ |
| 479 | 2,4,6-Cl₃-C₆H₂ |
| 480 | 3,4,5-Cl₃-C₆H₂ |
| 481 | 2-Br-C₆H₄ |
| 482 | 3-Br-C₆H₄ |
| 483 | 4-Br-C₆H₄ |
| 484 | 2,3-Br₂-C₆H₃ |
| 485 | 2,4-Br₂-C₆H₃ |
| 486 | 2,5-Br₂-C₆H₃ |
| 487 | 2,6-Br₂-C₆H₃ |
| 488 | 3,4-Br₂-C₆H₃ |
| 489 | 3,5-Br₂-C₆H₃ |
| 490 | 2-F, 3-Cl-C₆H₃ |
| 491 | 2-F, 4-Cl-C₆H₃ |
| 492 | 2-F, 5-Cl-C₆H₃ |
| 493 | 2-F, 3-Br-C₆H₃ |
| 494 | 2-F, 4-Br-C₆H₃ |
| 495 | 2-F, 5-Br-C₆H₃ |
| 496 | 2-Cl, 3-Br-C₆H₃ |
| 497 | 2-Cl, 4-Br-C₆H₃ |
| 498 | 2-Cl, 5-Br-C₆H₃ |
| 499 | 3-F, 4-Cl-C₆H₃ |
| 500 | 3-F, 5-Cl-C₆H₃ |
| 501 | 3-F, 6-Cl-C₆H₃ |
| 502 | 3-F, 4-Br-C₆H₃ |
| 503 | 3-F, 5-Br-C₆H₃ |
| 504 | 3-F, 6-Br-C₆H₃ |
| 505 | 3-Cl, 4-Br-C₆H₃ |
| 506 | 3-Cl, 5-Br-C₆H₃ |
| 507 | 3-Cl, 6-Br-C₆H₃ |
| 508 | 4-F, 5-Cl-C₆H₃ |
| 509 | 4-F, 6-Cl-C₆H₃ |
| 510 | 4-F, 5-Br-C₆H₃ |
| 511 | 4-F, 6-Br-C₆H₃ |
| 512 | 4-Cl, 5-Br-C₆H₃ |
| 513 | 5-F, 6-Cl-C₆H₃ |
| 514 | 5-F, 6-Br-C₆H₃ |
| 515 | 5-Cl, 6-Br-C₆H₃ |
| 516 | 3-Br, 4-Cl, 5-Br-C₆H₂ |
| 517 | 2-CN-C₆H₄ |
| 518 | 3-CN-C₆H₄ |
| 519 | 4-CN-C₆H₄ |
| 520 | 2-NO₂-C₆H₄ |
| 521 | 3-NO₂-C₆H₄ |
| 522 | 4-NO₂-C₆H₄ |
| 523 | 2-CH₃-C₆H₄ |
| 524 | 3-CH₃-C₆H₄ |
| 525 | 4-CH₃-C₆H₄ |
| 526 | 2,3-(CH₃)₂-C₆H₃ |
| 527 | 2,4-(CH₃)₂-C₆H₃ |
| 528 | 2,5-(CH₃)₂-C₆H₃ |
| 529 | 2,6-(CH₃)₂-C₆H₃ |
| 530 | 3,4-(CH₃)₂-C₆H₃ |
| 531 | 3,5-(CH₃)₂-C₆H₃ |
| 532 | 2-C₂H₅-C₆H₄ |
| 533 | 3-C₂H₅-C₆H₄ |
| 534 | 4-C₂H₅-C₆H₄ |
| 535 | 2-i-C₃H₇-C₆H₄ |
| 536 | 3-i-C₃H₇-C₆H₄ |
| 537 | 4-i-C₃H₇-C₆H₄ |
| 538 | 3-tert.-C₄H₉-C₆H₄ |
| 539 | 4-tert.-C₄H₉-C₆H₄ |
| 540 | 2-Vinyl-C₆H₄ |
| 541 | 3-Vinyl-C₆H₄ |
| 542 | 4-Vinyl-C₆H₄ |
| 543 | 2-Allyl-C₆H₄ |
| 544 | 3-Allyl-C₆H₄ |
| 545 | 4-Allyl-C₆H₄ |
| 546 | 2-C₆H₅-C₆H₄ |
| 547 | 3-C₆H₅-C₆H₄ |
| 548 | 4-C₆H₅-C₆H₄ |
| 549 | 3-CH₃, 5-tert.-C₄H₉-C₆H₃ |
| 550 | 2-OH-C₆H₄ |
| 551 | 3-OH-C₆H₄ |
| 552 | 4-OH-C₆H₄ |
| 553 | 2-OCH₃-C₆H₄ |
| 554 | 3-OCH₃-C₆H₄ |
| 555 | 4-OCH₃-C₆H₄ |
| 556 | 2,3-(OCH₃)₂-C₆H₃ |
| 557 | 2,4-(OCH₃)₂-C₆H₃ |
| 558 | 2,5-(OCH₃)₂-C₆H₃ |
| 559 | 3,4-(OCH₃)₂-C₆H₃ |
| 560 | 3,5-(OCH₃)₂-C₆H₃ |
| 561 | 3,4,5-(OCH₃)₃-C₆H₂ |
| 562 | 2-OC₂H₅-C₆H₄ |
| 563 | 3-OC₂H₅-C₆H₄ |
| 564 | 4-OC₂H₅-C₆H₄ |
| 565 | 2-O-(n-C₃H₇)-C₆H₄ |
| 566 | 3-O-(n-C₃H₇)-C₆H₄ |
| 567 | 4-O-(n-C₃H₇)-C₆H₄ |
| 568 | 2-O-(i-C₃H₇)-C₆H₄ |
| 569 | 3-O-(i-C₃H₇)-C₆H₄ |
| 570 | 4-O-(i-C₃H₇)-C₆H₄ |
| 571 | 4-O-(n-C₄H₉)-C₆H₄ |
| 572 | 3-O-(t-C₄H₉)-C₆H₄ |
| 573 | 4-O-(t-C₄H₉)-C₆H₄ |
| 574 | 2-O-Allyl-C₆H₄ |
| 575 | 3-O-Allyl-C₆H₄ |
| 576 | 4-O-Allyl-C₆H₄ |
| 577 | 2-CF₃-C₆H₄ |
| 578 | 3-CF₃-C₆H₄ |
| 579 | 4-CF₃-C₆H₄ |
| 580 | 2-Acetyl-C₆H₄ |
| 581 | 3-Acetyl-C₆H₄ |
| 582 | 4-Acetyl-C₆H₄ |
| 583 | 2-Methoxycarbonyl-C₆H₄ |
| 584 | 3-Methoxycarbonyl-C₆H₄ |
| 585 | 4-Methoxycarbonyl-C₆H₄ |
| 586 | 2-Aminocarbonyl-C₆H₄ |
| 587 | 3-Aminocarbonyl-C₆H₄ |
| 588 | 4-Aminocarbonyl-C₆H₄ |
| 589 | 2-Dimethylaminocarbonyl-C₆H₄ |
| 590 | 3-Dimethylaminocarbonyl-C₆H₄ |
| 591 | 4-Dimethylaminocarbonyl-C₆H₄ |
| 592 | 2-(N-Methylaminocarbonyl)-C₆H₄ |
| 593 | 3-(N-Methylaminocarbonyl)-C₆H₄ |
| 594 | 4-(N-Methylaminocarbonyl)-C₆H₄ |
| 595 | 2-H₂N-C₆H₄ |
| 596 | 3-H₂N-C₆H₄ |
| 597 | 4-H₂N-C₆H₄ |
| 598 | 2-Aminothiocarbonyl-C₆H₄ |
| 599 | 3-Aminothiocarbonyl-C₆H₄ |
| 600 | 4-Aminothiocarbonyl-C₆H₄ |
| 601 | 2-Methoxyiminomethyl-C₆H₄ |
| 602 | 3-Methoxyiminomethyl-C₆H₄ |
| 603 | 4-Methoxyiminomethyl-C₆H₄ |
| 604 | 3,4-Methylendioxy-C₆H₃ |
| 605 | 3,4-Difluormethylendioxy-C₆H₃ |
| 606 | 2,3-Methylendioxy-C₆H₃ |
| 607 | 2-(1'-Methoxyiminoeth-1'-yl)-C₆H₄ |
| 608 | 3-(1'-Methoxyiminoeth-1'-yl)-C₆H₄ |
| 609 | 4-(1'-Methoxyiminoeth-1'-yl)-C₆H₄ |
| 610 | 2-SCH₃-C₆H₄ |
| 611 | 3-SCH₃-C₆H₄ |
| 612 | 4-SCH₃-C₆H₄ |
| 613 | 2-SO₂CH₃-C₆H₄ |
| 614 | 3-SO₂CH₃-C₆H₄ |
| 615 | 4-SO₂CH₃-C₆H₄ |
| 616 | 2-OCF₃-C₆H₄ |
| 617 | 3-OCF₃-C₆H₄ |
| 618 | 4-OCF₃-C₆H₄ |
| 619 | 2-OCHF₂-C₆H₄ |
| 620 | 3-OCHF₂-C₆H₄ |
| 621 | 4-OCHF₂-C₆H₄ |
| 622 | 3-CF₃, 4-OCF₃-C₆H₃ |
| 623 | 2-NHCH₃-C₆H₄ |
| 624 | 3-NHCH₃-C₆H₄ |
| 625 | 4-NHCH₃-C₆H₄ |
| 626 | 2-N(CH₃)₂-C₆H₄ |
| 627 | 3-N(CH₃)₂-C₆H₄ |
| 628 | 4-N(CH₃)₂-C₆H₄ |
| 629 | 2-Ethoxycarbonyl-C₆H₄ |
| 630 | 3-Ethoxycarbonyl-C₆H₄ |
| 631 | 4-Ethoxycarbonyl-C₆H₄ |
| 632 | 2-CH₂CH₂F-C₆H₄ |
| 633 | 3-CH₂CH₂F-C₆H₄ |
| 634 | 4-CH₂CH₂F-C₆H₄ |
| 635 | 2-CH₂CF₃-C₆H₄ |
| 636 | 3-CH₂CF₃-C₆H₄ |
| 637 | 4-CH₂CF₃-C₆H₄ |
| 638 | 2-CF₂CHF₂-C₆H₄ |
| 639 | 3-CF₂CHF₂-C₆H₄ |
| 640 | 4-CF₂CHF₂-C₆H₄ |
| 641 | 2-CHF₂-C₆H₄ |
| 642 | 3-CHF₂-C₆H₄ |
| 643 | 4-CHF₂-C₆H₄ |
| 644 | 2-(1'-Oxo-n-prop-1-yl)-C₆H₄ |
| 645 | 3-(1'-Oxo-n-prop-1-yl)-C₆H₄ |
| 646 | 4-(1'-Oxo-n-prop-1-yl)-C₆H₄ |
| 647 | 2-(1'-Oxo-iso-prop-1-yl)-C₆H₄ |
| 648 | 3-(1'-Oxo-iso-prop-1-yl)-C₆H₄ |
| 649 | 4-(1'-Oxo-iso-prop-1-yl)-C₆H₄ |
| 650 | 3-Cyclopropyl-C₆H₄ |
| 651 | 4-Cyclopropyl-C₆H₄ |
| 652 | 4-Cyclohexyl-C₆H₄ |
| 653 | 1-Naphthyl |
| 654 | 2-Naphthyl |
| 655 | 2-Pyridyl |
| 656 | 3-Pyridyl |
| 657 | 4-Pyridyl |
| 658 | 5-CH₃-Pyridin-2-yl |
| 659 | 5-Cl-Pyridin-2-yl |
| 660 | 6-Cl-Pyridin-2-yl |
| 661 | 3,5-Cl₂-Pyridin-2-yl |
| 662 | 6-OCH₃-Pyridin-2-yl |
| 663 | 6-CH₃-Pyridin-2-yl |
| 664 | 6-Cl-Pyridin-3-yl |
| 665 | 6-CH₃-Pyridin-3-yl |
| 666 | 6-OCH₃-Pyridin-3-yl |
| 667 | 2-Pyrimidinyl |
| 668 | 4-OCH₃-Pyrimidin-2-yl |
| 669 | 4-OC₂H₅-Pyrimidin-2-yl |
| 670 | 4-Cl-Pyrimidin-2-yl |
| 671 | 4-CH₃-Pyrimidin-2-yl |
| 672 | 5-CH₃-Pyrimidin-2-yl |
| 673 | 5-Cl-Pyrimidin-2-yl |
| 674 | 5-OCH₃-Pyrimidin-2-yl |
| 675 | 5-OC₂H₅-Pyrimidin-2-yl |
| 676 | 4-Pyrimidinyl |
| 677 | 2-Cl-Pyrimidin-4-yl |
| 678 | 2-OCH₃-pyrimidin-4-yl |
| 679 | 2-CH₃-Pyrimidin-4-yl |
| 680 | 6-Cl-Pyrimidin-4-yl |
| 681 | 6-CH₃-Pyrimidin-4-yl |
| 682 | 6-OCH₃-Pyrimidin-4-yl |
| 683 | 5-Pyrimidinyl |
| 684 | 2-CH₃-Pyrimidin-5-yl |
| 685 | 2-Cl-Pyrimidin-5-yl |
| 686 | 2-OCH₃-Pyrimidin-5-yl |
| 687 | 2-OC₂H₅-Pyrimidin-5-yl |
| 688 | 2-Furyl |
| 689 | 4-C₂H₅-Fur-2-yl |
| 690 | 4-CH₃-Fur-2-yl |
| 691 | 4-Cl-Fur-2-yl |
| 692 | 4-CN-Fur-2-yl |
| 693 | 5-CH₃-Fur-2-yl |
| 694 | 5-Cl-Fur-2-yl |
| 695 | 5-CN-Fur-2-yl |
| 696 | 3-Furyl |
| 697 | 5-CH₃-Fur-3-yl |
| 698 | 5-Cl-Fur-3-yl |
| 699 | 5-CN-Fur-3-yl |
| 700 | 2-Thienyl |
| 701 | 4-CH₃-Thien-2-yl |
| 702 | 4-Cl-Thien-2-yl |
| 703 | 4-CN-Thien-2-yl |
| 704 | 5-CH₃-Thien-2-yl |
| 705 | 5-Cl-Thien-2-yl |
| 706 | 5-CN-Thien-2-yl |
| 707 | 3-Thienyl |
| 708 | 5-CH₃-Thien-3-yl |
| 709 | 5-Cl-Thien-3-yl |
| 710 | 5-CN-Thien-3-yl |
| 711 | 4-CH₃-Thien-3-yl |
| 712 | 5-F-Thien-3-yl |
| 713 | 2-Oxazolyl |
| 714 | 4-CH₃-Oxazol-2-yl |
| 715 | 4-Cl-Oxazol-2-yl |
| 716 | 4-CN-Oxazol-2-yl |
| 717 | 5-CH₃-Oxazol-2-yl |
| 718 | 5-Cl-Oxazol-2-yl |
| 719 | 5-CN-Oxazol-2-yl |
| 720 | 4-Oxazolyl |
| 721 | 2-CH₃-Oxazol-4-yl |
| 722 | 2-Cl-Oxazol-4-yl |
| 723 | 2-CN-Oxazol-4-yl |
| 724 | 5-Oxazolyl |
| 725 | 2-CH₃-Oxazol-5-yl |
| 726 | 2-Cl-Oxazol-5-yl |
| 727 | 2-CN-Oxazol-5-yl |
| 728 | 3-Isoxazolyl |
| 729 | 5-CH₃-Isoxazol-3-yl |
| 730 | 5-Cl-Isoxazol-3-yl |
| 731 | 5-CN-Isoxazol-3-yl |
| 732 | 5-Isoxxazolyl |
| 733 | 3-CH₃-Isoxazol-5-yl |
| 734 | 3-Cl-Isoxazol-5-yl |
| 735 | 3-CN-Isoxazol-5-yl |
| 736 | 2-Thiazolyl |
| 737 | 4-CH₃-Thiazol-2-yl |
| 738 | 4-Cl-Thiazol-2-yl |
| 739 | 4-CN-Thiazol-2-yl |
| 740 | 5-CH₃-Thiazol-2-yl |
| 741 | 5-Cl-Thiazol-2-yl |
| 742 | 5-CN-Thiazol-2-yl |
| 743 | 4-Thiazolyl |
| 744 | 2-CH₃-Thiazol-4-yl |
| 745 | 2-Cl-Thiazol-4-yl |
| 746 | 2-CN-Thiazol-4-yl |
| 747 | 2-SCH₃-Thiazol-4-yl |
| 748 | 5-Thiazolyl |
| 749 | 2-CH₃-Thiazol-5-yl |
| 750 | 2-Cl-Thiazol-5-yl |
| 751 | 2-CN-Thiazol-5-yl |
| 752 | 3-Isothiazolyl |
| 753 | 5-CH₃-Isothiazol-3-yl |
| 754 | 5-Cl-Isothiazol-3-yl |
| 755 | 5-CN-Isothiazol-3-yl |
| 756 | 5-Isothiazolyl |
| 757 | 3-CH₃-Isothiazol-5-yl |
| 758 | 3-Cl-Isothiazol-5-yl |
| 759 | 3-CN-Isothiazol-5-yl |
| 760 | 2-Imidazolyl |
| 761 | 4-CH₃-Imidazol-2-yl |
| 762 | 4-Cl-Imidazol-2-yl |
| 763 | 4-CN-Imidazol-2-yl |
| 764 | 1-CH₃-Imidazol-2-yl |
| 765 | 1-CH₃, 4-Cl-Imidazol-2-yl |
| 766 | 1,4-(CH₃)₂-Imidazol-2-yl |
| 767 | 1-CH₃, 5-Cl-Imidazol-2-yl |
| 768 | 1,5-(CH₃)₂-Imidazol-2-yl |
| 769 | ₄-Imidazolyl |
| 770 | 2-CH₃-Imidazol-4-yl |
| 771 | 2-Cl-Imidazol-4-yl |
| 772 | 1-CH₃-Imidazol-4-yl |
| 773 | 1,2-(CH₃)₂-Imidazol-4-yl |
| 774 | 1-CH₃, 2-Cl-Imidazol-4-yl |
| 775 | 1-CH₃-Imidazol-5-yl |
| 776 | 1-CH₃, 3-Cl-Imidazol-5-yl |
| 777 | 1,2-(CH₃)₂-Imidazol-5-yl |
| 778 | 3-Pyrazolyl |
| 779 | 5-CH₃-Pyrazol-3-yl |
| 780 | 5-Cl-Pyrazol-3-yl |
| 781 | 5-CN-Pyrazol-3-yl |
| 782 | 1-CH₃-Pyrazol-3-yl |
| 783 | 1-CH₃, 4-Cl-Pyrazol-3-yl |
| 784 | 1-CH₃, 5-Cl-Pyrazol-3-yl |
| 785 | 1,5-(CH₃)₂-Pyrazol-3-yl |
| 786 | 1-CH₃-Pyrazol-5-yl |
| 787 | 1-CH₃, 3-Cl-Pyrazol-5-yl |
| 788 | 1,3-(CH₃)₂-Pyrazol-5-yl |
| 789 | 4-Pyrazolyl |
| 790 | 3-Cl-Pyrazol-4-yl |
| 791 | 3-CH₃-Pyrazol-4-yl |
| 792 | 1-CH₃-Pyrazol-4-yl |
| 793 | 1-CH₃, 3-Cl-Pyrazol-4-yl |
| 794 | 1,3-(CH₃)₂-Pyrazol-4-yl |
| 795 | 1,3,4-Oxadiazol-5-yl |
| 796 | 2-CH₃-1,3,4-Oxadiazol-5-yl |
| 797 | 2-Cl-1,3,4-Oxadiazol-5-yl |
| 798 | 2-CF₃-1,3,4-Oxadiazol-5-yl |
| 799 | 2-i-C₃H₇-1,3,4-Oxadiazol-5-yl |
| 800 | 2-OCH₃-1,3,4-Oxadiazol-5-yl |
| 801 | 1,2,4-Oxadiazol-3-yl |
| 802 | 5-CH₃-1,2,4-Oxadiazol-3-yl |
| 803 | 5-i-C₃H₇-1,2,4-Oxadiazol-3-yl |
| 804 | 5-Cl-1,2,4-Oxadiazol-3-yl |
| 805 | 5-CF₃-1,2,4-Oxadiazol-3-yl |
| 806 | 1,2,4-Triazol-3-yl |
| 807 | 1-CH₃-1,2,4-Triazol-3-yl |
| 808 | 3-Fluorpyridin-2-yl |
| 809 | 3-Chlorpyridin-2-yl |
| 810 | 3-Brompyridin-2-yl |
| 811 | 3-Methylpyridin-2-yl |
| 812 | 3-Trifluormethylpyridin-2-yl |
| 813 | 3-Methoxypyridin-2-yl |
| 814 | 4-Fluorpyridin-2-yl |
| 815 | 4-Chlorpyridin-2-yl |
| 816 | 4-Brompyridin-2-yl |
| 817 | 4-Methylpyridin-2-yl |
| 818 | 4-Trifluormethylpyridín-2-yl |
| 819 | 4-Methoxypyridin-2-yl |
| 820 | 5-Fluorpyridin-2-yl |
| 821 | 5-Brompyridin-2-yl |
| 822 | 6-Trifluormethylpyridin-2-yl |
| 823 | 2-Fluorpyridin-3-yl |
| 824 | 2-Chlorpyridin-3-yl |
| 825 | 2-Brompyridin-3-yl |
| 826 | 2-Methylpyridin-3-yl |
| 827 | 2-Trifluormethylpyridin-3-yl |
| 828 | 3-Methoxypyridin-3-yl |
| 829 | 4-Fluorpyridin-3-yl |
| 830 | 4-Chlorpyridin-3-yl |
| 831 | 4-Brompyridin-3-yl |
| 832 | 4-Methylpyridin-3-yl |
| 833 | 4-Trifluormethylpyridin-3-yl |
| 834 | 4-Methoxypyridin-3-yl |
| 835 | 5-Fluorpyridin-3-yl |
| 836 | 5-Chlorpyridin-3-yl |
| 837 | 5-Brompyridin-3-yl |
| 838 | 5-Methylpyridin-3-yl |
| 839 | 5-Trifluormethylpyridin-3-yl |
| 840 | 5-Methoxypyridin-3-yl |
| 841 | 6-Fluorpyridin-3-yl |
| 842 | 6-Brompyridin-3-yl |
| 843 | 6-Trifluormethylpyridin-3-yl |
| 844 | 2-Fluorpyridin-4-yl |
| 845 | 2-Chlorpyridin-4-yl |
| 846 | 2-Brompyridin-4-yl |
| 847 | 2-Methylpyridin-4-yl |
| 848 | 2-Trifluormethylpyridin-4-yl |
| 849 | 2-Methoxypyridin-4-yl |
| 850 | 3-Fluorpyridin-4-yl |
| 851 | 3-Chlorpyridin-4-yl |
| 852 | 3-Brompyridin-4-yl |
| 853 | 3-Methylpyridin-4-yl |
| 854 | 3-Trifluormethylpyridin-4-yl |
| 855 | 3-Methoxypyridin-4-yl |
| 856 | 4-Fluorpyrimidin-2-yl |
| 857 | 4-Brompyrimidin-2-yl |
| 858 | 4-Trifluormethylpyrimidin-2-yl |
| 859 | 5-Fluorpyrimidin-2-yl |
| 860 | 5-Brompyrimidin-2-yl |
| 861 | 5-Trifluormethylpyrimidin-2-yl |
| 862 | 2-Fluorpyrimidin-4-yl |
| 863 | 2-Brompyrimidin-4-yl |
| 864 | 2-Trifluormethylpyrimidin-4-yl |
| 865 | 2-Trifluormethoxypyrimidin-4-yl |
| 866 | 5-Fluorpyrimidin-4-yl |
| 867 | 5-Chlorpyrimidin-4-yl |
| 868 | 5-Brompyrimidin-4-yl |
| 869 | 5-Methoxypyrimidin-4-yl |
| 870 | 5-Trifluormethylpyrimidin-4-yl |
| 871 | 5-Methoxypyrimidin-4-yl |
| 872 | 6-Fluorpyrimidin-4-yl |
| 873 | 6-Brompyrimidin-4-yl |
| 874 | 6-Trifluormethylpyrimidin-4-yl |
| 875 | 2-Fluorpyrimidin-5-yl |
| 876 | 2-Brompyrimidin-5-yl |
| 877 | 2-Trifluormethylpyrimidin-5-yl |
| 878 | 4-Fluorpyrimidin-5-yl |
| 879 | 4-Chlorpyrimidin-5-yl |
| 880 | 4-Brompyrimidin-5-yl |
| 881 | 4-Methylpyrimidin-5-yl |
| 882 | 4-Trifluormethylpyrimidin-5-yl |
| 883 | 3-Fluor-5-trifluormethylpyridin-2-yl |
| 884 | 3,6-Dichlor-5-trifluormethylpyridin-2-yl |
| 885 | 5,6-Dichlor-3-trifluormethylpyridin-2-yl |
| 886 | 5-Chlor-3-trifluormethylpyridin-2-yl |
| 887 | 3-Chlor-5-trifluormethylpyridin-2-yl |
| 888 | 6-Chlor-4-cyanopyridin-2-yl |
| 889 | 3-Cyano-5-nitropyridin-2-yl |
| 890 | 2-Chlor-6-fluorpyridin-4-yl |
| 891 | 6-Chlor-4-fluorpyridin-2-yl |
| 892 | 4,6-Difluorpyridin-2-yl |
| 893 | 3,5-Dichlor-6-fluorpyridin-2-yl |
| 894 | 6-Methoxy-3-nitropyridin-2-yl |
| 895 | 4-Cyano-6-fluorpyridin-2-yl |
| 896 | 6-Chlor-5-cyanopyridin-2-yl |
| 897 | 6-Chlor-3-cyanopyridin-2-yl |
| 898 | 4-Cyano-3,5,6-trifluorpyridin-2-yl |
| 899 | 6-Chlor-5-nitropyridin-2-yl |
| 900 | 6-Chlor-3-nitropyridin-2-yl |
| 901 | 5-Cyano-6-fluorpyridin-2-yl |
| 902 | 3-Cyano-6-fluorpyridin-2-yl |
| 903 | 4,6-Dicyanopyridin-2-yl |
| 904 | 5-Trichlormethylpyridin-2-yl |
| 905 | 5-Cyanopyridin-2-yl |
| 906 | 5-Brom-4-trifluormethylpyridin-2-yl |
| 907 | 3-Nitro-5-trifluormethylpyridin-2-yl |
| 908 | 5-Aminopyridin-2-yl |
| 909 | 2,3,5,6-Tetrafluorpyridin-4-yl |
| 910 | 5-Nitropyridin-2-yl |
| 911 | 4-Methyl-5-nitroypridin-2-yl |
| 912 | 5-Difluormethylpyridin-2-yl |
| 913 | 5-Fluormethylpyridin-2-yl |
| 914 | 4,6-Difluorpyrimidin-2-yl |
| 915 | 2,6-Difluorpyrimidin-4-yl |
| 916 | 2-Chlor-6-trichlormethylpyrimidin-4-yl |
| 917 | 2,6-Dichlorpyrimidin-4-yl |
| 918 | 5-Methoxycarbonylpyridin-2-yl |
| 919 | 5-Chlor-6-fluorpyridin-2-yl |
| 920 | 5-Chlor-6-hydroxypyridin-2-yl |
| 921 | 5-Chlor-6-methoxypyridin-2-yl |
| 922 | 5-Chlor-6-cyanopyridin-2-yl |
| 923 | 5,6-Dichlorpyridin-2-yl |
| 924 | 6-Brom-5-chlorpyridin-2-yl |
| 925 | 5-Brom-6-fluorpyridin-2-yl |
| 926 | 5-Brom-6-chlorpyridin-2-yl |
| 927 | 5-Brom-6-cyanopyridin-2-yl |
| 928 | 5-Brom-6-hydroxypyridin-2-yl |
| 929 | 5-Brom-6-methoxypyridin-2-yl |
| 930 | 5,6-Dibrompyridin-2-yl |
| 931 | 4-Cyanopyridin-2-yl |
| 932 | 6-Cyanopyridin-2-yl |
| 933 | 4-Chlor-6-methylpyrimidin-2-yl |
| 934 | 2-Choro-6-fluorpyridin-4-yl |
| 935 | 5-Brom-4-trifluormethylpyridin-2-yl |
| 936 | 4,5-Dichlorpyridin-2-yl |
| 937 | 4,5-Dibrompyridin-2-yl |
| 938 | 5,6-Dichlorpyridin-2-yl |
| 939 | 4,6-Dichlorpyridin-2-yl |
| 940 | 4,6-Dibrompyridin-2-yl |
| 941 | 5,6-Dibrompyridin-2-yl |
| 942 | 4-Brom-5-chlorpyridin-2-yl |
| 943 | 6-Brom-5-chlorpyridin-2-yl |
| 944 | 5-Brom-4-chlorpyridin-2-yl |
| 945 | 5-Brom-4-chlorpyridin-2-yl |
| 946 | 6-Brom-4-chlorpyridin-2-yl |
| 947 | 4-Brom-6-chlorpyridin-2-yl |
| 948 | 6-Chlor-4-methoxypyridin-2-yl |
| 949 | 6-Brom-4-methoxypyridin-2-yl |
| 950 | 6-Chlorchinazolin-2-yl |
| 951 | Chinazolin-2-yl |
| 952 | 4-Cyanopyridin-2-yl |
| 953 | 6-Cyanopyridin-2-yl |
| 954 | 5-Hydroxymethylpyridin-2-yl |
| 955 | 6-Chlor-4-trifluormethylpyridin-2-yl |
| 956 | 6-Chlor-4-trifluormethylpyridin-2-yl |
| 957 | 6-Chlor-4-methylpyridin-2-yl |
| 958 | 2,5-Dichlor-6-cyanopyridin-2-yl |
| 959 | 2,5-Dichlor-6-carboxypyridin-2-yl |
| 960 | 2,5-Dichlor-6-methoxycarbonyl-pyridin-2-yl |
| 961 | 6-Trifluormethylpyridin-2-yl |
| 962 | 6-Methoxycarbonylpyridin-2-yl |
| 963 | 6-Carboxypyridin-2-yl |
| 964 | 4-Phenoxypyridin-2-yl |
| 965 | 5-Phenoxypyridin-2-yl |
| 966 | 6-Phenoxypyridin-2-yl |
| 967 | 4-Phenoxypyrimidin-4-yl |
| 968 | 4-(4-Methylphenoxy)pyrimidin-4-yl |
| 969 | 4-Phenoxypyrimidin-2-yl |
| 970 | 4-(2-Fluorphenoxy)-pyrimidin-2-yl |
| 971 | 4-Phenoxypyrimidin-6-yl |
| 972 | 4-(4-Chlorphenoxy)pyrimidin-6-yl |
| 973 | 4-(2-Pyridyloxy)pyrimidin-6-yl |
| 974 | 4-(6-Chlor-2-pyridyloxy)pyrimidin-6-yl |
| 975 | 4-(3-Pyridyloxy)pyrimidin-6-yl |
| 976 | 4-(2-Methyl-3-pyridyloxy)pyrimidin-6-yl |
| 977 | 4-(4-Pyridyloxy)pyrimidin-6-yl |
| 978 | 5-Brom-2-thienyl |
| 979 | 5-Nitro-2-thienyl |
| 980 | 2-Chlor-3-thienyl |
| 981 | 2-Brom-3-thienyl |
| 982 | 1-Methyl-3-pyrrolyl |
| 983 | 1-Methyl-2-pyrrolyl |
| 984 | 1-Benzofuran-2-yl |
| 985 | 1-Benzofuran-3-yl |
| 986 | 1-Benzothiophen-2-yl |
| 987 | 1-Benzothiophen-3-yl |
| 988 | 3-Pyrrolyl |
| 989 | 2-Pyrrolyl |
| 990 | 3-Indolyl |
| 991 | 2-Indolyl |
| 992 | 1-Methyl-3-indolyl |
| 993 | 1-Methyl-2-indolyl |
| 994 | Isoxazol-4-yl |
| 995 | Isothiazol-4-yl |
| 996 | 1,2-Benzisoxazol-3-yl |
| 997 | 1,2-Benzisothiazol-3-yl |
| 998 | 1-Methylindaxol-3-yl |
| 999 | Benzoxazol-2-yl |
| 1000 | 5-Chlorbenzoxazol-2-yl |
| 1001 | 6-Fluorbenzoxazol-2-yl |
| 1002 | Benzthiazol-2-yl |
| 1003 | 5-Fluorbenzthiazol-2-yl |
| 1004 | 6-Fluorbenzthiazol-2-yl |
| 1005 | Pyrido[3,2-d]thiazol-2-yl |
| 1006 | (6-Chlor-pyrido)[3,2-d]thiazol-2-yl |
| 1007 | 1-Methyl-1,2,3-triazol-5-yl |
| 1008 | 1-Methyl-1,2,3-triazol-4-yl |
| 1009 | 1-Methyl-1,2,4-triazol-5-yl |
| 1010 | 1-Methyl-1,2,3,4-tetrazol-5-yl |
| 1011 | 2-Methyl-1,2,3,4-tetrazol-5-yl |
| 1012 | 5-Trifluormethyl-1,3,4-thiadiazol-2-yl |
| 1013 | 6-Chlorbenzoxazol-2-yl |
| 1014 | 5-Fluorbenzoxazol-2-yl |
| 1015 | 5-Nitrothiazol-2-yl |
| 1016 | 1-CH(CH₃)₂-pyrrol-3-yl |
| 1017 | 1-C(CH₃)₃-pyrrol-3-yl |
| 1018 | 1-cyclopropyl-pyrrol-3-yl |
| 1019 | 1-C₆H₅-pyrrol-3-yl |
| 1020 | 1-(2-CH₃-C₆H₄)-pyrrol-3-yl |
| 1021 | 1-(3-CH₃-C₆H₄)-pyrrol-3-yl |
| 1022 | 1-(4-CH₃-C₆H₄)-pyrrol-3-yl |
| 1023 | 1-(3-OCH₃-C₆H₄)-pyrrol-3-yl |
| 1024 | 1-(4-OCH₃-C₆H₄)-pyrrol-3-yl |
| 1025 | 1-(4-NO₂-C₆H₄)-pyrrol-3-yl |
| 1026 | 1-(3-NO₂-C₆N₄)-pyrrol-3-yl |
| 1027 | 1-(4-CN-C₆H₄)-pyrrol-3-yl |
| 1028 | 1-(3-CN-C₆H₄)-pyrrol-3-yl |
| 1029 | 1-(3-CF₃-C₆H₄)-pyrrol-3-yl |
| 1030 | 1-(4-CF₃-C₆H₄)-pyrrol-3-yl |
| 1031 | 1-(4-C(CH₃)₃-C₆H₄)-pyrrol-3-yl |
| 1032 | 1-(2-Cl-C₆H₄)-pyrrol-3-yl |
| 1033 | 1-(3-Cl-C₆H₄)-pyrrol-3-yl |
| 1034 | 1-(4-Cl-C₆H₄)-pyrrol-3-yl |
| 1035 | 1-(2-Br-C₆H₄)-pyrrol-3-yl |
| 1036 | 1-(3-Br-C₆H₄)-pyrrol-3-yl |
| 1037 | 1-(4-Br-C₆H₄)-pyrrol-3-yl |
| 1038 | 1-(2-F-C₆H₄)-pyrrol-3-yl |
| 1039 | 1-(3-F-C₆H₄)-pyrrol-3-yl |
| 1040 | 1-(4-F-C₆H₄)-pyrrol-3-yl |
| 1041 | 1-(2,4-Cl₂-C₆H₃)-pyrrol-3-yl |
| 1042 | 1-(2,5-Cl₂-C₆H₃)-pyrrol-3-yl |
| 1043 | 1-(2,6-Cl₂-C₆H₃)-pyrrol-3-yl |
| 1044 | 1-(3,4-Cl₂-C₆H₃)-pyrrol-3-yl |
| 1045 | 1-(2,4-F₂-C₆H₃)-pyrrol-3-yl |
| 1046 | 1-(2,5-F₂-C₆H₃)-pyrrol-3-yl |
| 1047 | 1-(2,6-F₂-C₆H₃)-pyrrol-3-yl |
| 1048 | 1-(3,4-F₂-C₆H₃)-pyrrol-3-yl |
| 1049 | 1-(2-Cl, 5-OCH₃-C₆H₃)-pyrrol-3-yl |
| 1050 | 1-(2-Cl, 5-CH₃-C₆H₃)-pyrrol-3-yl |
| 1051 | 1-(5-Cl, 2-OCH₃-C₆H₃)-pyrrol-3-yl |
| 1052 | 1-(5-Cl, 2-CH₃-C₆H₃)-pyrrol-3-yl |
| 1053 | 1-[2,5-(CH₃)₂-C₆H₃]-pyrrol-3-yl |
| 1054 | 1-CH(CH₃)₂-pyrrol-2-yl |
| 1055 | 1-C(CH₃)₃-pyrrol-2-yl |
| 1056 | 1-cyclopropyl-pyrrol-2-yl |
| 1057 | 1-C₆H₅-pyrrol-2-yl |
| 1058 | 1-(2-CH₃-C₆H₄)-pyrrol-2-yl |
| 1059 | 1-(3-CH₃-C₆H₄)-pyrrol-2-yl |
| 1060 | 1-(4-CH₃-C₆H₄)-pyrrol-2-yl |
| 1061 | 1-(3-OCH₃-C₆H₄)-pyrrol-2-yl |
| 1062 | 1-(4-OCH₃-C₆H₄)-pyrrol-2-yl |
| 1063 | 1-(4-NO₂-C₆H₄)-pyrrol-2-yl |
| 1064 | 1-(3-NO₂-C₆H₄)-pyrrol-2-yl |
| 1065 | 1-(4-CN-C₆H₄)-pyrrol-2-yl |
| 1066 | 1-(3-CN-C₆H₄)-pyrrol-2-yl |
| 1067 | 1-(3-CF₃-C₆H₄)-pyrrol-2-yl |
| 1068 | 1-(4-CF₃-C₆H₄)-pyrrol-2-yl |
| 1069 | 1-(4-C(CH₃)₃-C₆H₄)-pyrrol-2-yl |
| 1070 | 1-(2-Cl-C₆H₄)-pyrrol-2-yl |
| 1071 | 1-(3-Cl-C₆H₄)-pyrrol-2-yl |
| 1072 | 1-(4-Cl-C₆H₄)-pyrrol-2-yl |
| 1073 | 1-(2-Br-C₆H₄)-pyrrol-2-yl |
| 1074 | 1-(3-Br-C₆H₄)-pyrrol-2-yl |
| 1075 | 1-(4-Br-C₆H₄)-pyrrol-2-yl |
| 1076 | 1-(2-F-C₆H₄)-pyrrol-2-yl |
| 1077 | 1-(3-F-C₆H₄)-pyrrol-2-yl |
| 1078 | 1-(4-F-C₆H₄)-pyrrol-2-yl |
| 1079 | 1-(2,4-Cl₂-C₆H₃)-pyrrol-2-yl |
| 1080 | 1-(2,5-Cl₂-C₆H₃)-pyrrol-2-yl |
| 1081 | 1-(2,6-Cl₂-C₆H₃)-pyrrol-2-yl |
| 1082 | 1-(3,4-Cl₂-C₆H₃)-pyrrol-2-yl |
| 1083 | 1-(2,4-F₂-C₆H₃)-pyrrol-2-yl |
| 1084 | 1-(2,5-F₂-C₆H₃)-pyrrol-2-yl |
| 1085 | 1-(2,6-F₂-C₆H₃)-pyrrol-2-yl |
| 1086 | 1-(3,4-F₂-C₆H₃)-pyrrol-2-yl |
| 1087 | 1-(2-Cl, 5-OCH₃-C₆H₃)-pyrrol-2-yl |
| 1088 | 1-(2-Cl, 5-CH₃-C₆H₃)-pyrrol-2-yl |
| 1089 | 1-(5-Cl, 2-OCH₃-C₆H₃)-pyrrol-2-yl |
| 1090 | 1-(5-Cl, 2-CH₃-C₆H₃)-pyrrol-2-yl |
| 1091 | 1-[2,5-(CH₃)₂-C₆H₃]-pyrrol-2-yl |
| 1092 | 5-CH(CH₃)₂-furan-2-yl |
| 1093 | 5-C(CH₃)₃-furan-2-yl |
| 1094 | 5-cyclopropyl-furan-2-yl |
| 1095 | 5-C₆H₅-furan-2-yl |
| 1096 | 5-(2-CH₃-C₆H₄)-furan-2-yl |
| 1097 | 5-(3-CH₃-C₆H₄)-furan-2-yl |
| 1098 | 5-(4-CH₃-C₆H₄)-furan-2-yl |
| 1099 | 5-(3-OCH₃-C₆H₄)-furan-2-yl |
| 1100 | 5-(4-OCH₃-C₆H₄)-furan-2-yl |
| 1101 | 5-(4-NO₂-C₆H₄)-furan-2-yl |
| 1102 | 5-(3-NO₂-C₆H₄)-furan-2-yl |
| 1103 | 5-(4-CN-C₆H₄)-furan-2-yl |
| 1104 | 5-(3-CN-C₆H₄)-furan-2-yl |
| 1105 | 5-(3-CF₃-C₆H₄)-furan-2-yl |
| 1106 | 5-(4-CF₃-C₆H₄)-furan-2-yl |
| 1107 | 5-(4-C(CH₃)₃-C₆H₄)-furan-2-yl |
| 1108 | 5-(4-C₆H₅-C₆H₄)-furan-2-yl |
| 1109 | 5-(2-Cl-C₆H₄)-furan-2-yl |
| 1110 | 5-(3-Cl-C₆H₄)-furan-2-yl |
| 1111 | 5-(4-Cl-C₆H₄)-furan-2-yl |
| 1112 | 5-(2-Br-C₆H₄)-furan-2-yl |
| 1113 | 5-(3-Br-C₆H₄)-furan-2-yl |
| 1114 | 5-(4-Br-C₆H₄)-furan-2-yl |
| 1115 | 5-(2-F-C₆H₄)-furan-2-yl |
| 1116 | 5-(3-F-C₆H₄)-furan-2-yl |
| 1117 | 5-(4-F-C₆H₄)-furan-2-yl |
| 1118 | 5-(2,4-Cl₂-C₆H₃)-furan-2-yl |
| 1119 | 5-(2,5-Cl₂-C₆H₃)-furan-2-yl |
| 1120 | 5-(2,6-Cl₂-C₆H₃)-furan-2-yl |
| 1121 | 5-(3,4-Cl₂-C₆H₃)-furan-2-yl |
| 1122 | 5-(2,4-F₂-C₆H₃)-furan-2-yl |
| 1123 | 5-(2,5-F₂-C₆H₃)-furan-2-yl |
| 1124 | 5-(2,6-F₂-C₆H₃)-furan-2-yl |
| 1125 | 5-(3,4-F₂-C₆H₃)-furan-2-yl |
| 1126 | 5-(2-Cl, 5-OCH₃-C₆H₃)-furan-2-yl |
| 1127 | 5-(2-Cl, 5-CH₃-C₆H₃)-furan-2-yl |
| 1128 | 5-(5-Cl, 2-OCH₃-C₆H₃)-furan-2-yl |
| 1129 | 5-(5-Cl, 2-CH₃-C₆H₃)-furan-2-yl |
| 1130 | 5-[2,5-(CH₃)₂-C₆H₃]-furan-2-yl |
| 1131 | 4-CH(CH₃)₂-furan-2-yl |
| 1132 | 4-C(CH₃)₃-furan-2-yl |
| 1133 | 4-cyclopropyl-furan-2-yl |
| 1134 | 4-C₆H₅-furan-2-yl |
| 1135 | 4-(2-CH₃-C₆H₄)-furan-2-yl |
| 1136 | 4-(3-CH₃-C₆H₄)-furan-2-yl |
| 1137 | 4-(4-CH₃-C₆H₄)-furan-2-yl |
| 1138 | 4-(3-OCH₃-C₆H₄)-furan-2-yl |
| 1139 | 4-(4-OCH₃-C₆H₄)-furan-2-yl |
| 1140 | 4-(4-NO₂-C₆H₄)-furan-2-yl |
| 1141 | 4-(3-NO₂-C₆H₄)-furan-2-yl |
| 1142 | 4-(4-CN-C₆H₄)-furan-2-yl |
| 1143 | 4-(3-CN-C₆H₄)-furan-2-yl |
| 1144 | 4-(3-CF₃-C₆H₄)-furan-2-yl |
| 1145 | 4-(4-CF₃-C₆H₄)-furan-2-yl |
| 1146 | 4-(4-C(CH₃)₃-C₆H₄)-furan-2-yl |
| 1147 | 4-(2-Cl-C₆H₄)-furan-2-yl |
| 1148 | 4-(3-Cl-C₆H₄)-furan-2-yl |
| 1149 | 4-(4-Cl-C₆H₄)-furan-2-yl |
| 1150 | 4-(2-Br-C₆H₄)-furan-2-yl |
| 1151 | 4-(3-Br-C₆H₄)-furan-2-yl |
| 1152 | 4-(4-Br-C₆H₄)-furan-2-yl |
| 1153 | 4-(2-F-C₆H₄)-furan-2-yl |
| 1154 | 4-(3-F-C₆H₄)-furan-2-yl |
| 1155 | 4-(4-F-C₆H₄)-furan-2-yl |
| 1156 | 4-(2,4-Cl₂-C₆H₃)-furan-2-yl |
| 1157 | 4-(2,5-Cl₂-C₆H₃)-furan-2-yl |
| 1158 | 4-(2,6-Cl₂-C₆H₃)-furan-2-yl |
| 1159 | 4-(3,4-Cl₂-C₆H₃)-furan-2-yl |
| 1160 | 4-(2,4-F₂-C₆H₃)-furan-2-yl |
| 1161 | 4-(2,5-F₂-C₆H₃)-furan-2-yl |
| 1162 | 4-(2,6-F₂-C₆H₃)-furan-2-yl |
| 1163 | 4-(3,4-F₂-C₆H₃)-furan-2-yl |
| 1164 | 4-(2-Cl, 5-OCH₃-C₆H₃)-furan-2-yl |
| 1165 | 4-(2-Cl, 5-CH₃-C₆H₃)-furan-2-yl |
| 1166 | 4-(5-Cl, 2-OCH₃-C₆H₃)-furan-2-yl |
| 1167 | 4-(5-Cl, 2-CH₃-C₆H₃)-furan-2-yl |
| 1168 | 4-[2,5-(CH₃)₂-C₆H₃]-furan-2-yl |
| 1169 | 5-CH(CH₃)₂-thien-2-yl |
| 1170 | 5-C(CH₃)₃-thien-2-yl |
| 1171 | 5-cyclopropyl-thien-2-yl |
| 1172 | 5-C₆H₅-thien-2-yl |
| 1173 | 5-(2-CH₃-C₆H₄)-thien-2-yl |
| 1174 | 5-(3-CH₃-C₆H₄)-thien-2-yl |
| 1175 | 5-(4-CH₃-C₆H₄)-thien-2-yl |
| 1176 | 5-(3-OCH₃-C₆H₄)-thien-2-yl |
| 1177 | 5-(4-OCH₃-C₆H₄)-thien-2-yl |
| 1178 | 5-(4-NO₂-C₆H₄)-thien-2-yl |
| 1179 | 5-(3-NO₂-C₆H₄)-thien-2-yl |
| 1180 | 5-(4-CN-C₆H₄)-thien-2-yl |
| 1181 | 5-(3-CN-C₆H₄)-thien-2-yl |
| 1182 | 5-(3-CF₃-C₆H₄)-thien-2-yl |
| 1183 | 5-(4-CF₃-C₆H₄)-thien-2-yl |
| 1184 | 5-(4-C(CH₃)₃-C₆H₄)-thien-2-yl |
| 1185 | 5-(2-Cl-C₆H₄)-thien-2-yl |
| 1186 | 5-(3-Cl-C₆H₄)-thien-2-yl |
| 1187 | 5-(4-Cl-C₆H₄)-thien-2-yl |
| 1188 | 5-(2-Br-C₆H₄)-thien-2-yl |
| 1189 | 5-(3-Br-C₆H₄)-thien-2-yl |
| 1190 | 5-(4-Br-C₆H₄)-thien-2-yl |
| 1191 | 5-(2-F-C₆H₄)-thien-2-yl |
| 1192 | 5-(3-F-C₆H₄)-thien-2-yl |
| 1193 | 5-(4-F-C₆H₄)-thien-2-yl |
| 1194 | 5-(2,4-Cl₂-C₆H₃)-thien-2-yl |
| 1195 | 5-(2,5-Cl₂-C₆H₃)-thien-2-yl |
| 1196 | 5-(2,6-Cl₂-C₆H₃)-thien-2-yl |
| 1197 | 5-(3,4-Cl₂-C₆H₃)-thien-2-yl |
| 1198 | 5-(2,4-F₂-C₆H₃)-thien-2-yl |
| 1199 | 5-(2,5-F₂-C₆H₃)-thien-2-yl |
| 1200 | 5-(2,6-F₂-C₆H₃)-thien-2-yl |
| 1201 | 5-(3,4-F₂-C₆H₃)-thien-2-yl |
| 1202 | 5-(2-Cl, 5-OCH₃-C₆H₃)-thien-2-yl |
| 1203 | 5-(2-Cl, 5-CH₃-C₆H₃)-thien-2-yl |
| 1204 | 5-(5-Cl, 2-OCN₃-C₆H₃)-thien-2-yl |
| 1205 | 5-(5-Cl, 2-CH₃-C₆H₃)-thien-2-yl |
| 1206 | 5-[2,5-(CH₃)₂-C₆H₃]-thien-2-yl |
| 1207 | 4-CH(CH₃)₂-thien-2-yl |
| 1208 | 4-C(CH₃)₃-thien-2-yl |
| 1209 | 4-cyclopropyl-thien-2-yl |
| 1210 | 4-C₆H₅-thien-2-yl |
| 1211 | 4-(2-CH₃-C₆H₄)-thien-2-yl |
| 1212 | 4-(3-CH₃-C₆H₄)-thien-2-yl |
| 1213 | 4-(4-CH₃-C₆H₄)-thien-2-yl |
| 1214 | 4-(3-OCH₃-C₆H₄)-thien-2-yl |
| 1215 | 4-(4-OCH₃-C₆H₄)-thien-2-yl |
| 1216 | 4-(4-NO₂-C₆H₄)-thien-2-yl |
| 1217 | 4-(3-NO₂-C₆H₄)-thien-2-yl |
| 1218 | 4-(4-CN-C₆H₄)-thien-2-yl |
| 1219 | 4-(3-CN-C₆H₄)-thien-2-yl |
| 1220 | 4-(3-CF₃-C₆H₄)-thien-2-yl |
| 1221 | 4-(4-CF₃-C₆H₄)-thien-2-yl |
| 1222 | 4-(4-C(CH₃)₃-C₆H₄)-thien-2-yl |
| 1223 | 4-(2-Cl-C₆H₄)-thien-2-yl |
| 1224 | 4-(3-Cl-C₆H₄)-thien-2-yl |
| 1225 | 4-(4-Cl-C₆H₄)-thien-2-yl |
| 1226 | 4-(2-Br-C₆H₄)-thien-2-yl |
| 1227 | 4-(3-Br-C₆H₄)-thien-2-yl |
| 1228 | 4-(4-Br-C₆H₄)-thien-2-yl |
| 1229 | 4-(2-F-C₆H₄)-thien-2-yl |
| 1230 | 4-(3-F-C₆H₄)-thien-2-yl |
| 1231 | 4-(4-F-C₆H₄)-thien-2-yl |
| 1232 | 4-(2,4-Cl₂-C₆H₃)-thien-2-yl |
| 1233 | 4-(2,5-Cl₂-C₆H₃)-thien-2-yl |
| 1234 | 4-(2,6-Cl₂-C₆H₃)-thien-2-yl |
| 1235 | 4-(3,4-Cl₂-C₆H₃)-thien-2-yl |
| 1236 | 4- (2,4-F₂-C₆H₃)-thien-2-yl |
| 1237 | 4-(2,5-F₂-C₆H₃)-thien-2-yl |
| 1238 | 4-(2,6-F₂-C₆H₃)-thien-2-yl |
| 1239 | 4-(3,4-F₂-C₆H₃)-thien-2-yl |
| 1240 | 4-(2-Cl, 5-OCH₃-C₆H₃)-thien-2-yl |
| 1241 | 4-(2-Cl, 5-CH₃-C₆H₃)-thien-2-yl |
| 1242 | 4-(5-Cl, 2-OCH₃-C₆H₃)-thien-2-yl |
| 1243 | 4-(5-Cl, 2-CH₃-C₆H₃)-thien-2-yl |
| 1244 | 4-[2,5-(CH₃)₂-C₆H₃]-thien-2-yl |
| 1245 | 2-CH₃-thien-4-yl |
| 1246 | 2-CH(CH₃)₂-thien-4-yl |
| 1247 | 2-C(CH₃)₃-thien-4-yl |
| 1248 | 2-cyclopropyl-thien-4-yl |
| 1249 | 2-C₆H₅-thien-4-yl |
| 1250 | 2-(2-CH₃-C₆H₄)-thien-4-yl |
| 1251 | 2-(3-CH₃-C₆H₄)-thien-4-yl |
| 1252 | 2-(4-CH₃-C₆H₄)-thien-4-yl |
| 1253 | 2-(3-OCH₃-C₆H₄)-thien-4-yl |
| 1254 | 2-(4-OCH₃-C₆H₄)-thien-4-yl |
| 1255 | 2-(4-NO₂-C₆H₄)-thien-4-yl |
| 1256 | 2-(3-NO₂-C₆H₄)-thien-4-yl |
| 1257 | 2-(4-CN-C₆H₄)-thien-4-yl |
| 1258 | 2-(3-CN-C₆H₄)-thien-4-yl |
| 1259 | 2-(3-CF₃-C₆H₄)-thien-4-yl |
| 1260 | 2-(4-CF₃-C₆H₄)-thien-4-yl |
| 1261 | 2-(4-C(CH₃)₃-C₆H₄)-thien-4-yl |
| 1262 | 2- (2-Cl-C₆H₄)-thien-4-yl |
| 1263 | 2-(3-Cl-C₆H₄)-thien-4-yl |
| 1264 | 2-(4-Cl-C₆H₄)-thien-4-yl |
| 1265 | 2-(2-Br-C₆H₄)-thien-4-yl |
| 1266 | 2-(3-Br-C₆H₄)-thien-4-yl |
| 1267 | 2-(4-Br-C₆H₄)-thien-4-yl |
| 1268 | 2-(2-F-C₆H₄)-thien-4-yl |
| 1269 | 2-(3-F-C₆H₄)-thien-4-yl |
| 1270 | 2-(4-F-C₆H₄)-thien-4-yl |
| 1271 | 2-(2,4-Cl₂-C₆H₃)-thien-4-yl |
| 1272 | 2-(2,5-Cl₂-C₆H₃)-thien-4-yl |
| 1273 | 2-(2,6-Cl₂-C₆H₃)-thien-4-yl |
| 1274 | 2-(3,4-Cl₂-C₆H₃)-thien-4-yl |
| 1275 | 2-(2,4-F₂-C₆H₃)-thien-4-yl |
| 1276 | 2-(2,5-F₂-C₆H₃)-thien-4-yl |
| 1277 | 2-(2,6-F₂-C₆H₃)-thien-4-yl |
| 1278 | 2-(3,4-F₂-C₆H₃)-thien-4-yl |
| 1279 | 2-(2-Cl, 5-OCH₃-C₆H₃)-thien-4-yl |
| 1280 | 2-(2-Cl, 5-CH₃-C₆H₃)-thien-4-yl |
| 1281 | 2-(5-Cl, 2-OCH₃-C₆H₃)-thien-4-yl |
| 1282 | 2-(5-Cl, 2-CH₃-C₆H₃)-thien-4-yl |
| 1283 | 2-[2,5-(CH₃)₂-C₆H₃]-thien-4-yl |
| 1284 | 1-CH(CH₃)₂-pyrazol-4-yl |
| 1285 | 1-C(CH₃)₃-pyrazol-4-yl |
| 1286 | 1-cyclopropyl-pyrazol-4-yl |
| 1287 | 1-C₆H₅-pyrazol-4-yl |
| 1288 | 1-(2-CH₃-C₆H₄)-pyrazol-4-yl |
| 1289 | 1-(3-CH₃-C₆H₄)-pyrazol-4-yl |
| 1290 | 1-(4-CH₃-C₆H₄)-pyrazol-4-yl |
| 1291 | 1-(3-OCH₃-C₆H₄)-pyrazol-4-yl |
| 1292 | 1-(4-OCH₃-C₆H₄)-pyrazol-4-yl |
| 1293 | 1-(4-NO₂-C₆H₄)-pyrazol-4-yl |
| 1294 | 1-(3-NO₂-C₆H₄)-pyrazol-4-yl |
| 1295 | 1-(4-CN-C₆H₄)-pyrazol-4-yl |
| 1296 | 1-(3-CN-C₆H₄)-pyrazol-4-yl |
| 1297 | 1-(3-CF₃-C₆H₄)-pyrazol-4-yl |
| 1298 | 1-(4-CF₃-C₆H₄)-pyrazol-4-yl |
| 1299 | 1-(4-C(CH₃)₃-C₆H₄)-pyrazol-4-yl |
| 1300 | 1-(2-Cl-C₆H₄)-pyrazol-4-yl |
| 1301 | 1-(3-Cl-C₆H₄)-pyrazol-4-yl |
| 1302 | 1-(4-Cl-C₆H₄)-pyrazol-4-yl |
| 1303 | 1-(2-Br-C₆H₄)-pyrazol-4-yl |
| 1304 | 1-(3-Br-C₆H₄)-pyrazol-4-yl |
| 1305 | 1-(4-Br-C₆H₄)-pyrazol-4-yl |
| 1306 | 1-(2-F-C₆H₄)-pyrazol-4-yl |
| 1307 | 1-(3-F-C₆H₄)-pyrazol-4-yl |
| 1308 | 1-(4-F-C₆H₄)-pyrazol-4-yl |
| 1309 | 1-(2,4-Cl₂-C₆H₃)-pyrazol-4-yl |
| 1310 | 1-(2,5-Cl₂-C₆H₃)-pyrazol-4-yl |
| 1311 | 1-(2,6-Cl₂-C₆H₃)-pyrazol-4-yl |
| 1312 | 1-(3,4-Cl₂-C₆H₃)-pyrazol-4-yl |
| 1313 | 1-(2,4-F₂-C₆H₃)-pyrazol-4-yl |
| 1314 | 1-(2,5-F₂-C₆H₃)-pyrazol-4-yl |
| 1315 | 1-(2,6-F₂-C₆H₃)-pyrazol-4-yl |
| 1316 | 1-(3,4-F₂-C₆H₃)pyrazol-4-yl |
| 1317 | 1-(2-Cl, 5-OCH₃-C₆H₃)-pyrazol-4-yl |
| 1318 | 1-(2-Cl, 5-CH₃-C₆H₃)-pyrazol-4-yl |
| 1319 | 1-(5-Cl, 2-OCH₃-C₆H₃)-pyrazol-4-yl |
| 1320 | 1-(5-Cl, 2-CH₃-C₆H₃)-pyrazol-4-yl |
| 1321 | 1-[2,5-(CH₃)₂-C₆H₃]-pyrazol-4-yl |
| 1322 | 1-CH(CH₃)₂-pyrazol-3-yl |
| 1323 | 1-C(CH₃)₃-pyrazol-3-yl |
| 1324 | 1-cyclopropyl-pyrazol-3-yl |
| 1325 | 1-C₆H₅-pyrazol-3-yl |
| 1326 | 1-(2-CH₃-C₆H₄)-pyrazol-3-yl |
| 1327 | 1-(3-CH₃-C₆H₄)-pyrazol-3-yl |
| 1328 | 1-(4-CH₃-C₆H₄)-pyrazol-3-yl |
| 1329 | 1-(3-OCH₃-C₆H₄)-pyrazol-3-yl |
| 1330 | 1-(4-OCH₃-C₆H₄)-pyrazol-3-yl |
| 1331 | 1-(4-NO₂-C₆H₄)-pyrazol-3-yl |
| 1332 | 1-(3-NO₂-C₆H₄)-pyrazol-3-yl |
| 1333 | 1-(4-CN-C₆H₄)-pyrazol-3-yl |
| 1334 | 1-(3-CN-C₆H₄)-pyrazol-3-yl |
| 1335 | 1-(3-CF₃-C₆H₄)-pyrazol-3-yl |
| 1336 | 1-(4-CF₃-C₆H₄)-pyrazol-3-yl |
| 1337 | 1-(4-C(CH₃)₃-C₆H₄)-pyrazol-3-yl |
| 1338 | 1-(2-Cl-C₆H₄)-pyrazol-3-yl |
| 1339 | 1-(3-Cl-C₆H₄)-pyrazol-3-yl |
| 1340 | 1-(4-Cl-C₆H₄)-pyrazol-3-yl |
| 1341 | 1-(2-Br-C₆H₄)-pyrazol-3-yl |
| 1342 | 1-(3-Br-C₆H₄)-pyrazol-3-yl |
| 1343 | 1-(4-Br-C₆H₄)-pyrazol-3-yl |
| 1344 | 1-(2-F-C₆H₄)-pyrazol-3-yl |
| 1345 | 1-(3-F-C₆H₄)-pyrazol-3-yl |
| 1346 | 1-(4-F-C₆H₄)-pyrazol-3-yl |
| 1347 | 1-(2,4-Cl₂-C₆H₃)-pyrazol-3-yl |
| 1348 | 1-(2,5-Cl₂-C₆H₃)-pyrazol-3-yl |
| 1349 | 1-(2,6-Cl₂-C₆H₃)-pyrazol-3-yl |
| 1350 | 1-(3,4-Cl₂-C₆H₃)-pyrazol-3-yl |
| 1351 | 1-(2,4-F₂-C₆H₃)-pyrazol-3-yl |
| 1352 | 1-(2,5-F₂-C₆H₃)-pyrazol-3-yl |
| 1353 | 1-(2,6-F₂-C₆H₃)-pyrazol-3-yl |
| 1354 | 1-(3,4-F₂-C₆H₃)-pyrazol-3-yl |
| 1355 | 1-(2-Cl, 5-OCH₃-C₆H₃)-pyrazol-3-yl |
| 1356 | 1-(2-Cl, 5-CH₃-C₆H₃)-pyrazol-3-yl |
| 1357 | 1-(5-Cl, 2-OCH₃-C₆H₃)-pyrazol-3-yl |
| 1358 | 1-(5-Cl, 2-CH₃-C₆H₃)-pyrazol-3-yl |
| 1359 | 1-[2,5-(CH₃)₂-C₆H₃]-pyrazol-3-yl |
| 1360 | 3-CH(CH₃)₂-isoxazol-5-yl |
| 1361 | 3-C(CH₃)₃-isoxazol-5-yl |
| 1362 | 3-cyclopropyl-isoxazol-5-yl |
| 1363 | 3-C₆H₅-isoxazol-5-yl |
| 1364 | 3-(2-CH₃-C₆H₄)-isoxazol-5-yl |
| 1365 | 3-(3-CH₃-C₆H₄)-isoxazol-5-yl |
| 1366 | 3-(4-CH₃-C₆H₄)-isoxazol-5-yl |
| 1367 | 3-(3-OCH₃-C₆H₄)-isoxazol-5-yl |
| 1368 | 3-(4-OCH₃-C₆H₄)-isoxazol-5-yl |
| 1369 | 3-(4-NO₂-C₆H₄)-isoxazol-5-yl |
| 1370 | 3-(3-NO₂-C₆H₄)-isoxazol-5-yl |
| 1371 | 3-(4-CN-C₆H₄)-isoxazol-5-yl |
| 1372 | 3-(3-CN-C₆H₄)-isoxazol-5-yl |
| 1373 | 3-(3-CF₃-C₆H₄)-isoxazol-5-yl |
| 1374 | 3-(4-CF₃-C₆H₄)-isoxazol-5-yl |
| 1375 | 3-(4-C(CH₃)₃-C₆H₄)-isoxazol-5-yl |
| 1376 | 3-(2-Cl-C₆H₄)-isoxazol-5-yl |
| 1377 | 3-(3-Cl-C₆H₄)-isoxazol-5-yl |
| 1378 | 3-(4-Cl-C₆H₄)-isoxazol-5-yl |
| 1379 | 3-(2-Br-C₆H₄)-isoxazol-5-yl |
| 1380 | 3-(3-Br-C₆H₄)-isoxazol-5-yl |
| 1381 | 3-(4-Br-C₆H₄)-isoxazol-5-yl |
| 1382 | 3-(2-F-C₆H₄)-isoxazol-5-yl |
| 1383 | 3-(3-F-C₆H₄)-isoxazol-5-yl |
| 1384 | 3-(4-F-C₆H₄)-isoxazol-5-yl |
| 1385 | 3-(2,4-Cl₂-C₆H₃)-isoxazol-5-yl |
| 1386 | 3-(2,5-Cl₂-C₆H₃)-isoxazol-5-yl |
| 1387 | 3-(2,6-Cl₂-C₆H₃)-isoxazol-5-yl |
| 1388 | 3-(3,4-Cl₂-C₆H₃)-isoxazol-5-yl |
| 1389 | 3-(2,4-F₂-C₆H₃)-isoxazol-5-yl |
| 1390 | 3-(2,5-F₂-C₆H₃)-isoxazol-5-yl |
| 1391 | 3-(2,6-F₂-C₆H₃)-isoxazol-5-yl |
| 1392 | 3-(3,4-F₂-C₆H₃)-isoxazol-5-yl |
| 1393 | 3-(2-Cl, 5-OCH₃-C₆H₃)-isoxazol-5-yl |
| 1394 | 3-(2-Cl, 5-CH₃-C₆H₃)-isoxazol-5-yl |
| 1395 | 3-(5-Cl, 2-OCH₃-C₆H₃)-isoxazol-5-yl |
| 1396 | 3-(5-Cl, 2-CH₃-C₆H₃)-isoxazol-5-yl |
| 1397 | 3-[2,5-(CH₃)₂-C₆H₃]-isoxazol-5-yl |
| 1398 | 5-CH(CH₃)₂-isoxazol-3-yl |
| 1399 | 5-C(CH₃)₃-isoxazol-3-yl |
| 1400 | 5-cyclopropyl-isoxazol-3-yl |
| 1401 | 5-C₆H₅-isoxazol-3-yl |
| 1402 | 5-(2-CH₃-C₆H₄)-isoxazol-3-yl |
| 1403 | 5-(3-CH₃-C₆H₄)-isoxazol-3-yl |
| 1404 | 5-(4-CH₃-C₆H₄)-isoxazol-3-yl |
| 1405 | 5-(3-OCH₃-C₆H₄)-isoxazol-3-yl |
| 1406 | 5-(4-OCH₃-C₆H₄)-isoxazol-3-yl |
| 1407 | 5-(4-NO₂-C₆H₄)-isoxazol-3-yl |
| 1408 | 5-(3-NO₂-C₆H₄)-isoxazol-3-yl |
| 1409 | 5-(4-CN-C₆H₄)-isoxazol-3-yl |
| 1410 | 5-(3-CN-C₆H₄)-isoxazol-3-yl |
| 1411 | 5-(3-CF₃-C₆H₄)-isoxazol-3-yl |
| 1412 | 5-(4-CF₃-C₆H₄)-isoxazol-3-yl |
| 1413 | 5-(4-C(CH₃)₃-C₆H₄)-isoxazol-3-yl |
| 1414 | 5-(2-Cl-C₆H₄)-isoxazol-3-yl |
| 1415 | 5-(3-Cl-C₆H₄)-isoxazol-3-yl |
| 1416 | 5-(4-Cl-C₆H₄)-isoxazol-3-yl |
| 1417 | 5-(2-Br-C₆H₄)-isoxazol-3-yl |
| 1418 | 5-(3-Br-C₆H₄)-isoxazol-3-yl |
| 1419 | 5-(4-Br-C₆H₄)-isoxazol-3-yl |
| 1420 | 5-(2-F-C₆H₄)-isoxazol-3-yl |
| 1421 | 5-(3-F-C₆H₄)-isoxazol-3-yl |
| 1422 | 5-(4-F-C₆H₄)-isoxazol-3-yl |
| 1423 | 5-(2,4-Cl₂-C₆H₃)-isoxazol-3-yl |
| 1424 | 5-(2,5-Cl₂-C₆H₃)-isoxazol-3-yl |
| 1425 | 5-(2,6-Cl₂-C₆H₃)-isoxazol-3-yl |
| 1426 | 5-(3,4-Cl₂-C₆H₃)-isoxazol-3-yl |
| 1427 | 5-(2,4-F₂-C₆H₃)-isoxazol-3-yl |
| 1428 | 5-(2,5-F₂-C₆H₃)-isoxazol-3-yl |
| 1429 | 5- (2,6-F₂-C₆H₃) -isoxazol-3-yl |
| 1430 | 5-(3,4-F₂-C₆H₃)-isoxazol-3-yl |
| 1431 | 5-(2-Cl, 5-OCH₃-C₆H₃)-isoxazol-3-yl |
| 1432 | 5-(2-Cl, 5-CH₃-C₆H₃)-isoxazol-3-yl |
| 1433 | 5-(5-Cl, 2-OCH₃-C₆H₃)-isoxazol-3-yl |
| 1434 | 5-(5-Cl, 2-CH₃-C₆H₃)-isoxazol-3-yl |
| 1435 | 5-(2,5-(CH₃)₂-C₆H₃]-isoxazol-3-yl |
| 1436 | 3-CH(CH₃)₂-isothiazol-5-yl |
| 1437 | 3-C(CH₃)₃-isothiazol-5-yl |
| 1438 | 3-cyclopropyl-isothiazol-5-yl |
| 1439 | 3-C₆H₅-isothiazol-5-yl |
| 1440 | 3-(2-CH₃-C₆H₄)-isothiazol-5-yl |
| 1441 | 3-(3-CH₃-C₆H₄)-isothiazol-5-yl |
| 1442 | 3-(4-CH₃-C₆H₄)-isothiazol-5-yl |
| 1443 | 3-(3-OCH₃-C₆H₄)-isothiazol-5-yl |
| 1444 | 3-(4-OCH₃-C₆H₄)-isothiazol-5-yl |
| 1445 | 3-(4-NO₂-C₆H₄)-isothiazol-5-yl |
| 1446 | 3-(3-NO₂-C₆H₄)-isothiazol-5-yl |
| 1447 | 3-(4-CN-C₆H₄)-isothiazol-5-yl |
| 1448 | 3-(3-CN-C₆H₄)-isothiazol-5-yl |
| 1449 | 3-(3-CF₃-C₆H₄)-isothiazol-5-yl |
| 1450 | 3-(4-CF₃-C₆H₄)-isothiazol-5-yl |
| 1451 | 3-(4-C(CH₃)₃-C₆H₄)-isothiazol-5-yl |
| 1452 | 3-(2-Cl-C₆H₄)-isothiazol-5-yl |
| 1453 | 3-(3-Cl-C₆H₄)-isothiazol-5-yl |
| 1454 | 3-(4-Cl-C₆H₄)-isothiazol-5-yl |
| 1455 | 3-(2-Br-C₆H₄)-isothiazol-5-yl |
| 1456 | 3-(3-Br-C₆H₄)-isothiazol-5-yl |
| 1457 | 3-(4-Br-C₆H₄)-isothiazol-5-yl |
| 1458 | 3-(2-F-C₆H₄)-isothiazol-5-yl |
| 1459 | 3-(3-F-C₆H₄)-isothiazol-5-yl |
| 1460 | 3-(4-F-C₆H₄)-isothiazol-5-yl |
| 1461 | 3-(2,4-Cl₂-C₆H₃)-isothiazol-5-yl |
| 1462 | 3-(2,5-Cl₂-C₆H₃)-isothiazol-5-yl |
| 1463 | 3-(2,6-Cl₂-C₆H₃)-isothiazol-5-yl |
| 1464 | 3-(3,4-Cl₂-C₆H₃)-isothiazol-5-yl |
| 1465 | 3-(2,4-F₂-C₆H₃)-isothiazol-5-yl |
| 1466 | 3-(2,5-F₂-C₆H₃)-isothiazol-5-yl |
| 1467 | 3-(2,6-F₂-C₆H₃)-isothiazol-5-yl |
| 1468 | 3-(3,4-F₂-C₆H₃)-isothiazol-5-yl |
| 1469 | 3-(2-Cl, 5-OCH₃-C₆H₃)-isothiazol-5-yl |
| 1470 | 3-(2-Cl, 5-CH₃-C₆H₃)-isothiazol-5-yl |
| 1471 | 3-(5-Cl, 2-OCH₃-C₆H₃)-isothiazol-5-yl |
| 1472 | 3-(5-Cl, 2-CH₃-C₆H₃)-isothiazol-5-yl |
| 1473 | 3-[2,5-(CH₃)₂-C₆H₃]-isothiazol-5-yl |
| 1474 | 2-CH(CH₃)₂-oxazol-4-yl |
| 1475 | 2-C(CH₃)₃-oxazol-4-yl |
| 1476 | 2-cyclopropyl-oxazol-4-yl |
| 1477 | 2-C₆H₅-oxazol-4-yl |
| 1478 | 2-(2-CH₃-C₆H₄)-oxazol-4-yl |
| 1479 | 2-(3-CH₃-C₆H₄)-oxazol-4-yl |
| 1480 | 2-(4-CH₃-C₆H₄)-oxazol-4-yl |
| 1481 | 2-(3-OCH₃-C₆H₄)-oxazol-4-yl |
| 1482 | 2-(4-OCH₃-C₆H₄)-oxazol-4-yl |
| 1483 | 2-(4-NO₂-C₆H₄)-oxazol-4-yl |
| 1484 | 2-(3-NO₂-C₆H₄)-oxazol-4-yl |
| 1485 | 2-(4-CN-C₆H₄)-oxazol-4-yl |
| 1486 | 2-(3-CN-C₆H₄)-oxazol-4-yl |
| 1487 | 2-(3-CF₃-C₆H₄)-oxazol-4-yl |
| 1488 | 2-(4-CF₃-C₆H₄)-oxazol-4-yl |
| 1489 | 2-(4-C(CH₃)₃-C₆H₄)-oxazol-4-yl |
| 1490 | 2-(2-Cl-C₆H₄)-oxazol-4-yl |
| 1491 | 2-(3-Cl-C₆H₄)-oxazol-4-yl |
| 1492 | 2-(4-Cl-C₆H₄)-oxazol-4-yl |
| 1493 | 2-(2-Br-C₆H₄)-oxazol-4-yl |
| 1494 | 2-(3-Br-C₆H₄)-oxazol-4-yl |
| 1495 | 2-(4-Br-C₆H₄)-oxazol-4-yl |
| 1496 | 2-(2-F-C₆H₄)-oxazol-4-yl |
| 1497 | 2-(3-F-C₆H₄)-oxazol-4-yl |
| 1498 | 2-(4-F-C₆H₄)-oxazol-4-yl |
| 1499 | 2-(2,4-Cl₂-C₆H₃)-oxazol-4-yl |
| 1500 | 2-(2,5-Cl₂-C₆H₃)-oxazol-4-yl |
| 1501 | 2-(2,6-Cl₂-C₆H₃)-oxazol-4-yl |
| 1502 | 2-(3,4-Cl₂-C₆H₃)-oxazol-4-yl |
| 1503 | 2-(2,4-F₂-C₆H₃)-oxazol-4-yl |
| 1504 | 2-(2,5-F₂-C₆H₃)-oxazol-4-yl |
| 1505 | 2-(2,6-F₂-C₆H₃)-oxazol-4-yl |
| 1506 | 2-(3,4-F₂-C₆H₃)-oxazol-4-yl |
| 1507 | 2-(2-Cl, 5-OCH₃-C₆H₃)-oxazol-4-yl |
| 1508 | 2-(2-Cl, 5-CH₃-C₆H₃)-oxazol-4-yl |
| 1509 | 2-(5-Cl, 2-OCH₃-C₆H₃)-oxazol-4-yl |
| 1510 | 2-(5-Cl, 2-CH₃-C₆H₃)-oxazol-4-yl |
| 1511 | 2-[2,5-(CH₃)₂-C₆H₃]-oxazol-4-yl |
| 1512 | 2-CH(CH₃)₂-thiazol-4-yl |
| 1513 | 2-C(CH₃)₃-thiazol-4-yl |
| 1514 | 2-cyclopropyl-thiazol-4-yl |
| 1515 | 2-C₆H₅-thiazol-4-yl |
| 1516 | 2-(2-CH₃-C₆H₄)-thiazol-4-yl |
| 1517 | 2-(3-CH₃-C₆H₄)-thiazol-4-yl |
| 1518 | 2-(4-CH₃-C₆H₄)-thiazol-4-yl |
| 1519 | 2-(3-OCH₃-C₆H₄)-thiazol-4-yl |
| 1520 | 2-(4-OCH₃-C₆H₄)-thiazol-4-yl |
| 1521 | 2-(4-NO₂-C₆H₄)-thiazol-4-yl |
| 1522 | 2-(3-NO₂-C₆H₄)-thiazol-4-yl |
| 1523 | 2-(4-CN-C₆H₄)-thiazol-4-yl |
| 1524 | 2-(3-CN-C₆H₄)-thiazol-4-yl |
| 1525 | 2-(3-CF₃-C₆H₄)-thiazol-4-yl |
| 1526 | 2-(4-CF₃-C₆H₄)-thiazol-4-yl |
| 1527 | 2-(4-C(CH₃)₃-C₆H₄)-thiazol-4-yl |
| 1528 | 2-(2-Cl-C₆H₄)-thiazol-4-yl |
| 1529 | 2-(3-Cl-C₆H₄)-thiazol-4-yl |
| 1530 | 2-(4-Cl-C₆H₄)-thiazol-4-yl |
| 1531 | 2-(2-Br-C₆H₄)-thiazol-4-yl |
| 1532 | 2-(3-Br-C₆H₄)-thiazol-4-yl |
| 1533 | 2-(4-Br-C₆H₄)-thiazol-4-yl |
| 1534 | 2-(2-F-C₆H₄)-thiazol-4-yl |
| 1535 | 2-(3-F-C₆H₄)-thiazol-4-yl |
| 1536 | 2-(4-F-C₆H₄)-thiazol-4-yl |
| 1537 | 2-(2,4-Cl₂-C₆H₃)-thiazol-4-yl |
| 1538 | 2-(2,5-Cl₂-C₆H₃)-thiazol-4-yl |
| 1539 | 2-(2,6-Cl₂-C₆H₃)-thiazol-4-yl |
| 1540 | 2-(3,4-Cl₂-C₆H₃)-thiazol-4-yl |
| 1541 | 2-(2,4-F₂-C₆H₃)-thiazol-4-yl |
| 1542 | 2-(2,5-F₂-C₆H₃)-thiazol-4-yl |
| 1543 | 2-(2,6-F₂-C₆H₃)-thiazol-4-yl |
| 1544 | 2-(3,4-F₂-C₆H₃)-thiazol-4-yl |
| 1545 | 2-(2-Cl, 5-OCH₃-C₆H₃)-thiazol-4-yl |
| 1546 | 2-(2-Cl, 5-CH₃-C₆H₃)-thiazol-4-yl |
| 1547 | 2-(5-Cl, 2-OCH₃-C₆H₃)-thiazol-4-yl |
| 1548 | 2-(5-Cl, 2-CH₃-C₆H₃)-thiazol-4-yl |
| 1549 | 2-[2,5-(CH₃)₂-C₆H₃]-thiazol-4-yl |
| 1550 | 1,3-(CH₃)₂-1,2,4-triazol-5-yl |
| 1551 | 1-CH(CH₃)₂-1,2,4-triazol-3-yl |
| 1552 | 1-C(CH₃)₃-1,2,4-triazol-3-yl |
| 1553 | 1-cyclopropyl-1,2,4-triazol-3-yl |
| 1554 | 1-C₆H₅-1,2,4-triazol-3-yl |
| 1555 | 1-(2-CH₃-C₆H₄)-1,2,4-triazol-3-yl |
| 1556 | 1-(3-CH₃-C₆H₄)-1,2,4-triazol-3-yl |
| 1557 | 1-(4-CH₃-C₆H₄)-1,2,4-triazol-3-yl |
| 1558 | 1-(3-OCH₃-C₆H₄)-1,2,4-triazol-3-yl |
| 1559 | 1-(4-OCH₃-C₆H₄)-1,2,4-triazol-3-yl |
| 1560 | 1-(4-NO₂-C₆H₄)-1,2,4-triazol-3-yl |
| 1561 | 1-(3-NO₂-C₆H₄)-1,2,4-triazol-3-yl |
| 1562 | 1-(4-CN-C₆H₄)-1,2,4-triazol-3-yl |
| 1563 | 1-(3-CN-C₆H₄)-1,2,4-triazol-3-yl |
| 1564 | 1-(3-CF₃-C₆H₄)-1,2,4-triazol-3-yl |
| 1565 | 1-(4-CF₃-C₆H₄)-1,2,4-triazol-3-yl |
| 1566 | 1-(4-C(CH₃)₃-C₆H₄)-1,2,4-triazol-3-yl |
| 1567 | 1-(4-C₆H₅-C₆H₄)-1,2,4-triazol-3-yl |
| 1568 | 1-(2-Cl-C₆H₄)-1,2,4-triazol-3-yl |
| 1569 | 1-(3-Cl-C₆H₄)-1,2,4-triazol-3-yl |
| 1570 | 1-(4-Cl-C₆H₄)-1,2,4-triazol-3-yl |
| 1571 | 1-(2-Br-C₆H₄)-1,2,4-triazol-3-yl |
| 1572 | 1-(3-Br-C₆H₄)-1,2,4-triazol-3-yl |
| 1573 | 1-(4-Br-C₆H₄)-1,2,4-triazol-3-yl |
| 1574 | 1-(2-F-C₆H₄)-1,2,4-triazol-3-yl |
| 1575 | 1-(3-F-C₆H₄)-1,2,4-triazol-3-yl |
| 1576 | 1-(4-F-C₆H₄)-1,2,4-triazol-3-yl |
| 1577 | 1-(2,4-Cl₂-C₆H₃)-1,2,4-triazol-3-yl |
| 1578 | 1-(2,5-Cl₂-C₆H₃)-1,2,4-triazol-3-yl |
| 1579 | 1-(2,6-Cl₂-C₆H₃)-1,2,4-triazol-3-yl |
| 1580 | 1-(3,4-Cl₂-C₆H₃)-1,2,4-triazol-3-yl |
| 1581 | 1-(2,4-F₂-C₆H₃)-1,2,4-triazol -3-yl |
| 1582 | 1-(2,5-F₂-C₆H₃)-1,2,4-triazol-3-yl |
| 1583 | 1-(2,6-F₂-C₆H₃)-1,2,4-triazol-3-yl |
| 1584 | 1-(3,4-F₂-C₆H₃)-1,2,4-triazol-3-yl |
| 1585 | 1-(2-Cl, 5-OCH₃-C₆H₃)-1,2,4-triazol-3-yl |
| 1586 | 1-(2-Cl, 5-CH₃-C₆H₃)-1,2,4-triazol-3-yl |
| 1587 | 1-(5-Cl, 2-OCH₃-C₆H₃)-1,2,4-triazol-3-yl |
| 1588 | 1-(5-Cl, 2-CH₃-C₆H₃)-1,2,4-triazol-3-yl |
| 1589 | 1-[2,5-(CH₃)₂-C₆H₃]-1,2,4-triazol-3-yl |
| 1590 | 5-C(CH₃)₃-1,3,4-oxadiazol-2-yl |
| 1591 | 5-cyclopropyl-1,3,4-oxadiazol-2-yl |
| 1592 | 5-C₆H₅-1,3,4-oxadiazol-2-yl |
| 1593 | 5-(2-CH₃-C₆H₄)-1,3,4-oxadiazol-2-yl |
| 1594 | 5-(3-CH₃-C₆H₄)-1,3,4-oxadiazol-2-yl |
| 1595 | 5-(4-CH₃-C₆H₄)-1,3,4-oxadiazol-2-yl |
| 1596 | 5-(3-OCH₃-C₆H₄)-1,3,4-oxadiazol-2-yl |
| 1597 | 5-(4-OCH₃-C₆H₄)-1,3,4-oxadiazol-2-yl |
| 1598 | 5-(4-NO₂-C₆H₄)-1,3,4-oxadiazol-2-yl |
| 1599 | 5-(3-NO₂-C₆H₄)-1,3,4-oxadiazol-2-yl |
| 1600 | 5-(4-CN-C₆H₄)-1,3,4-oxadiazol-2-yl |
| 1601 | 5-(3-CN-C₆H₄)-1,3,4-oxadiazol-2-yl |
| 1602 | 5-(3-CF₃-C₆H₄)-1,3,4-oxadiazol-2-yl |
| 1603 | 5-(4-CF₃-C₆H₄)-1,3,4-oxadiazol-2-yl |
| 1604 | 5-(4-C(CH₃)₃-C₆H₄)-1,3,4-oxadiazol-2-yl |
| 1605 | 5-(2-Cl-C₆H₄)-1,3,4-oxadiazol-2-yl |
| 1606 | 5-(3-Cl-C₆H₄)-1,3,4-oxadiazol-2-yl |
| 1607 | 5-(4-Cl-C₆H₄)-1,3,4-oxadiazol-2-yl |
| 1608 | 5-(2-Br-C₆H₄)-1,3,4-oxadiazol-2-yl |
| 1609 | 5-(3-Br-C₆H₄)-1,3,4-oxadiazol-2-yl |
| 1610 | 5-(4-Br-C₆H₄)-1,3,4-oxadiazol-2-yl |
| 1611 | 5-(2-F-C₆H₄)-1,3,4-oxadiazol-2-yl |
| 1612 | 5-(3-F-C₆H₄)-1,3,4-oxadiazol-2-yl |
| 1613 | 5-(4-F-C₆H₄)-1,3,4-oxadiazol-2-yl |
| 1614 | 5-(2,4-Cl₂-C₆H₃)-1,3,4-oxadiazol-2-yl |
| 1615 | 5-(2,5-Cl₂-C₆H₃)-1,3,4-oxadiazol-2-yl |
| 1616 | 5-(2,6-Cl₂-C₆H₃)-1,3,4-oxadiazol-2-yl |
| 1617 | 5-(3,4-Cl₂-C₆H₃)-1,3,4-oxadiazol-2-yl |
| 1618 | 5-(2,4-F₂-C₆H₃)-1,3,4-oxadiazol-2-yl |
| 1619 | 5-(2,5-F₂-C₆H₃)-1,3,4-oxadiazol-2-yl |
| 1620 | 5-(2,6-F₂-C₆H₃)-1,3,4-oxadiazol-2-yl |
| 1621 | 5-(3,4-F₂-C₆H₃)-1,3,4-oxadiazol-2-yl |
| 1622 | 5-(2-Cl, 5-OCH₃-C₆H₃)-1,3,4-oxadiazol-2-yl |
| 1623 | 5-(2-Cl, 5-CH₃-C₆H₃)-1,3,4-oxadiazol-2-yl |
| 1624 | 5-(5-Cl, 2-OCH₃-C₆H₃)-1,3,4-oxadiazol-2-yl |
| 1625 | 5-(5-Cl, 2-CH₃-C₆H₃)-1,3,4-oxadiazol-2-yl |
| 1626 | 5-[2,5-(CH₃)₂-C₆H₃]-1,3,4-oxadiazol-2-yl |
| 1627 | 5-C(CH₃)₃-1,2,4-oxadiazol-3-yl |
| 1628 | 5-cyclopropyl-1,2,4-oxadiazol-3-yl |
| 1629 | 5-C₆H₅-1,2,4-oxadiazol-3-yl |
| 1630 | 5-(2-CH₃-C₆H₄)-1,2,4-oxadiazol-3-yl |
| 1631 | 5-(3-CH₃-C₆H₄)-1,2,4-oxadiazol-3-yl |
| 1632 | 5-(4-CH₃-C₆H₄)-1,2,4-oxadiazol-3-yl |
| 1633 | 5-(3-OCH₃-C₆H₄)-1,2,4-oxadiazol-3-yl |
| 1634 | 5-(4-OCH₃-C₆H₄)-1,2,4-oxadiazol-3-yl |
| 1635 | 5-(4-NO₂-C₆H₄)-1,2,4-oxadiazol-3-yl |
| 1636 | 5-(3-NO₂-C₆H₄)-1,2,4-oxadiazol-3-yl |
| 1637 | 5-(4-CN-C₆H₄)-1,2,4-oxadiazol-3-yl |
| 1638 | 5-(3-CN-C₆H₄)-1,2,4-oxadiazol-3-yl |
| 1639 | 5-(3-CF₃-C₆H₄)-1,2,4-oxadiazol-3-yl |
| 1640 | 5-(4-CF₃-C₆H₄)-1,2,4-oxadiazol-3-yl |
| 1641 | 5-(4-C(CH₃)₃-C₆H₄)-1,2,4-oxadiazol-3-yl |
| 1642 | 5-(2-Cl-C₆H₄)-1,2,4-oxadiazol-3-yl |
| 1643 | 5-(3-Cl-C₆H₄)-1,2,4-oxadiazol-3-yl |
| 1644 | 5-(4-Cl-C₆H₄)-1,2,4-oxadiazol-3-yl |
| 1645 | 5-(2-Br-C₆H₄)-1,2,4-oxadiazol-3-yl |
| 1646 | 5-(3-Br-C₆H₄)-1,2,4-oxadiazol-3-yl |
| 1647 | 5-(4-Br-C₆H₄)-1,2,4-oxadiazol-3-yl |
| 1648 | 5-(2-F-C₆H₄)-1,2,4-oxadiazol-3-yl |
| 1649 | 5-(3-F-C₆H₄)-1,2,4-oxadiazol-3-yl |
| 1650 | 5-(4-F-C₆H₄)-1,2,4-oxadiazol-3-yl |
| 1651 | 5-(2,4-Cl₂-C₆H₃)-1,2,4-oxadiazol-3-yl |
| 1652 | 5-(2,5-Cl₂-C₆H₃)-1,2,4-oxadiazol-3-yl |
| 1653 | 5-(2,6-Cl₂-C₆H₃)-1,2,4-oxadiazol-3-yl |
| 1654 | 5-(3,4-Cl₂-C₆H₃)-1,2,4-oxadiazol-3-yl |
| 1655 | 5-(2,4-F₂-C₆H₃)-1,2,4-oxadiazol-3-yl |
| 1656 | 5-(2,5-F₂-C₆H₃)-1,2,4-oxadiazol-3-yl |
| 1657 | 5-(2,6-F₂-C₆H₃)-1,2,4-oxadiazol-3-yl |
| 1658 | 5-(3,4-F₂-C₆H₃)-1,2,4-oxadiazol-3-yl |
| 1659 | 5-(2-Cl, 5-OCH₃-C₆H₃)-1,2,4-oxadiazol-3-yl |
| 1660 | 5-(2-Cl, 5-CH₃-C₆H₃)-1,2,4-oxadiazol-3-yl |
| 1661 | 5-(5-Cl, 2-OCH₃-C₆H₃)-1,2,4-oxadiazol-3-yl |
| 1662 | 5-(5-Cl, 2-CH₃-C₆H₃)-1,2,4-oxadiazol-3-yl |
| 1663 | 5-[2,5-(CH₃)₂-C₆H₃]-1,2,4-oxadiazol-3-yl |
| 1664 | 3-CH₃-1,2,4-oxadiazol-5-yl |
| 1665 | 3-CH(CH₃)₂-1,2,4-oxadiazol-5-yl |
| 1666 | 3-C(CH₃)₃-1,2,4-oxadiazol-5-yl |
| 1667 | 3-cyclopropyl-1,2,4-oxadiazol-5-yl |
| 1668 | 3-C₆H₅-1,2,4-oxadiazol-5-yl |
| 1669 | 3-(2-CH₃-C₆H₄)-1,2,4-oxadiazol-5-yl |
| 1670 | 3-(3-CH₃-C₆H₄)-1,2,4-oxadiazol-5-yl |
| 1671 | 3-(4-CH₃-C₆H₄)-1,2,4-oxadiazol-5-yl |
| 1672 | 3-(3-OCH₃-C₆H₄)-1,2,4-oxadiazol-5-yl |
| 1673 | 3-(4-OCH₃-C₆H₄)-1,2,4-oxadiazol-5-yl |
| 1674 | 3-(4-NO₂-C₆H₄)-1,2,4-oxadiazol-5-yl |
| 1675 | 3-(3-NO₂-C₆H₄)-1,2,4-oxadiazol-5-yl |
| 1676 | 3-(4-CN-C₆H₄)-1,2,4-oxadiazol-5-yl |
| 1677 | 3-(3-CN-C₆H₄)-1,2,4-oxadiazol-5-yl |
| 1678 | 3-(3-CF₃-C₆H₄)-1,2,4-oxadiazol-5-yl |
| 1679 | 3-(4-CF₃-C₆H₄)-1,2,4-oxadiazol-5-yl |
| 1680 | 3-(4-C(CH₃)₃-C₆H₄)-1,2,4-oxadiazol-5-yl |
| 1681 | 3-(2-Cl-C₆H₄)-1,2,4-oxadiazol-5-yl |
| 1682 | 3-(3-Cl-C₆H₄)-1,2,4-oxadiazol-5-yl |
| 1683 | 3-(4-Cl-C₆H₄)-1,2,4-oxadiazol-5-yl |
| 1684 | 3-(2-Br-C₆H₄)-1,2,4-oxadiazol-5-yl |
| 1685 | 3-(3-Br-C₆H₄)-1,2,4-oxadiazol-5-yl |
| 1686 | 3-(4-Br-C₆H₄)-1,2,4-oxadiazol-5-yl |
| 1687 | 3-(2-F-C₆H₄)-1,2,4-oxadiazol-5-yl |
| 1688 | 3-(3-F-C₆H₄)-1,2,4-oxadiazol-5-yl |
| 1689 | 3-(4-F-C₆H₄)-1,2,4-oxadiazol-5-yl |
| 1690 | 3-(2,4-Cl₂-C₆H₃)-1,2,4-oxadiazol-5-yl |
| 1691 | 3-(2,5-Cl₂-C₆H₃)-1,2,4-oxadiazol-5-yl |
| 1692 | 3-(2,6-Cl₂-C₆H₃)-1,2,4-oxadiazol-5-yl |
| 1693 | 3-(3,4-Cl₂-C₆H₃)-1,2,4-oxadiazol-5-yl |
| 1694 | 3-(2,4-F₂-C₆H₃)-1,2,4-oxadiazol-5-yl |
| 1695 | 3-(2,5-F₂-C₆H₃)-1,2,4-oxadiazol-5-yl |
| 1696 | 3-(2,6-F₂-C₆H₃)-1,2,4-oxadiazol-5-yl |
| 1697 | 3-(3,4-F₂-C₆H₃)-1,2,4-oxadiazol-5-yl |
| 1698 | 3-(2-Cl, 5-OCH₃-C₆H₃)-1,2,4-oxadiazol-5-yl |
| 1699 | 3-(2-Cl, 5-CH₃-C₆H₃)-1,2,4-oxadiazol-5-yl |
| 1700 | 3-(5-Cl, 2-OCH₃-C₆H₃)-1,2,4-oxadiazol-5-yl |
| 1701 | 3-(5-Cl, 2-CH₃-C₆H₃)-1,2,4-oxadiazol-5-yl |
| 1702 | 3-[2,5-(CH₃)₂-C₆H₃]-1,2,4-oxadiazol-5-yl |
| 1703 | 5-CH₃-1,2,4-thiadiazol-3-yl |
| 1704 | 5-CH(CH₃)₂-1,2,4-thiadiazol-3-yl |
| 1705 | 5-C(CH₃)₃-1,2,4-thiadiazol-3-yl |
| 1706 | 5-cyclopropyl-1,2,4-thiadiazol-3-yl |
| 1707 | 5-C₆H₅-1,2,4-thiadiazol-3-yl |
| 1708 | 5-(2-CH₃-C₆H₄)-1,2,4-thiadiazol-3-yl |
| 1709 | 5-(3-CH₃-C₆H₄)-1,2,4-thiadiazol-3-yl |
| 1710 | 5-(4-CH₃-C₆H₄)-1,2,4-thiadiazol-3-yl |
| 1711 | 5-(3-OCH₃-C₆H₄)-1,2,4-thiadiazol-3-yl |
| 1712 | 5-(4-OCH₃-C₆H₄)-1,2,4-thiadiazol-3-yl |
| 1713 | 5-(4-NO₂-C₆H₄)-1,2,4-thiadiazol-3-yl |
| 1714 | 5-(3-NO₂-C₆H₄)-1,2,4-thiadiazol-3-yl |
| 1715 | 5-(4-CN-C₆H₄)-1,2,4-thiadiazol-3-yl |
| 1716 | 5-(3-CN-C₆H₄)-1,2,4-thiadiazol-3-yl |
| 1717 | 5-(3-CF₃-C₆H₄)-1,2,4-thiadiazol-3-yl |
| 1718 | 5-(4-CF₃-C₆H₄)-1,2,4-thiadiazol-3-yl |
| 1719 | 5-(4-C(CH₃)₃-C₆H₄)-1,2,4-thiadiazol-3-yl |
| 1720 | 5-(2-Cl-C₆H₄)-1,2,4-thiadiazol-3-yl |
| 1721 | 5-(3-Cl-C₆H₄)-1,2,4-thiadiazol-3-yl |
| 1722 | 5-(4-Cl-C₆H₄)-1,2,4-thiadiazol-3-yl |
| 1723 | 5-(2-Br-C₆H₄)-1,2,4-thiadiazol-3-yl |
| 1724 | 5-(3-Br-C₆H₄)-1,2,4-thiadiazol-3-yl |
| 1725 | 5-(4-Br-C₆H₄)-1,2,9-thiadiazol-3-yl |
| 1726 | 5-(2-F-C₆H₄)-1,2,4-thiadiazol-3-yl |
| 1727 | 5-(3-F-C₆H₄)-1,2,4-thiadiazol-3-yl |
| 1728 | 5-(4-F-C₆H₄)-1,2,4-thiadiazol-3-yl |
| 1729 | 5-(2,4-Cl₂-C₆H₃)-1,2,4-thiadiazol-3-yl |
| 1730 | 5-(2,5-Cl₂-C₆H₃)-1,2,4-thiadiazol-3-yl |
| 1731 | 5-(2,6-Cl₂-C₆H₃)-1,2,4-thiadiazol-3-yl |
| 1732 | 5-(3,4-Cl₂-C₆H₃)-1,2,4-thiadiazol-3-yl |
| 1733 | 5-(2,4-F₂-C₆H₃)-1,2,4-thiadiazol-3-yl |
| 1734 | 5-(2,5-F₂-C₆H₃)-1,2,4-thiadiazol-3-yl |
| 1735 | 5-(2,6-F₂-C₆H₃)-1,2,4-thiadiazol-3-yl |
| 1736 | 5-(3,4-F₂-C₆H₃)-1,2,4-thiadiazol-3-yl |
| 1737 | 5-(2-Cl, 5-OCH₃-C₆H₃)-1,2,4-thiadiazol-3-yl |
| 1738 | 5-(2-Cl, 5-CH₃-C₆H₃)-1,2,4-thiadiazol-3-yl |
| 1739 | 5-(5-Cl, 2-OCH₃-C₆H₃)-1,2,4-thiadiazol-3-yl |
| 1740 | 5-(5-Cl, 2-CH₃-C₆H₃)-1,2,4-thiadiazol-3-yl |
| 1741 | 5-[2,5-(CH₃)₂-C₆H₃]-1,2,4-thiadiazol-3-yl |
| 1742 | 5-CH₃-1,3,4-thiadiazol-2-yl |
| 1743 | 5-CH(CH₃)₂-1,3,4-thiadiazol-2-yl |
| 1744 | 5-C(CH₃)₃-1,3,4-thiadiazol-2-yl |
| 1745 | 5-cyclopropyl-1,3,4-thiadiazol-2-yl |
| 1746 | 5-C₆H₅-1,3,4-thiadiazol-2-yl |
| 1747 | 5-(2-CH₃-C₆H₄)-1,3,4-thiadiazol-2-yl |
| 1748 | 5-(3-CH₃-C₆H₄)-1,3,4-thiadiazol-2-yl |
| 1749 | 5-(4-CH₃-C₆H₄)-1,3,4-thiadiazol-2-yl |
| 1750 | 5-(3-OCH₃-C₆H₄)-1,3,4-thiadiazol-2-yl |
| 1751 | 5-(4-OCH₃-C₆H₄)-1,3,4-thiadiazol-2-yl |
| 1752 | 5-(4-NO₂-C₆H₄)-1,3,4-thiadiazol-2-yl |
| 1753 | 5-(3-NO₂-C₆H₄)-1,3,4-thiadiazol-2-yl |
| 1754 | 5-(4-CN-C₆H₄)-1,3,4-thiadiazol-2-yl |
| 1755 | 5-(3-CN-C₆H₄)-1,3,4-thiadiazol-2-yl |
| 1756 | 5-(3-CF₃-C₆H₄)-1,3,4-thiadiazol-2-yl |
| 1757 | 5-(4-CF₃-C₆H₄)-1,3,4-thiadiazol-2-yl |
| 1758 | 5-(4-C(CH₃)₃-C₆H₄)-1,3,4-thiadiazol-2-yl |
| 1759 | 5-(2-Cl-C₆H₄)-1,3,4-thiadiazol-2-yl |
| 1760 | 5-(3-Cl-C₆H₄)-1,3,4-thiadiazol-2-yl |
| 1761 | 5-(4-Cl-C₆H₄)-1,3,4-thiadiazol-2-yl |
| 1762 | 5-(2-Br-C₆H₄)-1,3,4-thiadiazol-2-yl |
| 1763 | 5-(3-Br-C₆H₄)-1,3,4-thiadiazol-2-yl |
| 1764 | 5-(4-Br-C₆H₄)-1,3,4-thiadiazol-2-yl |
| 1765 | 5-(2-F-C₆H₄)-1,3,4-thiadiazol-2-yl |
| 1766 | 5-(3-F-C₆H₄)-1,3,4-thiadiazol-2-yl |
| 1767 | 5-(4-F-C₆H₄)-1,3,4-thiadiazol-2-yl |
| 1768 | 5-(2,4-Cl₂-C₆H₃)-1,3,4-thiadiazol-2-yl |
| 1769 | 5-(2,5-Cl₂-C₆H₃)-1,3,4-thiadiazol-2-yl |
| 1770 | 5-(2,6-Cl₂-C₆H₃)-1,3,4-thiadiazol-2-yl |
| 1771 | 5-(3,4-Cl₂-C₆H₃)-1,3,4-thiadiazol-2-yl |
| 1772 | 5-(2,4-F₂-C₆H₃)-1,3,4-thiadiazol-2-yl |
| 1773 | 5-(2,5-F₂-C₆H₃)-1,3,4-thiadiazol-2-yl |
| 1774 | 5-(2,6-F₂-C₆H₃)-1,3,4-thiadiazol-2-yl |
| 1775 | 5-(3,4-F₂-C₆H₃)-1,3,4-thiadiazol-2-yl |
| 1776 | 5-(2-Cl, 5-OCH₃-C₆H₃)-1,3,4-thiadiazol-2-yl |
| 1777 | 5-(2-Cl, 5-CH₃-C₆H₃)-1,3,4-thiadiazol-2-yl |
| 1778 | 5-(5-Cl, 2-OCH₃-C₆H₃)-1,3,4-thiadiazol-2-yl |
| 1779 | 5-(5-Cl, 2-CH₃-C₆H₃)-1,3,4-thiadiazol-2-yl |
| 1780 | 5-[2,5-(CH₃)₂-C₆H₃]-1,3,4-thiadiazol-2-yl |
| 1781 | 1-CH(CH₃)₂-imidazol-4-yl |
| 1782 | 1-C(CH₃)₃-imidazol-4-yl |
| 1783 | 1-cyclopropyl-imidazol-4-yl |
| 1784 | 1-C₆H₅-imidazol-4-yl |
| 1785 | 1-(2-CH₃-C₆H₄)-imidazol-4-yl |
| 1786 | 1-(3-CH₃-C₆H₄)-imidazol-4-yl |
| 1787 | 1-(4-CH₃-C₆H₄)-imidazol-4-yl |
| 1788 | 1-(3-OCH₃-C₆H₄)-imidazol-4-yl |
| 1789 | 1-(4-OCH₃-C₆H₄)-imidazol-4-yl |
| 1790 | 1-(4-NO₂-C₆H₄)-imidazol-4-yl |
| 1791 | 1-(3-NO₂-C₆H₄)-imidazol-4-yl |
| 1792 | 1-(4-CN-C₆H₄)-imidazol-4-yl |
| 1793 | 1-(3-CN-C₆H₄)-imidazol-4-yl |
| 1794 | 1-(3-CF₃-C₆H₄)-imidazol-4-yl |
| 1795 | 1-(4-CF₃-C₆H₄)-imidazol-4-yl |
| 1796 | 1-(4-C(CH₃)₃-C₆H₄)-imidazol-4-yl |
| 1797 | 1-(2-Cl-C₆H₄)-imidazol-4-yl |
| 1798 | 1-(3-Cl-C₆H₄)-imidazol-4-yl |
| 1799 | 1-(4-Cl-C₆H₄)-imidazol-4-yl |
| 1800 | 1-(2-Br-C₆H₄)-imidazol-4-yl |
| 1801 | 1-(3-Br-C₆H₄)-imidazol-4-yl |
| 1802 | 1-(4-Br-C₆H₄)-imidazol-4-yl |
| 1803 | 1-(2-F-C₆H₄)-imidazol-4-yl |
| 1804 | 1-(3-F-C₆H₄)-imidazol-4-yl |
| 1805 | 1-(4-F-C₆H₄)-imidazol-4-yl |
| 1806 | 1-(2,4-Cl₂-C₆H₃)-imidazol-4-yl |
| 1807 | 1-(2,5-Cl₂-C₆H₃)-imidazol-4-yl |
| 1808 | 1-(2,6-Cl₂-C₆H₃)-imidazol-4-yl |
| 1809 | 1-(3,4-Cl₂-C₆H₃)-imidazol-4-yl |
| 1810 | 1-(2,4-F₂-C₆H₃)-imidazol-4-yl |
| 1811 | 1-(2,5-F₂-C₆H₃)-imidazol-4-yl |
| 1812 | 1-(2,6-F₂-C₆H₃)-imidazol-4-yl |
| 1813 | 1-(3,4-F₂-C₆H₃)-imidazol-4-yl |
| 1814 | 1-(2-Cl, 5-OCH₃-C₆H₃)-imidazol-4-yl |
| 1815 | 1-(2-Cl, 5-CH₃-C₆H₃)-imidazol-4-yl |
| 1816 | 1-(5-Cl, 2-OCH₃-C₆H₃)-imidazol-4-yl |
| 1817 | 1-(5-Cl, 2-CH₃-C₆H₃)-imidazol-4-yl |
| 1818 | 1-[2,5-(CH₃)₂-C₆H₃]-imidazol-4-yl |

Die Verbindungen I eignen sich zur Bekämpfung von Schadpilzen und tierischen Schädlingen.

Sie können in Abhängigkeit von ihren chemischen und physikalischen Eigenschaften mit üblichen, also dem Fachmann geläufigen, Formulierungshilfsmitteln formuliert werden. Die Produkte dieses Vorgangs werden als "Mittel" bezeichnet.

Geeignete Formulierungshilfsmittel sind z.B. feste oder flüssige Trägerstoffe, oberflächenaktive Mittel und Haftmittel.

Unter flüssigen Trägerstoffen werden flüssige Lösungsmittel wie Wasser und organische Lösungsmittel verstanden, wobei letztere vor allem bei Verwendung von Wasser als Lösungsmittel die Funktion eines Hilfslösungsmittels haben. Als organische Lösungsmittel können verwendet werden: Aromaten wie Xylol, Toluol und Alkylnaphthaline, chlorierte Aromaten oder chlorierte aliphatische Kohlenwasserstoffe wie Chlorbenzole, Chlorethylene und Methylenchlorid, aliphatische Kohlenwasserstoffe wie Cyclohexan und Paraffine, z.B. Mineralölfraktionen, Alkohole wie Butanol, iso-Butanol, Cyclohexanol und Glykol sowie die zugehörigen Ether und Ester, Ketone wie Aceton, Methylethylketon, Methyliso-butylketon und Cyclohexanon, aprotisch dipolare Lösungsmittel wie Dimethylformamid, N-Methyl-2-pyrrolidon und Dimethylsulfoxid.

Als feste Trägerstoffe kommen beispielsweise in Betracht: Natürliche Gesteinsmehle und Mineralerden wie Kieselsäuren, Silicate, Kaoline, Tonerden, Bolus, Löß, Talkum, Kreide, Kalkstein, Kalk, Dolomit, Magnesiumoxid, Quarz, Attapulgit, Montmorillonit und Diatomeenerde; synthetische Gesteinsmehle wie hochdisperse Kieselsäure oder Mehle von synthetischem Aluminiumoxid und von synthetischen Silikaten. Insbesondere für Granulate geeignete feste Trägerstoffe sind beispielsweise: Gebrochene und fraktionierte natürliche Gesteine wie Calcit, Marmor, Bims, Sepiolith; synthetische Granulate aus anorganischen und organischen Mehlen; Granulate aus organischem Material wie Sägemehl, Kokosnußschalen, Maiskolben oder Tabakstengel.

Geeignete oberflächenaktive Mittel sind nichtionogene und anionische Emulgiermittel/schaumerzeugende Mittel und Dispergiermittel:
- Fettsäure-Polyoxyethylenester wie Laurylalkohol-Polyoxyethylenetheracetat,
- Alkyl-Polyoxyethylen- oder -Polyoxypropylenether etwa von iso-Tridecylalkohol und Fettalkohol-Polyoxyethylenether,
- Alkylarylalkohol-Polyoxyethylenether wie Octylphenol-Polyoxyethylenether,
- Tributylphenol-Polyoxyethylenether,
- ethoxyliertes iso-Octyl-, Octyl- oder Nonylphenol oder Rizinusöl,
- Sorbitester,
- Arylsulfonsäuren, Alkylsulfonsäuren, Alkylschwefelsäuren, - Alkali-, Erdalkali- und Ammoniumsalze von Arylsulfonsäuren, z.B. Lignin-, Phenol-, Naphthalin- und Dibutylnaphthalinsulfonsäure, Alkylsulfonsäuren, Alkylarylsulfonsäuren, Alkyl-, Laurylether- und Fettalkoholschwefelsäuren, Fettsäuren, sulfatierten Hexa-, Hepta- und Octadecanolen und Fettalkoholglykolethern,
- Kondensationsprodukte von sulfoniertem Naphthalin und seiner Derivate mit Formaldehyd,
- Kondensationsprodukte von Naphthalinsulfonsäuren mit Phenol und Formaldehyd,
- Eiweißhydrolysate und
- insbesondere als Dispergiermittel: Lignin-Sulfitablaugen und Methylcellulose.

Als Haftmittel eignen sich beispielsweise: Carboxymethylcellulose; natürliche und synthetische pulverige, körnige oder latexförmige Polymere wie Gummiarabicum, Polyvinylalkohol, Polyvinylacetat, natürliche Phospholipide wie Kephaline und Lecithine, synthetische Phospholipide.

Weiterhin können die Mittel einen oder mehrere Vertreter der folgenden Stoffgruppen enthalten: Farbstoffe, andere bekannte Wirkstoffe, Spurennährstoffe und weitere Additive.

Als Farbstoffe kommen z.B. anorganische Pigmente wie Eisenoxid, Titanoxid, Ferrocyanblau, ferner organische Pigmente wie Alizarin-, Azo- und Metallphthalocyaninfarbstoffe in Betracht. Unter anderen bekannten Wirkstoffen sind etwa andere Fungizide sowie Insektizide, Akarizide, Herbizide und Wachstumsregulatoren zu verstehen. Spurennährstoffe sind beispielsweise Salze von Eisen, Mangan, Bor, Kupfer, Kobalt, Molybdän und Zink. Als weitere Additive sind etwa mineralische und vegetabile Öle geeignet.

Die Mittel können darüberhinaus mit sonstigen, praktisch bedeutsamen Mischungspartnern wie Düngemittel oder sonstige fertige wirkstoffhaltige Mittel vermischt sein.

Die Herstellung der Mittel erfolgt in an sich bekannter Weise, nämlich in Abhängigkeit von den chemischen und physikalischen Eigenschaften der eingesetzten Stoffe z.B. durch Mischen, gemeinsames Vermahlen, Aufsprühen, Extrudieren, Granulieren oder Auflösen in Wasser, letzteres ggf. unter Zuhilfenahme eines organischen Lösungsmittels. Pulver, Streu- und Stäubemittel sind z.B. durch Mischen oder gemeinsames Vermahlen der Verbindungen I mit einem festen Trägerstoff erhältlich.

Bei den Mitteln handelt es sich in Abhängigkeit von den eingesetzten Stoffen z.B. um Lösungen, Emulsionen, Suspensionen, Pulver, Schäume, Pasten, Granulate, Aerosole oder Feinstverkapselungen in polymeren Stoffen oder in Saatgut-Hüllmassen.

Zur Anwendung werden die für den Handel in der Regel als Konzentrate vorliegenden Mittel gegebenenfalls wie üblich aufgelöst, verdünnt usw., bei Spritzpulvern, wasserdispergierbaren Granulaten, emulgierbaren Konzentraten, Dispersionen und teilweise auch bei Mikrogranulaten normalerweise unter Verwendung von Wasser. Staubförmige und granulierte Zubereitungen sowie versprühbare Lösungen werden vor der Anwendung meist nicht mehr mit weiteren inerten Stoffen verdünnt.

Die Ausbringung der Mittel erfolgt in an sich bekannter Weise, etwa durch Versprühen, Vernebeln, Verstäuben, Verstreuen oder Gießen. Die Pflanzen werden in der Regel mit den Mitteln besprüht oder bestäubt. Alternativ oder zusätzlich behandelt man die Samen der Pflanzen in an sich bekannter Weise.

### Beispiele für solche Zubereitungen sind:

- I.: eine Lösung aus 90 Gew.-Teilen einer erfindungsgemäßen Verbindung I und 10 Gew.-Teilen N-Methyl-2-pyrrolidon, die zur Anwendung in Form kleinster Tropfen geeignet ist;
- II.: eine Mischung aus 20 Gew.-Teilen einer erfindungsgemäßen Verbindung I, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen des Calciumsalzes der Dodecylbenzolsulfonsäure, 5 Gew.-Teilen des Anlagerungsproduktes von 40 Mol Ethylenoxid an 1 Mol Ricinusöl: durch feines Verteilen der Lösung in Wasser erhält man eine Dispersion;
- III.: eine wäßrige Dispersion aus 20 Gew.-Teilen einer erfindungsgemäßen Verbindung I, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
- IV.: eine wäßrige Dispersion aus 20 Gew.-Teilen einer erfindungsgemäßen Verbindung I, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
- V.: eine in einer Hammermühle vermahlene Mischung aus 80 Gew.-Teilen einer erfindungsgemäßen Verbindung I, 3 Gew.-Teilen des Natriumsalzes der Diisobutylnaphtalin-1-sulfonsäure, 10 Gew.-Teilen des Natriumsalzes einer Ligninsulfonsäure aus einer Sulfitablauge und 7 Gew.-Teilen pulverförmigem Kieselsäuregel: durch feines Verteilen der Mischung in Wasser erhält man eine Spritzbrühe;
- VI.: eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
- VII.: eine innige Mischung aus 30 Gew.-Teilen einer erfindungsgemäßen Verbindung I, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
- VIII.: eine stabile wäßrige Dispersion aus 40 Gew.-Teilen einer erfindungsgemäßen Verbindung I, 10 Gew.-Teilen des Natriumsalzes eines Phenolsulfonsäure-Harnstoff-Formaldehyd-Kondensates, 2 Gew.-Teilen Kieselgel und 48 Gew.-Teilen Wasser, die weiter verdünnt werden kann;
- IX.: eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkoholpolyglykolether, 20 Gew.-Teilen des Natriumsalzes eines Phenolsulfonsäure-Harnstoff-Formaldehyd-Kondensates und 68 Gew.-Teilen eines paraffinischen Mineralöls.

Werden die Verbindungen I als solche appliziert, so kommt es vor allem auf deren feine Verteilung an.

Die Verbindungen I und die erfindungsgemäßen Mittel zeichnen sich durch eine hervorragende Wirksamkeit gegen ein breites Spektrum von Schadpilzen (pflanzenpathogene Pilze), insbesondere aus der Klasse der
- Ascomyceten,
- Basidiomyceten,
- Deuteromyceten und
- Phycomyceten
aus. Sie sind zum Teil systemisch wirksam und können als Blatt-und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen sowie an den Samen dieser Pflanzen.

Die Verbindungen I und ihre Salze sowie die erfindungsgemäßen Mittel werden angewendet, indem man die Schadpilze, deren Lebensraum oder die vor Pilzbefall zu schützenden Saatgüter, Pflanzen, Flächen, Materialien oder Räume mit einer fungizid wirksamen Menge der Mittel oder der Verbindungen I bzw. ihren Salzen behandelt. Die Anwendung kann vor oder nach dem Befall durch die Pilze erfolgen.

Speziell eignen sich die erfindungsgemäßen Mittel und die Verbindungen I zur Bekämpfung folgender Pflanzenkrankheiten:
Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Zierpflanzen und Gemüse, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Pseudoperonospora-Arten in Hopfen und Gurken, Alternaria-Arten an Gemüse und Obst.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:

Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylen-diamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;

Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthal-säure-di-isopropylester;

heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4-triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio) -tetrahydrophthalimid, N-Trichlormethylthio-tetrahydrophthalimid, N-Trichlormethylthiophthalimid,

N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-amino-butyrolacton, DL-N-(2,6-Dimethylphenyl)-N-(phenylacetyl)-alanin-methylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl) -1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Strobilurine wie Methyl-E-methoximino-[α-(o-tolyloxy)-o-tolyl]-acetat, Methyl-E-2-{2-[6-(2-cyanophenoxy)-pyrimidin-4-yloxy]-phenyl}-3-methoxyacrylat, Methyl-E-methoximino-[α-(2-phenoxyphenyl)]-acetamid, Methyl-E-methoximino-[α-(2,5-dimethylphenoxy)-o-tolyl]-acetamid.

Anilinopyrimidine wie N-(4,6-Dimethylpyrimidin-2-yl)-anilin, N-[4-Methyl-6-(1-propinyl)-pyrimidin-2-yl]-anilin, N-(4-Methyl-6-cyclopropyl-pyrimidin-2-yl)-anilin.
Phenylpyrrole wie 4-(2,2-difluor-1,3-benzodioxol-4-yl)-pyrrol-3-carbonitril, Zimtsäureamide wie 3-(4-Chlorphenyl)-3-(3,4-dimethoxyphenyl)-acrylsäuremorpholid,
(2RS,3SR)-1-[3-(2-Chlorphenyl)-2-[4-fluorphenyl]oxiran-2-yl-methyl]-1H-1,2,4-triazol.

Die Verbindungen der Formel I sind außerdem geeignet, tierische Schädlinge, vor allem aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

### Synthesebeispiele

Die in den nachstehenden Synthesebeispielen wiedergegebenen Vorschriften können unter Abwandlung der Ausgangsverbindungen zur Gewinnung weiterer Vertreter der Verbindungen I benutzt werden.

Die chemischen Verschiebungen (δ [ppm]) der ¹H-NMR-Spektren wurde gemessen gegen Tetramethylsilan (br = breites Signal, s = Singulett, d = Dublett, m = Multiplett).

### Beispiel 1

### (E,E)-2-Methoxyimino-2-(2'-[{1"-methyl, 1"-benzoyl}iminooxymethyl]phenyl)essigsäuremethylester

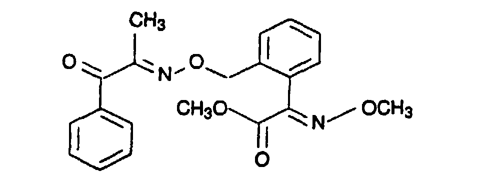

Zu einer Lösung von 24,5 g (0,15 mol) 1-Phenyl-1,2-propandion-2-E-oxim und 43 g (0,15 mol) E-2-Methoxyimino-2-[(2'-brommethyl)phenyl)essigsäuremethylester (vgl. EP-A 400 417) in 150 ml N,N-Dimethylformamid gab man 27 g Natriummethylatlösung (30 %-ig in Methanol; 0,15 mol) und ließ 2 h bei Raumtemperatur rühren. Das Reaktionsgemisch wurde auf kalte verdünnte Salzsäure gegossen, mit Methyl-tert.-butylether extrahiert, die organische Phase mit Wasser gewaschen, über Na₂SO₄ getrocknet und das Lösungsmittel abdestilliert. Nach Verreiben des Rückstandes mit Methanol erhielt man 42,7 g der Titelverbindung als weißes Pulver vom Schmelzpunkt 94-95°C.
¹H-NMR (CDCl₃): 2,09 (s,3H); 3,81 (s,3H); 4,01 (s,3H); 5,13 (s,2H); 7,18-7,79 (m,9H).

### Beispiel 2

### (E,E)-2-Methoxyimino-2-(2'-[{1"-methyl, 1"-benzoyl}iminooxymethyl]phenyl)essigsäuremonomethylamid

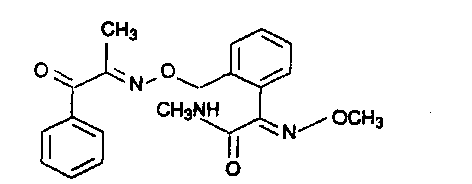

42,7 g (0,12 mol) des Produktes aus Beispiel 1 wurden in 400 ml THF gelöst, mit 100 ml 40 gew.-%-iger wäßriger Monomethylamin-Lösung versetzt und 16 Stunden bei Raumtemperatur stehengelassen. Anschließend wurde mit 2 N Salzsäure versetzt, mit Methyl-tert.-butylether extrahiert, die organische Phase mit Wasser gewaschen, über Natriumsulfat getrocknet und das Lösungsmittel entfernt. Als Rückstand verblieben 34,7 g der Titelverbindung als weißes Pulver vom Schmelzpunkt 114-118°C.
¹H-NMR (CDCl₃): 2,09 (s,3H); 2,85 (d,3H); 3,90 (s,3H); 5,15 (s,2H); 6,71 (s,br,1H); 7,19-7,80 (m,9H).

### Beispiel 3

### (E,E,E)-2-Methoxyimino-2-[2'-[[1"-methyl, 1"-[(1‴-cyanimino, 1‴-phenyl)methyl]]iminooxymethyl]phenyl]essigsäuremonomethylamid

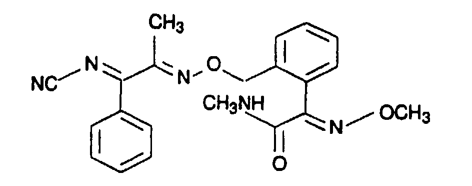

Zu einer Lösung von 2,5 g (6,8 mmol) des Produkts aus Beispiel 2 in 50 ml Methylenchlorid gab man 5,0 g (27 mmol) Bis(trimethylsilyl)carbodiimid und ließ 60 Stunden unter Feuchtigkeitsausschluß bei Raumtemperatur rühren. Anschließend versetzte man mit 5,1 g (27 mmol) Titantetrachlorid und ließ erneut 16 Stunden bei Raumtemperatur rühren. Der Reaktionsansatz wurde danach auf Eiswasser gegossen und mit Methylenchlorid extrahiert. Die organische Phase wurde mit Wasser gewaschen, über Na₂SO₄ getrocknet und das Lösungsmittel abdestilliert. Als Rückstand verblieben 1,8 g der Titelverbindung als farbloses Öl.
¹H-NMR (CDCl₃): 2,12 (s,3H); 2,90 (d,3H); 3,91 (s,3H); 5,10 (s,2H); 6,77 (s,br,1H); 7,15-7,56 (m,9H).
IR (KBr): 3361, 2182, 1661, 1564, 1555, 1038, 1002, 996, 978, 704 cm⁻¹

### Beispiel 4

### (E,E)-2-Methoxyimino-2-{2'-[(1"-methyl, 1"- (4'" -chlorbenzoyl))-iminooxymethyl]-phenyl}essigsäuremethylester

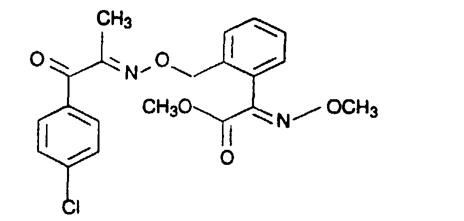

Zu einer Lösung von 5,6g (35mmol) 1-(4'-Chlorphenyl)-1,2-propandion-2-E-oxim und 10g (35mmol) E-2-Methoxyimino-2-[(2-'brommethyl)phenyl]essigsäuremethylester [vgl. EP-A 400 417] in 150 ml N,N-Dimethylformamid gab man 6,3g Natriummethylatlösung (30%-ig in Methanol; 35mmol) und ließ 16 Stunden bei Raumtemperatur (ca. 25°C) rühren. Das Reaktionsgemisch wurde auf kalte verdünnte Salzsäure gegossen und mit tert.-Butylmethylether extrahiert. Die organische Phase wurde mit Wasser gewaschen, anschließend getrocknet und bei vermindertem Druck eingeengt. Man erhielt so 12,3g der Titelverbindung als hellgelbes Öl.
¹H-NHR (CDCl₃): 2,07 (s,3H); 3,82 (s,3H); 4,01 (s,3H); 5,13 (s,2H); 7,17-7,71 (m,8H).

### Beispiel 5

### (E,E,E)-2-Methoxyimino-2-{2'-[(1"-methyl, 1"-((1"'-cyanimino, 1'"- (4'"-chlorphenyl))methyl))-iminooxymethyl]-phenyl}essigsäuremethylester

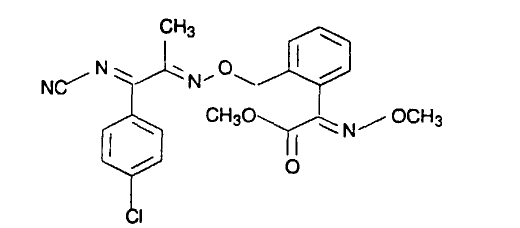

Zu einer Lösung von 5,0g (12,4mmol) des Produktes aus Beispiel 4 in 100 ml Methylenchlorid gab man zunächst 9,2g (49,7mmol) Bis(trimethylsilyl)carbodiimid und anschließend 9,4g Titantetrachlorid und ließ die Mischung dann 60 Stunden unter Ausschluß von Feuchtigkeit bei Raumtemperatur (ca. 25°C) rühren. Die Reaktionsmischung wurde danach auf Eiswasser gegeben umd mit Methylenchlorid extrahiert. Die organische Phase wurde mit Wasser gewaschen, getrocknet und bei vermindertem Druck eingeengt. Der so erhaltene Rückstand wurde über eine Kieselgelsäule chromatographiert (tert.-Butylmethylether/Cyclohexan). Man erhielt 1,7g der Titelverbindung als farbloses Öl.
¹H-NMR (CDCl₃): 2,10 (s,3H); 3,84 (s,3H); 4,00 (s,3H); 5,09 (s,2H); 7,15-7,48 (m,8H).

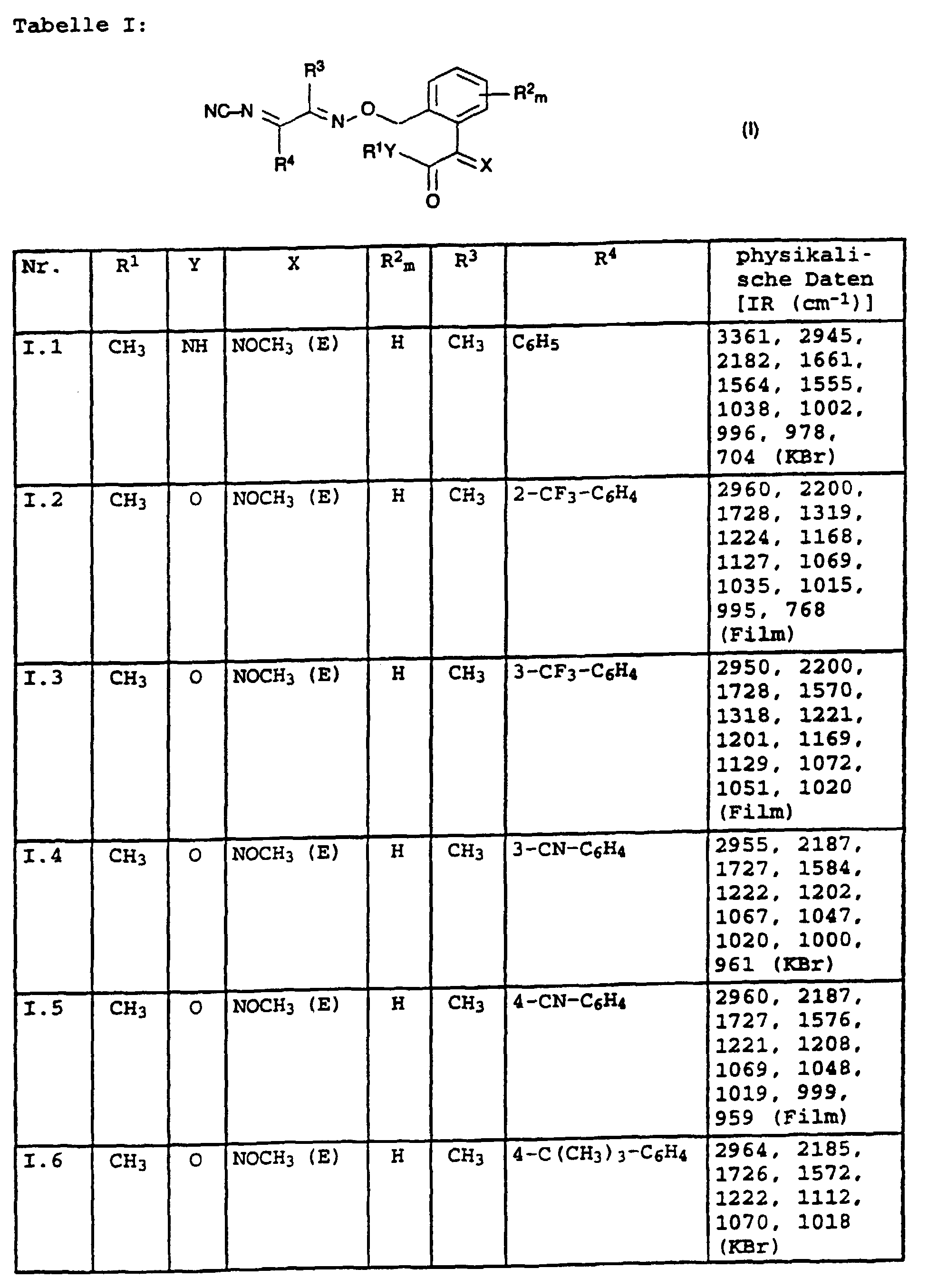

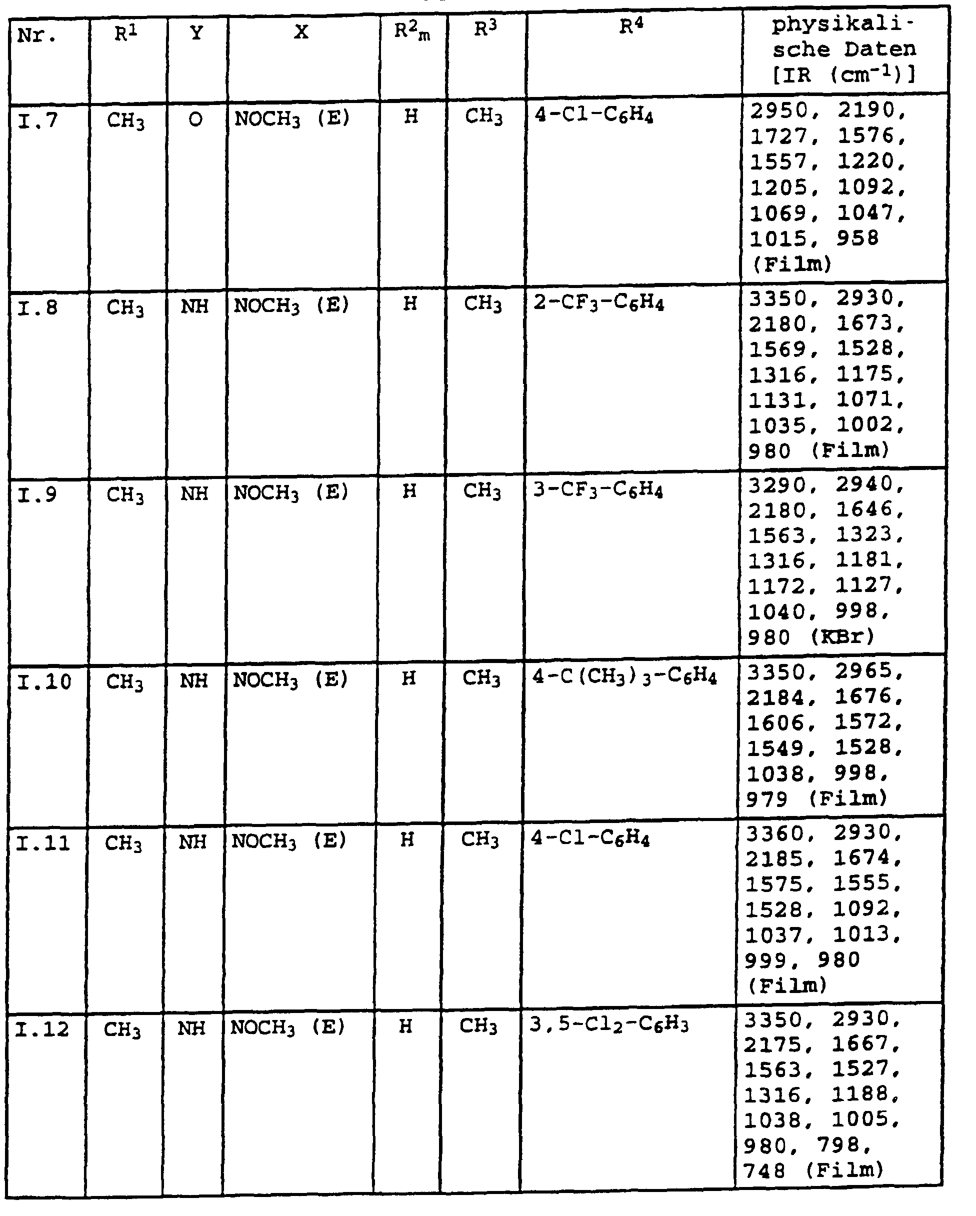

### Anwendungsbeispiele

### 1. Beispiel zur Wirkung gegen Schadpilze

Für die folgenden Versuche zur fungiziden Wirkung der Verbindungen I wurde eine Emulsion verwendet, welche zu 10 Gew.-% aus dem Wirkstoff und zu 90 Gew.-% aus einem Gemisch aus

| | |
|---|---|
| 70 Gew.-% | Cyclohexanol, |
| 20 Gew.-% | Nekanil® LN (Lutensol® AP6, Netzmittel mit Emulgier- und Dispergierwirkung auf der Basis ethoxylierter Alkylphenole) und |
| 10 Gew.-% | Emulphor® EL (Emulan® EL, Emulgator auf der Basis ethoxylierter Fettalkohole) |

bestand. Die gewünschten Wirkstoff-Konzentrationen wurden durch Verdünnen dieser Emulsion mit Wasser eingestellt.

Als Vergleichssubstanz A diente die Verbindung 3.81 (WO-A 95/18,789, Tabelle 3); als Vergleichssubstanz *B* diente die Verbindung H-2 (WO-A 93/16,986, Tabelle 1).

### Anwendungsbeispiel 1

### Wirksamkeit gegen Weizenmehltau

Blätter von in Töpfen gewachsenen Weizenkeimlingen der Sorte "Frühgold" wurden mit wäßriger Spritzbrühe, die 10 Gew.-% Wirkstoff, 63 % Cyclohexanon und 27 Gew.-% Emulgiermittel in der Trockensubstanz enthielt, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung visuell ermittelt.

In diesem Test zeigten die mit 63 ppm-haltiger wäßriger Aufbereitung der Verbindungen I.1, I.3, I.7, I.9, I.11 und I.12 behandelten Pflanzen einen Befall von 15% und weniger während die mit der gleichen Menge der Verbindungen ***A*** und ***B*** behandelten Pflanzen ebenso wie die unbehandelten Pflanzen zu 60% befallen waren.

### Anwendungsbeisiel 2

### Wirksamkeit gegen Plasmopara viticola

Blätter von Topfreben der Sorte "Müller-Thurgau" wurden mit wäßriger Spritzbrühe, die 10 Gew.-% Wirkstoff, 63 % Cyclohexanon und 27 Gew.-% Emulgiermittel in der Trockensubstanz enthielt, besprüht. Um die Wirkungsdauer des Wirkstoffs beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die visuelle Beurteilung des Ausmaßes des Pilzausbruchs auf den Blattunterseiten.

In diesem Test zeigten die mit 63 ppm-haltiger wäßriger Aufbereitung der Verbindungen I.1, I.2, I.3, I.4, I.6, I.7, I.8, I.9, I.10 und I.11 behandelten Pflanzen einen Befall von 15% und weniger während die mit der gleichen Menge der Verbindung ***A*** behandelten Pflanzen zu 65% befallen waren. Die unbehandelten Pflanzen waren zu 80% befallen.

### Anwendungsbeispiel 3

### Wirkung gegen Pyricularia oryzae (Reisbrand; protektiv)

Reiskeimlinge (Sorte: "Tai Nong 67") wurden mit der Wirkstoffaufbereitung tropfnaß gespritzt (Aufwandmenge: 63ppm; Aufbereitung: 10% Wirkstoff, 63% Cyclohexanon und 27% Emulgiermittel). Nach 24 Stunden wurden die Pflanzen mit einer wäßrigen Sporensuspension des Pilzes *Pyricularia oryzae* besprüht und 6 Tage bei 22-24°C bei einer relativen Luftfeuchtigkeit von 95-99% bewahrt. Die Beurteilung erfolgte visuell.

In diesem Test zeigten die mit den Verbindungen I.1, I.3, I.4, I.5, I.6, I.7 und I.10 behandelten Pflanzen einen Befall von 15% und weniger während die unbehandelten Pflanzen zu 75% befallen waren. Die mit den Vergleichssubstanzen ***A*** bzw. ***B*** behandelten Pflanzen waren zu 65% und zu 40% befallen.

### 2. Beispiele zur Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. Cyaniminooximether der Formel I 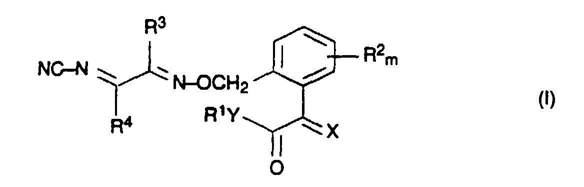 in der die Variablen die folgenden Bedeutungen haben:
X NOCH₃, CHOCH₃ oder CHCH₃;
Y O oder NZ, wobei Z für Wasserstoff oder C₁-C₄-Alkyl steht;
R¹ Wasserstoff oder C₁-C₄-Alkyl;
R² Cyano, Nitro, Halogen, C₁-C₄-Alkyl, Trifluormethyl oder C₁-C₄-Alkoxy;
m 0, 1 oder 2, wobei die Reste R² verschieden sein können, wenn m für 2 steht;
R³ Wasserstoff, Cyano, Alkyl, Halogenalkyl, Alkoxy oder Cycloalkyl;
R⁴ Wasserstoff oder gegebenenfalls substituiertes; Alkyl, Alkenyl, Alkinyl, Cycloalkyl, Cycloalkenyl, Heterocyclyl, Aryl oder Heteroaryl,
sowie deren Salze.

2. Verfahren zur Herstellung von Verbindungen der Formel I gemäß Anspruch 1, dadurch gekennzeichnet, daß man ein Benzylderivat der Formel II 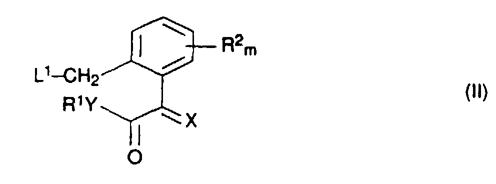 in der L¹ für eine nucleophil austauschbare Abgangsgruppe steht, mit einem Hydroxyimin der Formel III 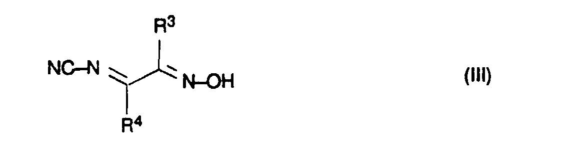 umsetzt.

3. Verfahren zur Herstellung von Verbindungen der Formel I gemäß Anspruch 1, dadurch gekennzeichnet, daß man ein Benzylderivat der Formel II gemäß Anspruch 2 mit einem Carbonylhydroxyiminoderivat der Formel IV 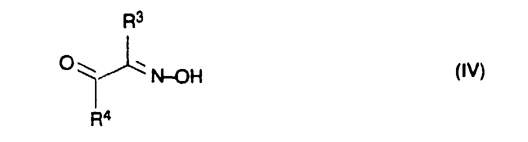 zu einer Verbindung der Formel V 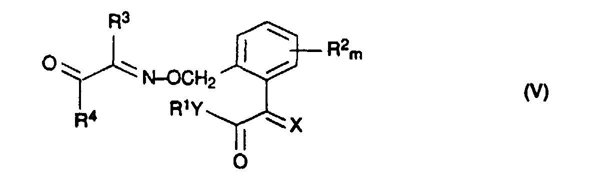 und V anschließend mit Bis(trimethylsilyl)carbodiimid umsetzt.

4. Zur Bekämpfung von tierischen Schädlingen oder von Schadpilzen geeignete Mittel, enthaltend eine Verbindung der Formel I oder eines ihrer Salze gemäß Anspruch 1 und mindestens ein Formulierungshilfsmittel.

5. Mittel nach Anspruch 4 zur Bekämpfung tierischer Schädlinge aus der Klasse der Insekten, Spinntiere oder Nematoden.

6. Verfahren zur Bekämpfung von tierischen Schädlingen oder Schadpilzen, dadurch gekennzeichnet, daß man die Schädlinge oder Schadpilze, deren Lebensraum oder die von ihnen freizuhaltenden Pflanzen, Flächen, Materialien oder Räume mit einer wirksamen Menge einer Verbindung der Formel I oder eines ihrer Salze gemäß Anspruch 1 behandelt.

7. Verwendung der Verbindungen I gemäß Anspruch 1 zur Herstellung von Mitteln gegen tierische Schädlinge oder gegen Schadpilze.

8. Verwendung der Verbindungen I gemäß Anspruch 1 zur Bekämpfung von tierischen Schädlingen oder von Schadpilzen.

9. Verbindungen der Formel III 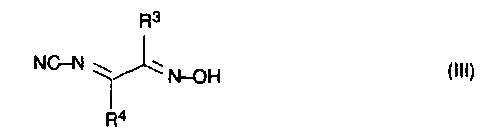 wobei die Variablen die in Anspruch 1 angegebenen Bedeutungen haben.

10. Verbindungen der Formel VII 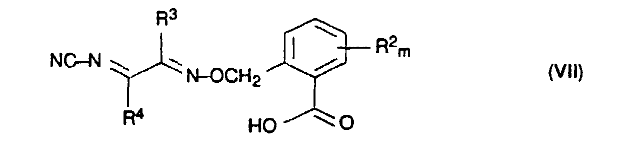 wobei die Variablen die in Anspruch 1 angegebenen Bedeutungen haben.

11. Verbindungen der Formel VIII 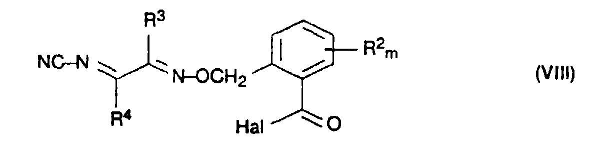 Hal = Halogen, wobei die übrigen Variablen die in Anspruch 1 angegebenen Bedeutungen haben.

12. Verbindungen der Formel IX 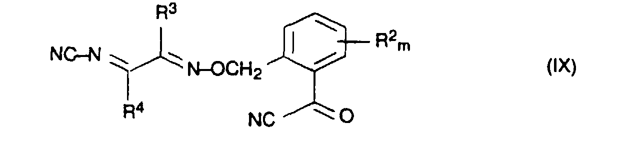 wobei die Variablen die in Anspruch 1 angegebenen Bedeutungen haben.

13. Verbindungen der Formel X 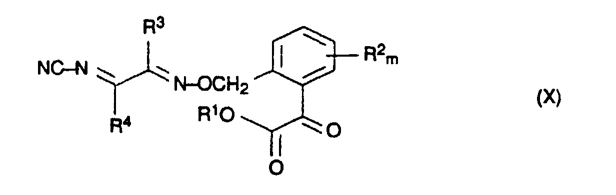 wobei die Variablen die in Anspruch 1 angegebenen Bedeutungen haben.

14. Verbindungen der Formel XI 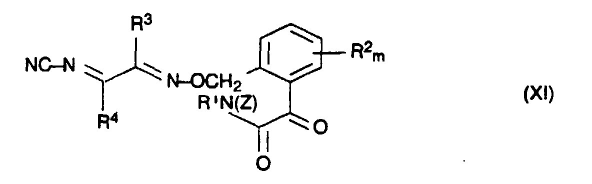 wobei die Variablen die in Anspruch 1 angegebenen Bedeutungen haben.

15. Verbindungen der Formel XII 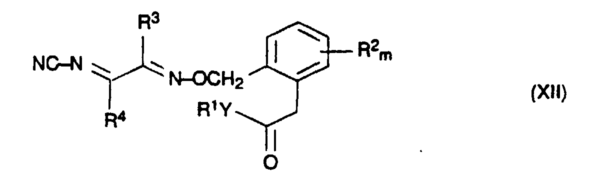 wobei die Variablen die in Anspruch 1 angegebenen Bedeutungen haben.

16. Verwendung der Verbindungen gemäß den Ansprüchen 9 bis 15 als Zwischenprodukte.

## Claims

1. A cyanoiminooxime ether of the formula I 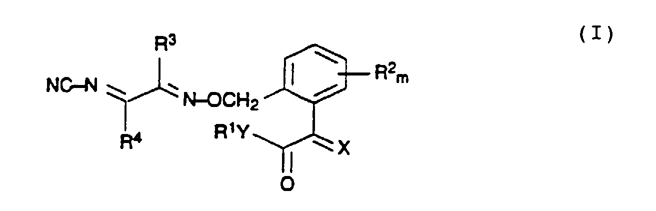 where the variables have the following meanings:
X is NOCH₃, CHOCH₃ or CHCH₃;
Y is O or NZ where Z is hydrogen or C₁-C₄-alkyl;
R¹ is hydrogen or C₁-C₄-alkyl;
R² is cyano, nitro, halogen, C₁-C₄-alkyl, trifluoromethyl or C₁-C₄-alkoxy;
m is 0, 1 or 2, it being possible for the radicals R² to be different when m is 2;
R³ is hydrogen, cyano, alkyl, haloalkyl, alkoxy or cycloalkyl;
R⁴ is hydrogen or unsubstituted or substituted: alkyl, alkenyl, alkynyl, cycloalkyl, cycloalkenyl, heterocyclyl, aryl or hetaryl,
or a salt thereof.

2. A process for the preparation of a compound of the formula I as claimed in claim 1, which comprises reacting a benzyl derivative of the formula II 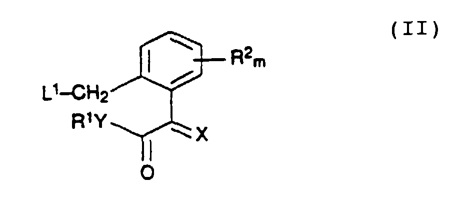 where L¹ is a nucleophilically exchangeable leaving group with a hydroxyimine of the formula III

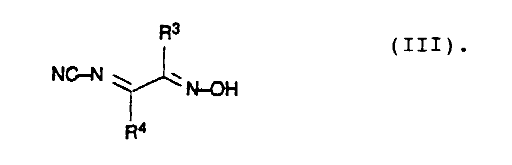

3. A process for the preparation of a compound of the formula I as claimed in claim 1, which comprises reacting a benzyl derivative of the formula II as claimed in claim 2 with a carbonylhydroxyimino derivative of the formula IV 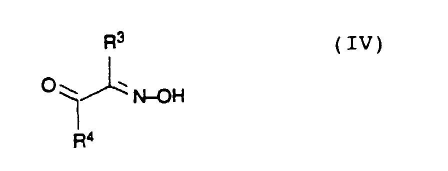 to give a compound of the formula V 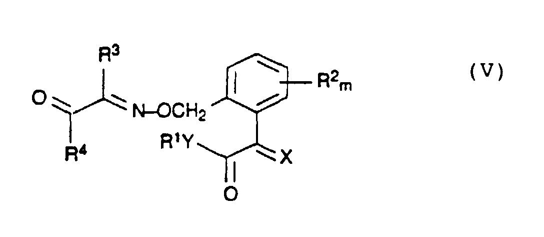 and subsequently reacting V with bis(trimethylsilyl)carbodiimide.

4. A composition suitable for controlling animal pests or harmful fungi, comprising a compound of the formula I or of a salt thereof as claimed in claim 1 and at least one formulation auxiliary.

5. A composition as claimed in claim 4 for controlling animal pests from the classes of the insects, arachnids or nematodes.

6. A method of controlling animal pests or harmful fungi, which comprises treating the pests or harmful fungi, their environment, or the plants, areas, materials or spaces to be kept free from them, with an effective amount of a compound of the formula I or of a salt thereof as claimed in claim 1.

7. The use of a compound I as claimed in claim 1 for the preparation of compositions against animal pests or against harmful fungi.

8. The use of a compound I as claimed in claim 1 for controlling animal pests or harmful fungi.

9. A compound of the formula III 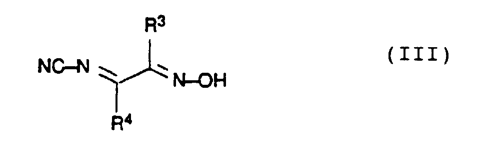 where the variables have the meanings given in claim 1.

10. A compound of the formula VII 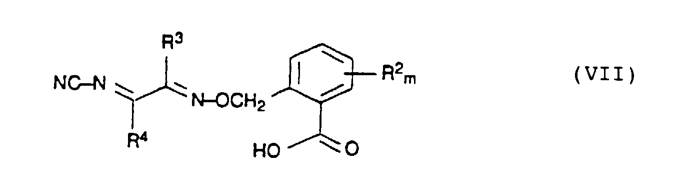 where the variables have the meanings given in claim 1.

11. A compound of the formula VIII 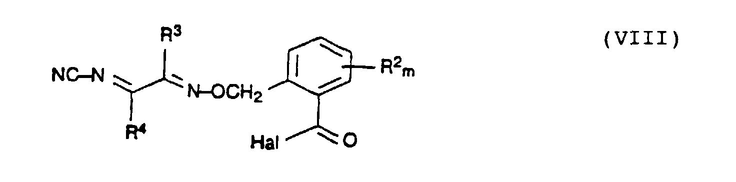 Hal = halogen, the remaining variables having the meanings given in claim 1.

12. A compound of the formula IX 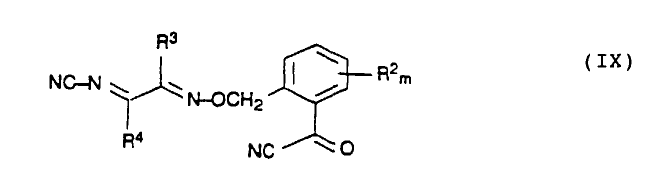 where the variables have the meanings given in claim 1.

13. A compound of the formula X 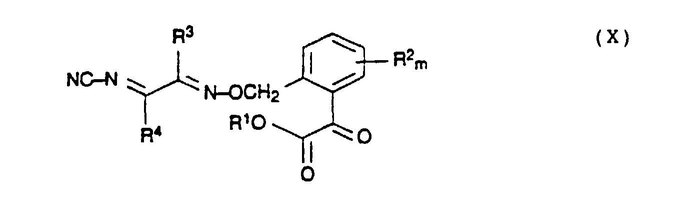 where the variables have the meanings given in claim 1.

14. A compound of the formula XI 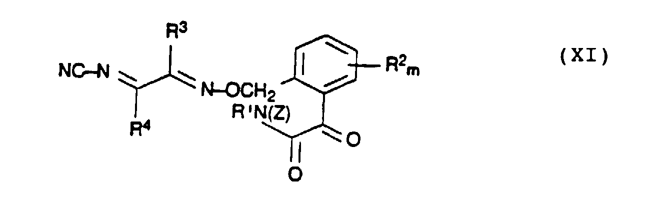 where the variables have the meanings given in claim 1.

15. A compound of the formula XII 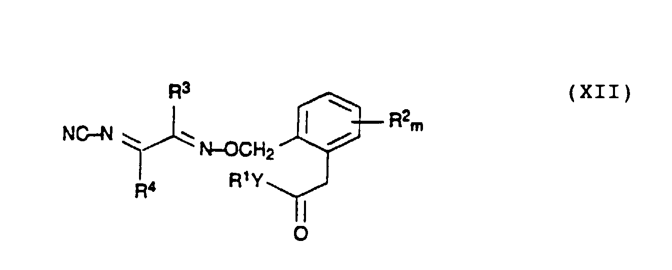 where the variables have the meanings given in claim 1.

16. The use of the compounds as claimed in any of claims 9 to 15 as intermediates.

## Revendications

1. Ethers de cyaniminoximes de formule I 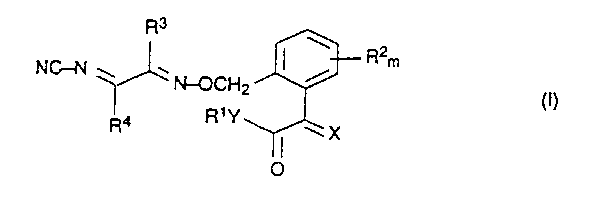 dans laquelle les symboles ont les significations suivantes :
X : NOCH₃, CHOCH₃ ou CHCH₃;
Y : O ou NZ, Z représentant l'hydrogène ou un groupe alkyle en C1-C4;
R¹ : l'hydrogène ou un groupe alkyle en C1-C4;
R² : un groupe cyano, nitro, un halogène, un groupe alkyle en C1-C4, trifluorométhyle ou alcoxy en C1-C4;
m : 0, 1 ou 2, les symboles R² pouvant avoir des significations différentes lorsque m est égal à 2;
R³ : l'hydrogène, un groupe cyano, alkyle, halogénoalkyle, alcoxy ou cycloalkyle;
R⁴ : l'hydrogène ou un groupe alkyle, alcényle, alcynyle, cycloalkyle, cycloalcényle, hétérocyclyle, aryle ou hétéroaryle, chacun d'eux éventuellement substitué, et leurs sels.

2. Procédé de préparation des composés de formule I de la revendication 1, caractérisé par le fait que l'on fait réagir un dérivé benzylique de formule II 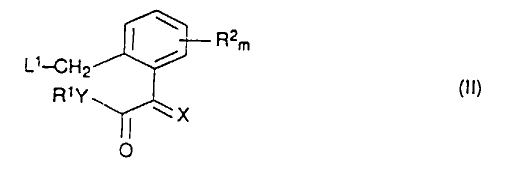 dans laquelle L1 représente un groupe éliminable par échange nucléophique, avec une hydroxyimine de formule III

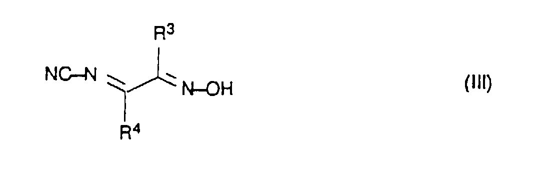

3. Procédé de préparation des composés de formule I de la revendication 1, caractérisé par le fait que l'on fait réagir un dérivé benzylique de formule II de la revendication 2 avec un dérivé carbonylhydroxyiminé de formule IV 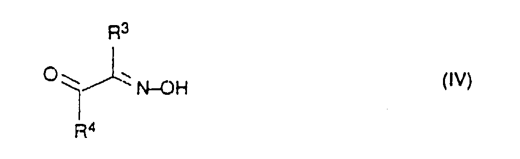 ce qui donne un composé de formule V 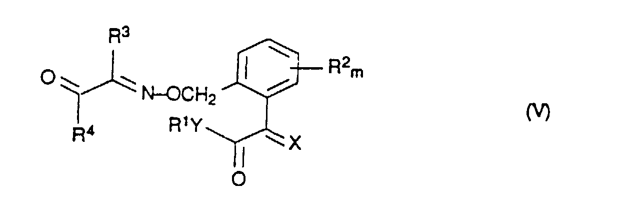 qu'on fait ensuite réagir avec le bis-(triméthylsilyl)-carbodiimide.

4. Produit approprié à l'utilisation pour la lutte contre les parasites animaux ou contre les mycètes nuisibles, contenant un composé de formule I ou l'un de ses sels selon la revendication 1, et au moins un produit auxiliaire de formulation.

5. Produit selon la revendication 4, pour la lutte contre les parasites animaux appartenant aux classes des insectes, des arachnides ou des nématodes.

6. Procédé pour combattre des parasites animaux ou des mycètes nuisibles, caractérisé par le fait que l'on traite les parasites ou les mycètes nuisibles, leur habitat ou les végétaux, surfaces, matériaux ou locaux qu'on veut débarrasser de ces parasites par une quantité efficace d'un composé de formule I ou d'un de ses sels selon la revendication 1.

7. Utilisation des composés de formule I selon la revendication 1 pour la préparation de produits contre les parasites animaux ou contre les mycètes nuisibles.

8. Utilisation des composés de formule I selon la revendication 1 pour la lutte contre les parasites animaux ou contre les mycètes nuisibles.

9. Composés de formule III 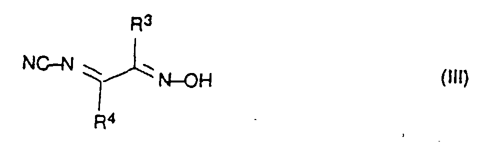 dans laquelle les variables ont les significations de la revendication 1.

10. Composés de formule VII 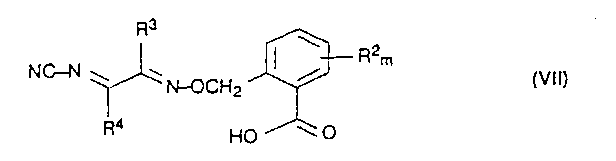 dans laquelle les variables ont les significations de la revendication 1.

11. Composés de formule VIII 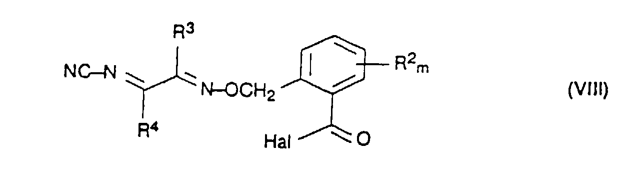 dans laquelle :
Hal = halogène, et dans laquelle les autres variables ont les significations de la revendication 1.

12. Composés de formule IX 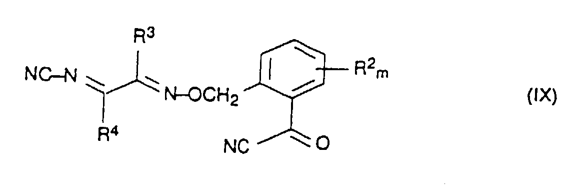 dans laquelle les variables ont les significations de la revendication 1.

13. Composés de formule X 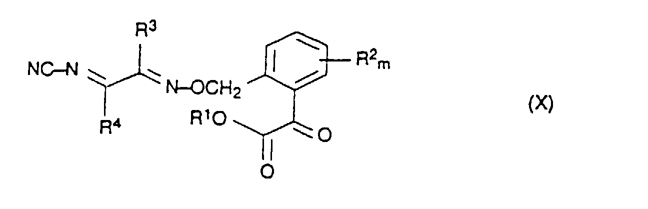 dans laquelle les variables ont les significations de la revendication 1.

14. Composés de formule XI 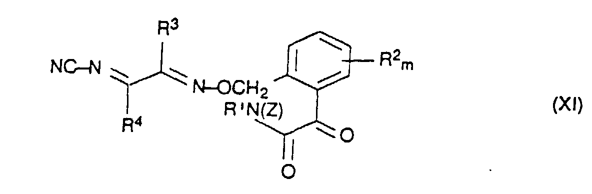 dans laquelle les variables ont les significations de la revendication 1.

15. Composés de formule XII 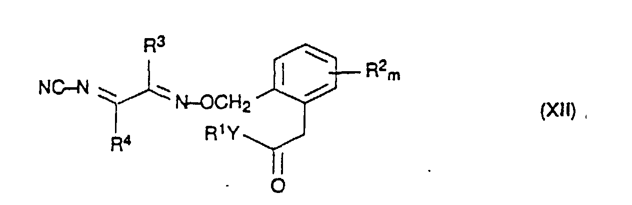 dans laquelle les variables ont les significations de la revendication 1.

16. Utilisation des composés selon les revendications 9 à 15 comme produits intermédiaires.
